# EUROPÄISCHE PATENTSCHRIFT

(11) **EP 3 868 761 B1**
(45) Veröffentlichungstag und Bekanntmachung des Hinweises auf die Patenterteilung: **01.03.2023**
(21) Anmeldenummer: 21165573.3
(22) Anmeldetag: 31.03.2016
(51) Int. Cl.: C07D 471/04, C07D 519/00, A61P 35/00, A61K 31/4375, A61K 45/06

(54) **IMIDAZOLONYLCHINOLINE UND DEREN VERWENDUNG ALS ATM KINASE INHIBITOREN**
IMIDAZOLONYLCHINOLINES AND THEIR USE AS ATM KINASE INHIBITORS
IMIDAZOLONYL-QUINOLÉINES ET LEUR UTILISATION COMME INHIBITEURS DU ATM KINASE

(30) Priorität: 02.04.2015 EP 15000968
(43) Veröffentlichungstag der Anmeldung: 25.08.2021
(62) Teilanmeldung aus: 19172903.7
(73) Patentinhaber: Merck Patent GmbH, 64293 Darmstadt (DE)
(72) Erfinder: Fuchss, Thomas, 64293 Darmstadt (DE); Schiemann, Kai, 64293 Darmstadt (DE)

(56) Entgegenhaltungen:
- WO-A1-2010/139731
- WO-A1-2011/054846
- WO-A1-2012/028233

## Beschreibung

### HINTERGRUND DER ERFINDUNG

Die vorliegende Erfindung betrifft spezifische Imidazolonylchinolin-Verbindungen und deren Verwendung bei der Hemmung, Regulierung und/oder Modulation der Signaltransduktion von Kinasen, insbesondere der ATM Kinase, ferner pharmazeutische Zusammensetzungen, die diese Verbindungen enthalten, sowie die Verwendung der Verbindungen zur Behandlung von Krankheiten, die einen Bezug zu ATM Kinase aufweisen, insbesondere Krebs.

Die Serin/Threonin Protein-Kinase ATM (*Ataxia* Telangiectasia Mutated Kinase) gehört zur PIKK- Familie von Kinasen mit katalytischen Domänen, die Homologie zu den Phosphoinositid-3-Kinasen (PI3-Kinase, PI3K) aufweisen. Diese Kinasen sind an einer Vielzahl von zellulären Schlüsselfunktionen, wie Zellwachstum, Zellproliferation, Migration, Differenzierung, Überleben und Zelladhäsion beteiligt. insbesondere reagieren diese Kinasen auf DNA-Schädigung durch Aktivierung des Zellzyklus-Arrests und DNA-Reparaturprogrammen (DDR: DNA Damage Response). ATM ist ein Produkt des ATM Gens und spielt eine Schlüsselrolle bei der Reparatur von Schäden am DNA-Doppelstrang (DSB. Double strand breaks) durch homologe Rekombination und nicht homologe End-zu-End-Verbindung (NHEJ: non-homologous end joining). Derartige Doppelstrangschäden sind besonders zytotoxisch.

Eines der durchgängigen Merkmale von Tumoren beim Menschen ist deren genomische Instabilität, wobei die spezifischen Defekte des DNA-Reparaturmechanismus bei den meisten Krebsarten bislang nicht bekannt sind. Diese Instabilität stellt den therapeutischen Ansatzpunkt für die seit langem vorherrschend praktizierte Chemotherapie dar. Es gibt darüber hinaus einige wenige Syndrome, bei denen der zugrundeliegende genetische Einflussfaktor auf eine mit Funktionsverlust einhergehende Mutation eines Gens zurück zu führen ist, welches die Reaktion auf Schäden am DNA-Doppelstrang moduliert. Dazu gehört *Ataxia* Telangiectasia, welches durch ein defektes ATM Gen hervorgerufen wird. Eine Gemeinsamkeit all dieser Syndrome ist, dass sie eine extreme Strahlungsempfindlichkeit verursachen (Lavin & Shiloh (1997) Annu. Rev. Immunol. 15: 177; Rotman & Shiloh (1998) Hum. Mol. Genet. 7: 1555). ATM-defektive Zellen sind entsprechend empfindlich gegenüber Mitteln bzw. Maßnahmen, die Schäden am DNA-Doppelstrang hervorrufen, was ATM zu einem attraktiven Target für die Chemo- und Strahlungssensibilisierung bei der Krebsbehandlung macht.

Die zunächst vor diesem Hintergrund untersuchten Moleküle Koffein und Wortmannin zeigten zwar die unter anderem auf eine Inhibierung von ATM zurück geführte Radiosensibilisierung, sie sind jedoch *in vivo* zu toxisch für eine mögliche therapeutische Anwendung. Ausgehend von der chemischen Struktur des P13K-Inhibitors LY294002 wurde von KuDOS Pharmaceuticals der erste potente und selektive ATM Inhibitor entwickelt: KU-55933 (2-Morpholino-6-(thianthren-1-yl)-4H-pyran-4-one). Mit ihm gelang eine Sensibilisierung gegenüber ionisierender Strahlung und DNA-Doppelstrangschädigenden Chemotherapeutika (Hickson, I., et al. (2004).Cancer Res 64, 9152-9159). Allerdings erwies sich KU-55933 mutmaßlich aufgrund hoher Lipophilie als ungeeignet für eine *in vivo* Anwendung. Basierend auf KU-55933 wurden unter leichter Modifizierung des Grundgerüsts KU-60019 (2-((2S,6R)-2,6-Dimethylmorpholino)-N-(5-(6-morpholino-4-oxo-4H-pyran-2-yl)-9H-thioxanthen-2-yl)acetamid) und KU-559403 (2-(4-Methylpiperazin-1-yl)-N-[5-(6-morpholino-4-oxo-pyran-2-yl)thioxanthen-2-yl]acetamid) entwickelt, wobei Löslichkeit und Potenz verbessert werden konnten. Zwischenzeitlich wurde beispielsweise berichtet, dass Glioblastoma-initiierende Zellen durch KU-60019 sicher und effektiv für Bestrahlung sensibilisiert werden konnten, woraus geschlossen wurde, dass KU-60019 für die Strahlungssensibilisierung einer ganzen Reihe von Hirntumoren fungieren kann (Vecchio D. et al. (2015), Int. J. Cancer 136: 1445).

DNA-PK ist eine weitere Serin/Threonin-Proteinkinase aus der PI3K/PI4K-Familie, der auch die Kinase ATM angehört. WO2012/028233 offenbart Imidazo[4,5-c]chinoline, welche DNA-PK hemmen.

Die Bereitstellung von kleinen Molekülen, die die Signaltransduktion von Kinasen, und zwar insbesondere ATM Kinase, wirksam hemmen, regulieren und/oder modulieren, ist daher wünschenswert und Aufgabe der vorliegenden Erfindung.

Weiter ist es wünschenswert, dass derartige Kinase-Inhibitoren selektiv sind, das heißt keine bzw. eine deutlich geringere Aktivität gegenüber anderen Kinasen aufweisen. So können Off-Target-Effekte bzw. damit zusammenhängende Toxizitäten verringert werden.

### BESCHREIBUNG DER ERFINDUNG

Die Aufgabe wurde überraschend gelöst durch Verbindungen gemäß Anspruch 1, und/oder pharmazeutisch verwendbare Salze, Solvate und Tautomere, davon..

Wie nachfolgend detaillierter ausgeführt wird, sind die Verbindungen dahingehend zu verstehen, dass die Atome, wie etwa H, C, N, jeweils auch die schwereren Isotope dieser Atome umfassen. Das gilt insbesondere für H, wobei Deuterium vorteilhaft eingesetzt werden kann, wie durch die Beispiele aufgezeigt wird.

Erfindungsgemäße Verbindungen haben sich überraschend als potente Inhibitoren von ATM Kinase erwiesen. Noch überraschender ist die Selektivität gegenüber anderen verwandten Kinasen, wie etwa mTOR (mammalian target of rapamycin) Kinase, einer weiteren Protein-Kinase aus der Familie der PIK-Kinasen (auch als Klasse IV PI3K bezeichnet).

Ganz im Gegensatz zur vorliegenden Erfindung wird in der internationalen Patentanmeldung WO 2010/139731 für die dort beschriebenen und beanspruchten Imidazolonylchinolin-Verbindungen offenbart, dass es sich bevorzugt um Inhibitoren von Klasse I PI3 Kinasen und/oder mTOR Kinase handelt. Klasse I PI3 Kinasen sind Lipidkinasen. Entsprechend zeigen experimentellen Daten IC₅₀-Werte für mTOR im Bereich bis hinunter zu weniger als 3 nM, also sehr starke Inhibition.

Die erfindungsgemäßen Verbindungen belegen wieder einmal eindrucksvoll, wie auf den ersten Blick relativ gering erscheinende strukturelle Unterschiede einen entscheidenden Einfluss auf die biologische Aktivität ausüben.

Darüber hinaus zeichnen sich die erfindungsgemäßen Verbindungen auch durch das Fehlen der häufig beobachteten, unerwünschten Hemmung kardialer lonenkanäle, insbesondere des Kv1.11 hERG aus, dessen Blockade zu lebensbedrohlichen Arrhytmien führen kann.

Die erfindungsgemäßen Verbindungen eröffnen damit völlig neue Möglichkeiten bei der Krebstherapie, beispielsweise als Monotherapie bei Tumoren mit defektiver DNA-Doppelstrang-Reparaturfähigkeit oder in Kombination mit Radio- oder Chemotherapie, insbesondere als Radio- und Chemosensibilisierer bei der Behandlung von Krebs, besonders bevorzugt als Radiosensibilisierer.

Die erfindungsgemäßen Verbindungen können mithin zur Hemmung von sowie zur Sensibilisierung von Krebszellen gegenüber Antikrebsmitteln und/oder ionisierender Strahlung verwendet werden. Gegenstand der Erfindung ist auch die Verwendung der erfindungsgemäßen Verbindungen bei der Behandlung von Krebs, Tumoren, und/oder Metastasen, in Kombination mit Radiotherapie und/oder einem Antikrebsmittel, bevorzugt Radiotherapie.

Den vorliegend verwendeten Bezeichnungen zur Definition der Verbindungen liegen im Allgemeinen die Regeln der IUPAC-Organisation für chemische Verbindungen und insbesondere organische Verbindungen zugrunde.

Erfindungsgemäße Verbindungen sind die in der nachfolgenden Tabelle 1 aufgeführten Verbindungen, sowie deren pharmazeutisch verwendbaren Salze, Solvate und Tautomere. Tab. 1

| | |
|---|---|
| 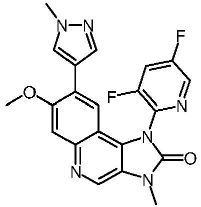 | 1-(3,5-Difluor-pyridin-2-yl)-7-methoxy-3-methyl-8-(1-methyl-1H-pyrazol-4-yl)-1,3-dihydro-imidazo[4,5-c]chinolin-2-on |
| 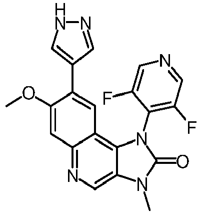 | 1-(3,5-Difluor-pyridin-4-yl)-7-methoxy-3-methyl-8-(1H-pyrazol-4-yl)-1,3-dihydro-imidazo[4,5-c]chinolin-2-on |
| 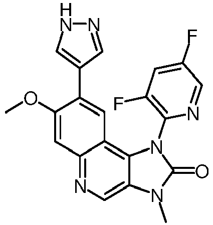 | 1-(3,5-Difluor-pyridin-2-yl)-7-methoxy-3-methyl-8-(1H-pyrazol-4-yl)-1,3-dihydro-imidazo[4,5-c]chinolin-2-on |
| 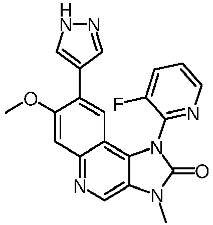 | 1-(3-Fluor-pyridin-2-yl)-7-methoxy-3-methyl-8-(1H-pyrazol-4-yl)-1,3-dihydro-imidazo[4,5-c]chinolin-2-on |
| 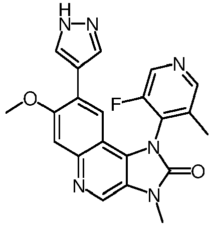 | 1-(3-Fluor-5-methyl-pyridin-4-yl)-7-methoxy-3-methyl-8-(1H-pyrazol-4-yl)-1,3-dihydro-imidazo[4,5-c]chinolin-2-on |
| 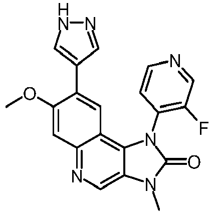 | 1-(3-Fluor-pyridin-4-yl)-7-methoxy-3-methyl-8-(1H-pyrazol-4-yl)-1,3-dihydro-imidazo[4,5-c]chinolin-2-on |
| 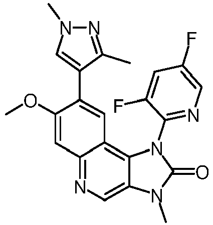 | 1-(3,5-Difluor-pyridin-2-yl)-8-(1,3-dimethyl-1H-pyrazol-4-yl)-7-methoxy-3-methyl-1,3-dihydro-imidazo[4,5-c]chinolin-2-on |
| 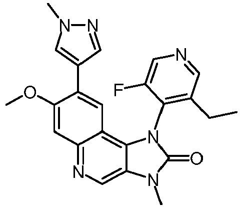 | 1-(3-Ethyl-5-fluor-pyridin-4-yl)-7-methoxy-3-methyl-8-(1-methyl-1H-pyrazol-4-yl)-1,3-dihydro-imidazo[4,5-c]chinolin-2-on |
| 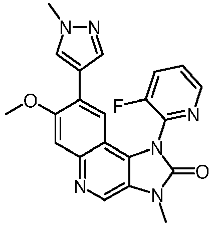 | 1-(3-Fluor-pyridin-2-yl)-7-methoxy-3-methyl-8-(1-methyl-1H-pyrazol-4-yl)-1,3-dihydro-imidazo[4,5-c]chinolin-2-on |
| 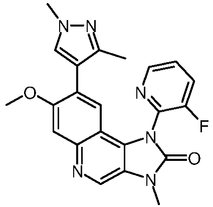 | 8-(1,3-Dimethyl-1H-pyrazol-4-yl)-1-(3-fluor-pyridin-2-yl)-7-methoxy-3-methyl-1,3-dihydro-imidazo[4,5-c]chinolin-2-on |
| 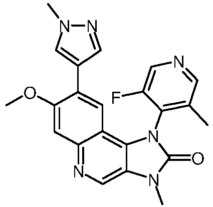 | 1-(3-Fluor-5-methyl-pyridin-4-yl)-7-methoxy-3-methyl-8-(1-methyl-1H-pyrazol-4-yl)-1,3-dihydro-imidazo[4,5-c]chinolin-2-on |
| 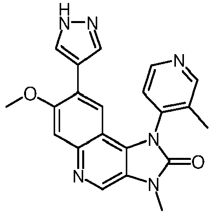 | 7-Methoxy-3-methyl-1-(3-methyl-pyridin-4-yl)-8-(1H-pyrazol-4-yl)-1,3-dihydro-imidazo[4,5-c]chinolin-2-on |
| 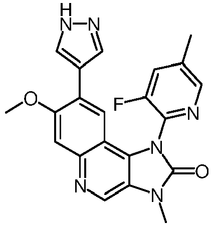 | 1-(3-Fluor-5-methyl-pyridin-2-yl)-7-methoxy-3-methyl-8-(1H-pyrazol-4-yl)-1,3-dihydro-imidazo[4,5-c]chinolin-2-on |
| 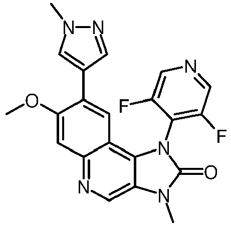 | 1-(3,5-Difluor-pyridin-4-yl)-7-methoxy-3-methyl-8-(1-methyl-1H-pyrazol-4-yl)-1 ,3-dihydro-imidazo[4,5-c]chinolin-2-on |
| 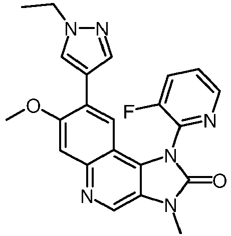 | 8-(1-Ethyl-1H-pyrazol-4-yl)-1-(3-fluor-pyridin-2-yl)-7-methoxy-3-methyl-1,3-dihydro-imidazo[4,5-c]chinolin-2-on |
| 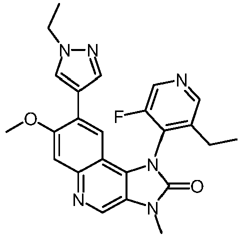 | 1-(3-Ethyl-5-fluor-pyridin-4-yl)-8-(1-ethyl-1H-pyrazol-4-yl)-7-methoxy-3-methyl-1,3-dihydro-imidazo[4,5-c]chinolin-2-on |
| 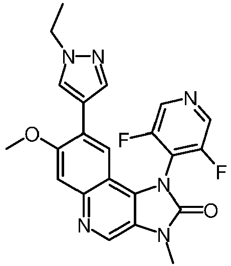 | 1-(3,5-Difluor-pyridin-4-yl)-8-(1-ethyl-1H-pyrazol-4-yl)-7-methoxy-3-methyl-1,3-dihydro-imidazo[4,5-c]chinolin-2-on |
| 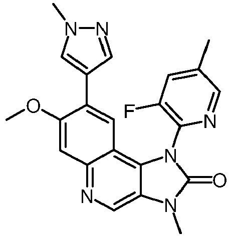 | 1-(3-Fluor-5-methyl-pyridin-2-yl)-7-methoxy-3-methyl-8-(1-methyl-1H-pyrazol-4-yl)-1,3-dihydro-imidazo[4,5-c]chinolin-2-on |
| 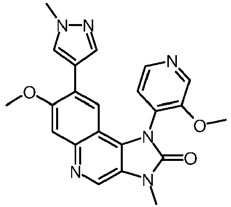 | 7-Methoxy-1-(3-methoxy-pyridin-4-yl)-3-methyl-8-(1-methyl-1H-pyrazol-4-yl)-1,3-dihydro-imidazo[4,5-c]chinolin-2-on |
| 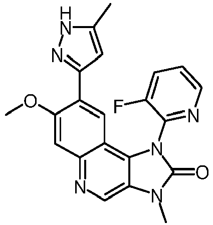 | 1-(3-Fluor-pyridin-2-yl)-7-methoxy-3-methyl-8-(5-methyl-1H-pyrazol-3-yl)-1,3-dihydro-imidazo[4,5-c]chinolin-2-on |
| 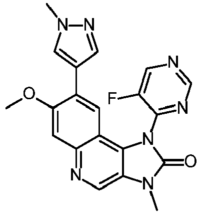 | 1-(5-Fluorpyrimidin-4-yl)-7-methoxy-3-methyl-8-(1-methyl-1H-pyrazol-4-yl)-1H,2H,3H-imidazo[4,5-c]chinolin-2-on |
| 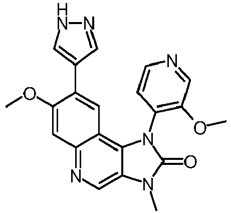 | 7-Methoxy-1-(3-methoxy-pyridin-4-yl)-3-methyl-8-(1H-pyrazol-4-yl)-1,3-dihydro-imidazo[4,5-c]chinolin-2-on |
| 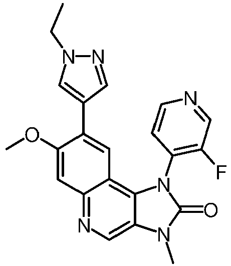 | 8-(1-Ethyl-1H-pyrazol-4-yl)-1-(3-fluor-pyridin-4-yl)-7-methoxy-3-methyl-1,3-dihydro-imidazo[4,5-c]chinolin-2-on |
| 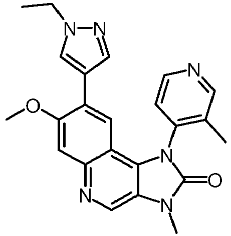 | 8-(1-Ethyl-1H-pyrazol-4-yl)-7-methoxy-3-methyl-1-(3-methyl-pyridin-4-yl)-1,3-dihydro-imidazo[4,5-c]chinolin-2-on |
| 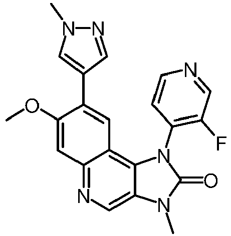 | 1-(3-Fluor-pyridin-4-yl)-7-methoxy-3-methyl-8-(1-methyl-1H-pyrazol-4-yl)-1,3-dihydro-imidazo[4,5-c]chinolin-2-on |
| 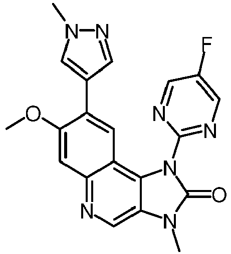 | 1-(5-Fluor-pyrimidin-2-yl)-7-methoxy-3-methyl-8-(1-methyl-1H-pyrazol-4-yl)-1,3-dihydro-imidazo[4,5-c]chinolin-2-on |
| 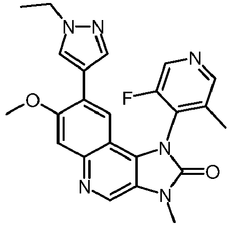 | 8-(1-Ethyl-1H-pyrazol-4-yl)-1-(3-fluor-5-methyl-pyridin-4-yl)-7-methoxy-3-methyl-1,3-dihydro-imidazo[4,5-c]chinolin-2-on |
| 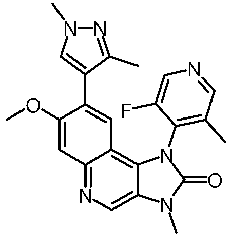 | 8-(1,3-Dimethyl-1H-pyrazol-4-yl)-1-(3-fluor-5-methyl-pyridin-4-yl)-7-methoxy-3-methyl-1,3-dihydro-imidazo[4,5-c]chinolin-2-on |
| 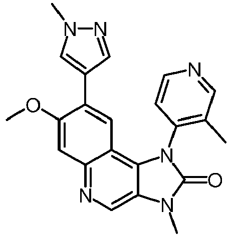 | 7-Methoxy-3-methyl-8-(1-methyl-1H-pyrazol-4-yl)-1-(3-methyl-pyridin-4-yl)-1,3-dihydro-imidazo[4,5-c]chinolin-2-on |
| 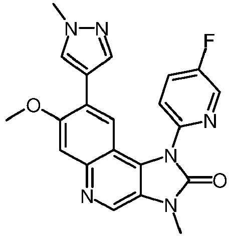 | 1-(5-Fluor-pyridin-2-yl)-7-methoxy-3-methyl-8-(1-methyl-1H-pyrazol-4-yl)-1,3-dihydro-imidazo[4,5-c]chinolin-2-on |
| 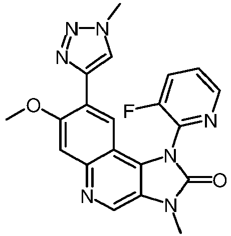 | 1-(3-Fluor-pyridin-2-yl)-7-methoxy-3-methyl-8-(1-methyl-1H-[1,2,3]triazol-4-yl)-1,3-dihydro-imidazo[4,5-c]chinolin-2-on |
| 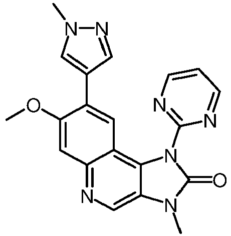 | 7-Methoxy-3-methyl-8-(1-methyl-1H-pyrazol-4-yl)-1-pyrimidin-2-yl-1,3-dihydro-imidazo[4,5-c]chinolin-2-on |
| 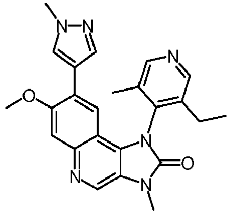 | 1-(3-Ethyl-5-methyl-pyridin-4-yl)-7-methoxy-3-methyl-8-(1-methyl-1H-pyrazol-4-yl)-1,3-dihydro-imidazo[4,5-c]chinolin-2-on |
| 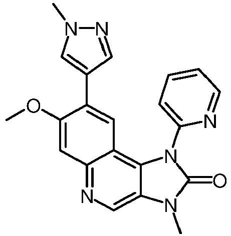 | 7-Methoxy-3-methyl-8-(1-methyl-1H-pyrazol-4-yl)-1-pyridin-2-yl-1,3-dihydro-imidazo[4,5-c]chinolin-2-on |
| 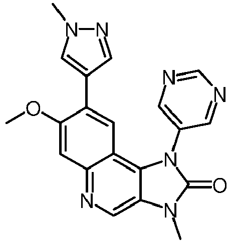 | 7-Methoxy-3-methyl-8-(1-methyl-1H-pyrazol-4-yl)-1-pyrimidin-5-yl-1,3-dihydro-imidazo[4,5-c]chinolin-2-on |
| 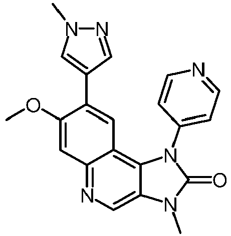 | 7-Methoxy-3-methyl-8-(1-methyl-1H-pyrazol-4-yl)-1-pyridin-4-yl-1,3-dihydro-imidazo[4,5-c]chinolin-2-on |
| 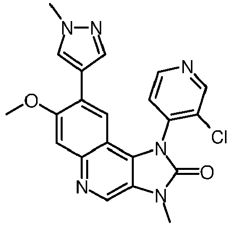 | 1-(3-Chlor-pyridin-4-yl)-7-methoxy-3-methyl-8-(1-methyl-1H-pyrazol-4-yl)-1,3-dihydro-imidazo[4,5-c]chinolin-2-on |
| 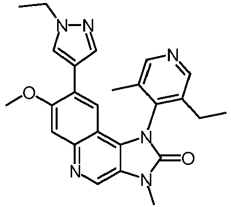 | 1-(3-Ethyl-5-methyl-pyridin-4-yl)-8-(1-ethyl-1H-pyrazol-4-yl)-7-methoxy-3-methyl-1,3-dihydro-imidazo[4,5-c]chinolin-2-on |
| 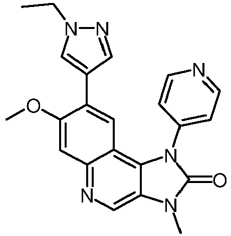 | 8-(1-Ethyl-1H-pyrazol-4-yl)-7-methoxy-3-methyl-1-pyridin-4-yl-1,3-dihydro-imidazo[4,5-c]chinolin-2-on |
| 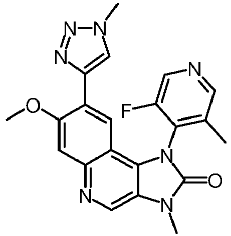 | 1-(3-Fluor-5-methyl-pyridin-4-yl)-7-methoxy-3-methyl-8-(1-methyl-1H-[1,2,3]triazol-4-yl)-1,3-dihydro-imidazo[4,5-c]chinolin-2-on |
| 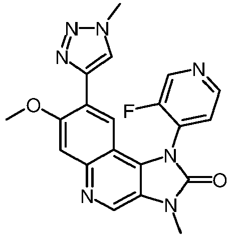 | 1-(3-Fluor-pyridin-4-yl)-7-methoxy-3-methyl-8-(1-methyl-1H-[1,2,3]triazol-4-yl)-1,3-dihydro-imidazo[4,5-c]chinolin-2-on |
| 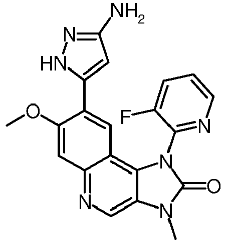 | 8-(3-Amino-1H-pyrazol-5-yl)-1-(3-fluor-2-pyridyl)-7-methoxy-3-methyl-imidazo[4,5-c]chinolin-2-on |
| 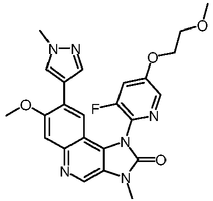 | 1-[3-Fluor-5-(2-methoxyethoxy)-2-pyridyl]-7-methoxy-3-methyl-8-(1-methylpyrazol-4-yl)imidazo[4,5-c]chinolin-2-on |
| 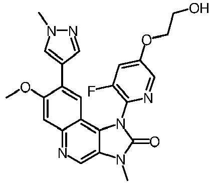 | 1-[3-Fluor-5-(2-hydroxyethoxy)-2-pyridyl]-7-methoxy-3-methyl-8-(1-methylpyrazol-4-yl)imidazo[4,5-c]chinolin-2-on |
| 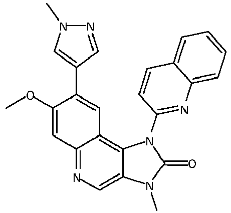 | 7-Methoxy-3-methyl-8-(1-methyl-1H-pyrazol-4-yl)-1-chinolin-2-yl-1,3-dihydro-imidazo[4,5-c]chinolin-2-on |
| 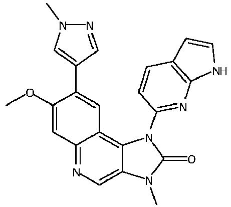 | 7-Methoxy-3-methyl-8-(1-methyl-1H-pyrazol-4-yl)-1-(1H-pyrrolo[2,3-b]pyridin-6-yl)1,3-dihydro-imidazo[4,5-c]chinolin-2-on |
| 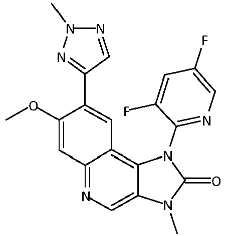 | 1-(3,5-Difluor-pyridin-2-yl)-7-methoxy-3-methyl-8-(2-methyl-2H-[1,2,3]triazol-4-yl)-1,3-dihydro-imidazo[4,5-c] chinolin-2-on |
| 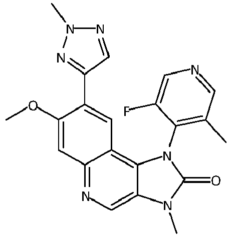 | 1-(3-Fluor-5-methyl-pyridin-4-yl)-7-methoxy-3-methyl-8-(2-methyl-2H-[1,2,3]triazol-4-yl)-1,3-dihydro-imidazo[4,5-c] chinolin-2-on |
| 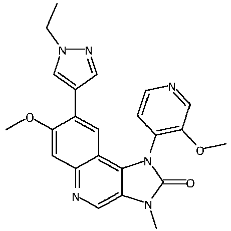 | 8-(1-Ethyl-1H-pyrazol-4-yl)-7-methoxy-1-(3-methoxy-pyridin-4-yl)-3-methyl-1,3-dihydro-imidazo[4,5-c] chinolin-2-on |
| 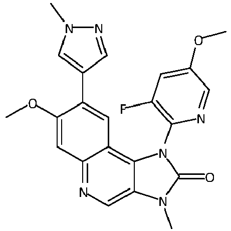 | 1-(3-Fluor-5-methoxy-pyridin-2-yl)-7-methoxy-3-methyl-8-(1-methyl-1H-pyrazol-4-yl)-1,3-dihydro-imidazo[4,5-c]chinolin-2-on |
| 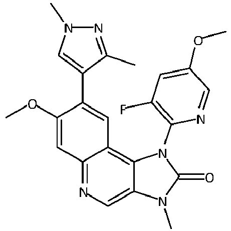 | 8-(1,3-Dimethyl-1H-pyrazol-4-yl)-1-(3-fluor-5-methoxy-pyridin-2-yl)-7-methoxy-3-methyl-1,3-dihydro-imidazo[4,5-c]chinolin-2-on |
| 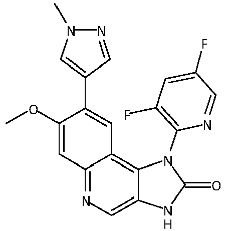 | 1-(3,5-Difluor-pyridin-2-yl)-7-methoxy-8-(1-methyl-1H-pyrazol-4-yl)-1,3-dihydro-imidazo[4,5-c]chinolin-2-on |
| 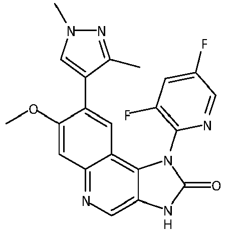 | 1-(3,5-Difluor-pyridin-2-yl)-8-(1,3-dimethyl-1H-pyrazol-4-yl)-7-methoxy-1,3-dihydro-imidazo[4,5-c]chinolin-2-on |
| 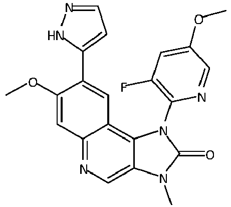 | 1-(3-Fluor-5-methoxy-pyridin-2-yl)-7-methoxy-3-methyl-8-(2H-pyrazol-3-yl)-1,3-dihydro-imidazo[4,5-c] chinolin-2-on |
| 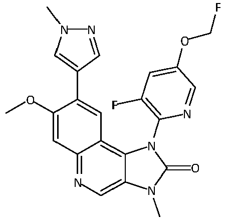 | 1-(3-Fluor-5-fluormethoxy-pyridin-2-yl)-7-methoxy-3-methyl-8-(1-methyl-1H-pyrazol-4-yl)-1,3-dihydro-imidazo[4,5-c]chinolin-2-on |
| 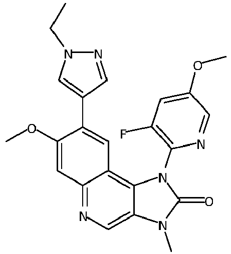 | 8-(1-Ethyl-1H-pyrazol-4-yl)-1-(3-fluor-5-methoxy-pyridin-2-yl)-7-methoxy-3-methyl-1,3-dihydro-imidazo[4,5-c] chinolin-2-on |
| 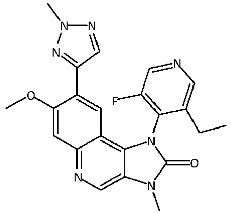 | 1-(3-Ethyl-5-fluor-pyridin-4-yl)-7-methoxy-3-methyl-8-(2-methyl-2H-[1,2,3]triazol-4-yl)-1,3-dihydro-imidazo[4,5-c] chinolin-2-on |
| 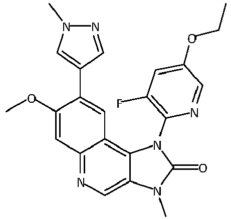 | 1-(5-Ethoxy-3-fluor-pyridin-2-yl)-7-methoxy-3-methyl-8-(1-methyl-1H-pyrazol-4-yl)-1,3-dihydro-imidazo[4,5-c]chinolin-2-on |
| 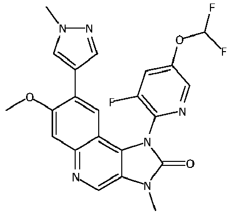 | 1-(5-Difluormethoxy-3-fluor-pyridin-2-yl)-7-methoxy-3-methyl-8-(1-methyl-1H-pyrazol-4-yl)-1,3-dihydro-imidazo[4,5-c]chinolin-2-on |
| 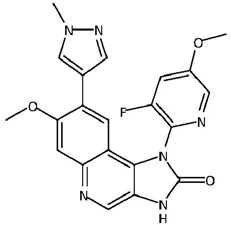 | 1-(3-Fluor-5-methoxy-pyridin-2-yl)-7-methoxy-8-(1-methyl-1H-pyrazol-4-yl)-1 ,3-dihydro-imidazo[4,5-c]chinolin-2-on |
| 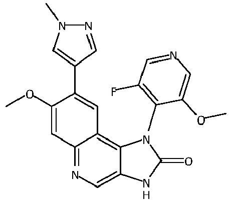 | 1-(3-Fluor-5-methoxy-pyridin-4-yl)-7-methoxy-8-(1-methyl-1H-pyrazol-4-yl)-1 ,3-dihydro-imidazo[4,5-c]chinolin-2-on |
| 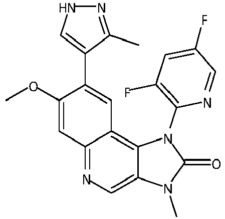 | 1-(3,5-Difluor-pyridin-2-yl)-7-methoxy-3-methyl-8-(3-methyl-1H-pyrazol-4-yl)-1,3-dihydro-imidazo[4,5-c]chinolin-2-on |
| 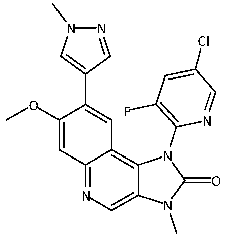 | 1-(5-Chlor-3-fluor-pyridin-2-yl)-7-methoxy-3-methyl-8-(1-methyl-1H-pyrazol-4-yl)-1 ,3-dihydro-imidazo[4,5-c]chinolin-2-on |
| 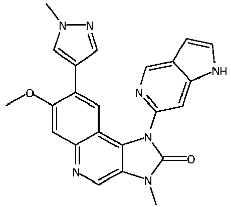 | 7-Methoxy-3-methyl-8-(1-methyl-1H-pyrazol-4-yl)-1-(1H-pyrrolo[3,2-c]pyridin-6-yl)-1,3-dihydro-imidazo[4,5-c]chinolin-2-on |
| 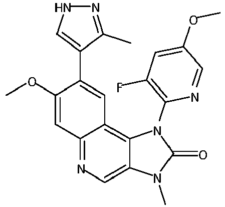 | 1-(3-Fluor-5-methoxy-pyridin-2-yl)-7-methoxy-3-methyl-8-(3-methyl-1H-pyrazol-4-yl)-1,3-dihydro-imidazo[4,5-c] chinolin-2-on |
| 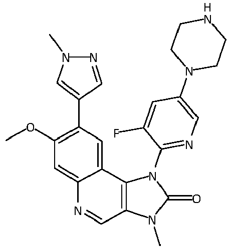 | 1-(3-Fluor-5-piperazin-1-yl-pyridin-2-yl)-7-methoxy-3-methyl-8-(1-methyl-1H-pyrazol-4-yl)-1,3-dihydro-imidazo[4,5-c]chinolin-2-on |
| 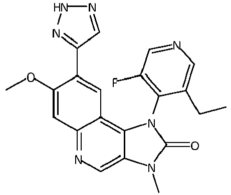 | 1-(3-Ethyl-5-fluor-pyridin-4-yl)-7-methoxy-3-methyl-8-(2H-[1,2,3]triazol-4-yl)-1,3-dihydro-imidazo[4,5-c] chinolin-2-on |
| 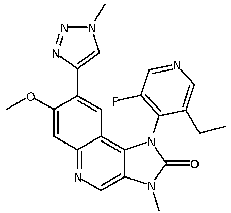 | 1-(3-Ethyl-5-fluor-pyridin-4-yl)-7-methoxy-3-methyl-8-(1-methyl-1H-[1,2,3]triazol-4-yl)-1,3-dihydro-imidazo[4,5-c] chinolin-2-on |
| 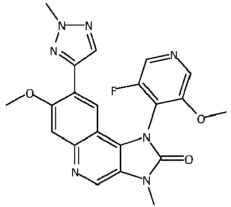 | 1-(3-Fluor-5-methoxy-pyridin-4-yl)-7-methoxy-3-methyl-8-(2-methyl-2H-[1,2,3]triazol-4-yl)-1,3-dihydro-imidazo[4,5-c] chinolin-2-on |
| 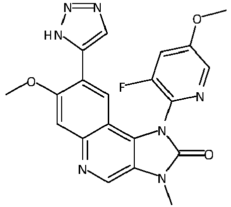 | 1-(3-Fluor-5-methoxy-pyridin-2-yl)-7-methoxy-3-methyl-8-(3H-[1,2,3]triazol-4-yl)-1,3-dihydro-imidazo[4,5-c] chinolin-2-on |
| 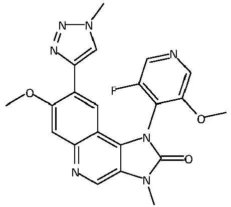 | 1-(3-Fluor-5-methoxy-pyridin-4-yl)-7-methoxy-3-methyl-8-(1-methyl-1H-[1,2,3]triazol-4-yl)-1,3-dihydro-imidazo[4,5-c]chinolin-2-on |
| 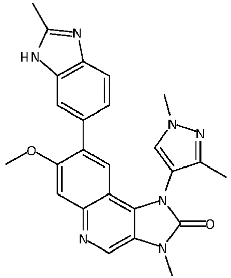 | 1-(1,3-Dimethyl-1H-pyrazol-4-yl)-7-methoxy-3-methyl-8-(2-methyl-3H-benzoimidazol-5-yl)-1,3-dihydro-imidazo[4,5-c]chinolin-2-on |
| 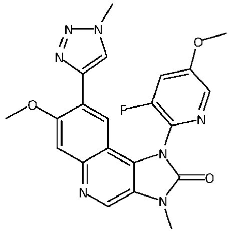 | 1-(3-Fluor-5-methoxy-pyridin-2-yl)-7-methoxy-3-methyl-8-(1-methyl-1H-[1,2,3]triazol-4-yl)-1,3-dihydro-imidazo[4,5-c] chinolin-2-on |
| 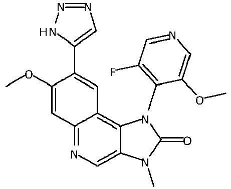 | 1-(3-Fluor-5-methoxy-pyridin-4-yl)-7-methoxy-3-methyl-8-(3H-[1,2,3]triazol-4-yl)-1 ,3-dihydro-imidazo[4,5-c] chinolin-2-on |
| 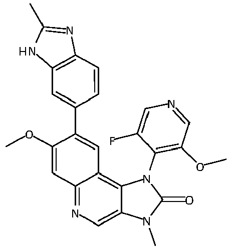 | 1-(3-Fluor-5-methoxy-pyridin-4-yl)-7-methoxy-3-methyl-8-(2-methyl-3H-benzoimidazol-5-yl)-1,3-dihydro-imidazo[4,5-c]chinolin-2-on |
| 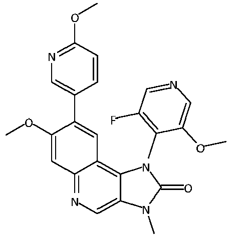 | 1-(3-Fluor-5-methoxy-pyridin-4-yl)-7-methoxy-8-(6-methoxy-pyridin-3-yl)-3-methyl-1,3-dihydro-imidazo[4,5-c]chinolin-2-on |
| 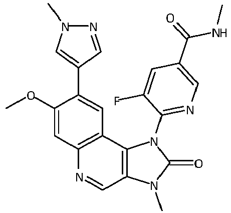 | 5-Fluor-6-[7-methoxy-3-methyl-8-(1-methyl-1H-pyrazol-4-yl)-2-oxo-2,3-dihydro-imidazo[4,5-c]chinolin-1-yl]-N-methyl-nicotinamid |
| 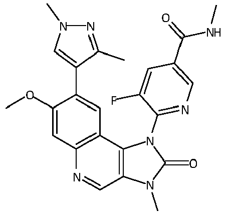 | 6-[8-(1,3-Dimethyl-1H-pyrazol-4-yl)-7-methoxy-3-methyl-2-oxo-2,3-dihydro-imidazo[4,5-c]chinolin-1-yl]-5-fluor-N-methyl-nicotinamid |
| 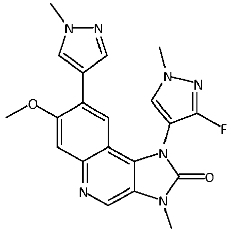 | 1-(3-Fluor-1-methyl-1H-pyrazol-4-yl)-7-methoxy-3-methyl-8-(1-methyl-1H-pyrazol-4-yl)-1,3-dihydro-imidazo[4,5-c]chinolin-2-on |
| 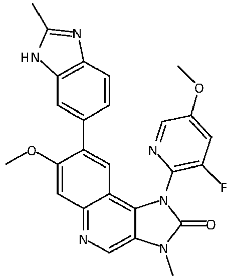 | 1-(3-Fluor-5-methoxy-pyridin-2-yl)-7-methoxy-3-methyl-8-(2-methyl-3H-benzoimidazol-5-yl)-1,3-dihydro-imidazo[4,5-c]chinolin-2-on |
| 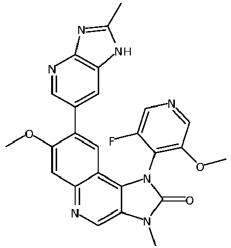 | 1-(3-Fluor-5-methoxy-pyridin-4-yl)-7-methoxy-3-methyl-8-(2-methyl-1H-imidazo[4,5-b]pyridin-6-yl)-1,3-dihydro-imidazo[4,5-c]chinolin-2-on |
| 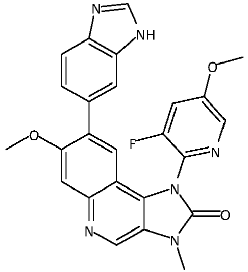 | 8-(3H-Benzimidazol-5-yl)-1-(3-fluor-5-methoxy-pyridin-2-yl)-7-methoxy-3-methyl-1,3-dihydro-imidazo[4,5-c]chinolin-2-on |
| 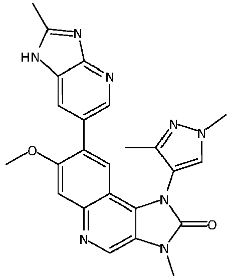 | 1-(1,3-Dimethyl-1H-pyrazol-4-yl)-7-methoxy-3-methyl-8-(2-methyl-1H-imidazo[4,5-b]pyridin-6-yl)-1,3-dihydro-imidazo[4,5-c]chinolin-2-on |
| 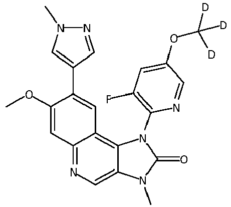 | 1-[3-Fluor-5-(trideuteriomethoxy)-2-pyridyl]-7-methoxy-3-methyl-8-(1-methylpyrazol-4-yl) imidazo[4,5-c]chinolin-2-on |
| 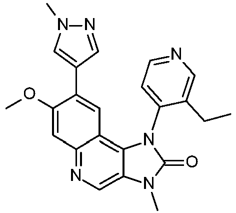 | 1-(3-Ethyl-pyridin-4-yl)-7-methoxy-3-methyl-8-(1-methyl-1H-pyrazol-4-yl)-1,3-dihydro-imidazo[4,5-c] chinolin-2-on |
| 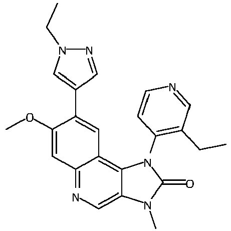 | 8-(1-Ethyl-1H-pyrazol-4-yl)-1-(3-ethyl-pyridin-4-yl)-7-methoxy-3-methyl-1,3-dihydro-imidazo[4,5-c] chinolin-2-on |
| 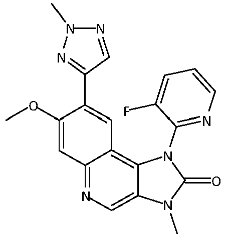 | 1-(3-Fluor-pyridin-2-yl)-7-methoxy-3-methyl-8-(2-methyl-2H-[1,2,3]triazol-4-yl)-1,3-dihydro-imidazo[4,5-c] chinolin-2-on |
| 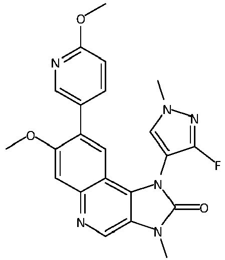 | 1-(3-Fluor-1-methyl-1H-pyrazol-4-yl)-7-methoxy-8-(6-methoxy-pyridin-3-yl)-3-methyl-1,3-dihydro-imidazo[4,5-c]chinolin-2-on |
| 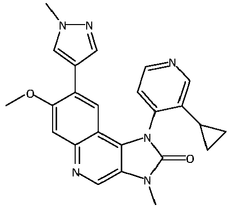 | 1-(3-Cyclopropyl-pyridin-4-yl)-7-methoxy-3-methyl-8-(1-methyl-1H-pyrazol-4-yl)-1 ,3-dihydro-imidazo[4,5-c] chinolin-2-on |
| 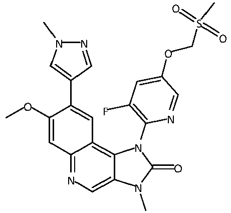 | 1-(3-Fluor-5-methylsulfonylmethoxy-pyridin-2-yl)-7-methoxy-3-methyl-8-(1-methyl-1H-pyrazol-4-yl)-1,3-dihydro-imidazo[4,5-c]chinolin-2-on |
| 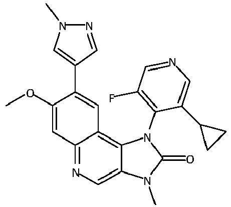 | 1-(3-Cyclopropyl-5-fluor-pyridin-4-yl)-7-methoxy-3-methyl-8-(1-methyl-1H-pyrazol-4-yl)-1,3-dihydro-imidazo[4,5-c]chinolin-2-on |
| 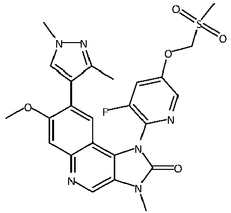 | 8-(1,3-Dimethyl-1H-pyrazol-4-yl)-1-(3-fluor-5-methylsulfonylmethoxy-pyridin-2-yl)-7-methoxy-3-methyl-1,3-dihydro-imidazo[4,5-c]chinolin-2-on |
| 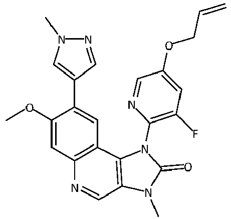 | 1-(5-Allyloxy-3-fluor-pyridin-2-yl)-7-methoxy-3-methyl-8-(1-methyl-1H-pyrazol-4-yl)-1,3-dihydro-imidazo[4,5-c]chinolin-2-on |
| 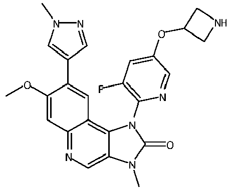 | 1-[5-(Azetidin-3-yloxy)-3-fluor-pyridin-2-yl]-7-methoxy-3-methyl-8-(1-methyl-1H-pyrazol-4-yl)-1,3-dihydro-imidazo[4,5-c]chinolin-2-on |
| 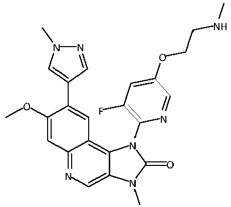 | 1-[3-Fluor-5-(2-methylamino-ethoxy)-pyridin-2-yl]-7-methoxy-3-methyl-8-(1-methyl-1H-pyrazol-4-yl)-1,3-dihydro-imidazo[4,5-c] chinolin-2-on |
| 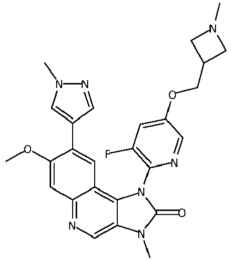 | 1-[3-Fluor-5-(1-methyl-azetidin-3-ylmethoxy)-pyridin-2-yl]-7-methoxy-3-methyl-8-(1-methyl-1H-pyrazol-4-yl)-1 ,3-dihydro-imidazo[4,5-c]chinolin-2-on |
| 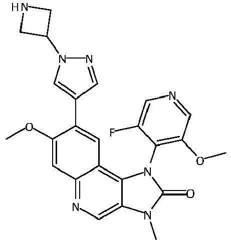 | 8-[1-(Azetidin-3-yl)-1H-pyrazol-4-yl]-1-(3-fluor-5-methoxypyridin-4-yl)-7-methoxy-3-methyl-1H,2H,3H-imidazo[4,5-c]chinolin-2-on |

### Herstellung

Die erfindungsgemäßen Verbindungen und auch die Ausgangsstoffe zu ihrer Herstellung werden nach an sich bekannten Methoden hergestellt, wie sie in der Literatur (z.B. in Standardwerken wie Houben-Weyl, Methoden der organischen Chemie, Georg-Thieme-Verlag, Stuttgart) beschrieben und/oder Fachmann bekannt sind, sowie unter Reaktionsbedingungen, die für die genannten Umsetzungen bekannt und geeignet sind. Dabei kann man auch von an sich bekannten, hier nicht näher erwähnten Varianten Gebrauch machen. Die erfindungsgemäßen Verbindungen können durch geeignete Wahl bzw. Anpassung der Ausgangsmaterialien mittels bzw. analog der in den BEISPIELEN 1 bis 13 detailliert beschriebenen Synthesen hergestellt werden. Für die meisten erfindungsgemäßen Verbindungen kann die in BEISPIEL 1 dargestellte Synthese geeignet abgewandelt werden und damit analog eingesetzt werden. In den entsprechenden Synthesen einsetzbare Amin-Derivate und Boronsäureester oder Analoga sind im Schema 4 unten zusammengefasst. Ergänzend wird auch auf die Offenbarung WO 2010/139731 verwiesen.

Das Verfahren und die anschließende Aufarbeitung des Reaktionsgemisches können grundsätzlich als Batch-Reaktion oder in kontinuierlicher Reaktionsweise durchgeführt werden. Die kontinuierliche Reaktionsweise umfasst z.B. die Reaktion in einem kontinuierlichen Rührkesselreaktor, einer Rührkesselkaskade, einem Schlaufen- oder Querstromreaktor, einem Strömungsrohr oder in einem Mikroreaktor. Die Aufarbeitung der Reaktionsgemische erfolgt wahlweise, je nach Bedarf, durch Filtration über feste Phasen, Chromatographie, Separation zwischen unmischbaren Phasen (z. B. Extraktion), Adsorption an festen Trägern, Abdestillieren von Lösungsmitteln und/oder azeotropen Gemischen, selektive Destillation, Sublimation, Kristallisation, Co-Kristallisation oder durch Nanofiltration an Membranen.

Die Ausgangsverbindungen sind in der Regel bekannt. Sind sie neu, so können sie nach an sich bekannten Methoden hergestellt werden. Falls gewünscht, können die Ausgangsstoffe in-situ gebildet werden, so dass man sie aus dem Reaktionsgemisch nicht isoliert, sondern sofort weiter zu den erfindungsgemäßen Verbindungen umsetzt. Es ist ebenso möglich, die Reaktion schrittweise durchzuführen.

### Pharmazeutisch verwendbare Formen

Im Rahmen der Erfindung sind die erfindungsgemäßen Verbindungen so definiert, dass auch pharmazeutisch verwendbare Salze, Solvate einschl. Hydrate, und Tautomere umfasst sind.

Unter Solvaten der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Die genannten erfindungsgemäßen Verbindungen lassen sich in ihrer endgültigen Nichtsalzform verwenden. Andererseits umfasst die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der erfindungsgemäßen Verbindungen werden größtenteils konventionell hergestellt. Sofern die Verbindungen eine Carbonsäuregruppe enthalten, lässt sich eines ihrer geeigneten Salze dadurch bilden, dass man die Verbindung mit einer geeigneten Base zum entsprechenden Basenadditionssalz umsetzt. Solche Basen sind zum Beispiel Alkalimetallhydroxide (z.B. Kaliumhydroxid, Natriumhydroxid und Lithiumhydroxid), Erdalkalimetallhydroxide (z.B. Bariumhydroxid und Calciumhydroxid), Alkalimetallalkoholate (z.B. Kaliumethanolat und Natriumpropanolat) sowie verschiedene organische Basen wie Piperidin, Diethanolamin und N-Methylglutamin. Eine Base kann mit einer Säure in das zugehörige Säureadditionssalz übergeführt werden, beispielsweise durch Umsetzung äquivalenter Mengen der Base und der Säure in einem inerten Lösungsmittel, wie z.B. Ethanol, und Eindampfen im Anschluss. Für diese Umsetzung kommen insbesondere Säuren in Frage, die physiologisch unbedenkliche Salze liefern, wie z.B. Halogenwasserstoffe (z.B. Chlorwasserstoff, Bromwasserstoff oder Jodwasserstoff), andere Mineralsäuren und ihre entsprechenden Salzen (z.B. Sulfat, Nitrat oder Phosphat und dergleichen), Alkyl- und Monoarylsulfonaten (z.B. Ethansulfonat, Toluolsulfonat und Benzolsulfonat) sowie andere organische Säuren und ihre entsprechenden Salze (z.B. Acetat, Trifluoracetat, Tartrat, Maleat, Succinat, Citrat, Benzoat, Salicylat, Ascorbat und dergleichen. Salze mit physiologisch bedenklichen Säuren, z.B. Pikrate, können zur Isolierung und/oder Aufreinigung der erfindungsgemäßen Verbindungen verwendet werden.

Im Hinblick auf das oben Gesagte sieht man, dass unter dem Ausdruck "pharmazeutisch verwendbares Salz", oder auch "pharmazeutisch unbedenkliches" oder "pharmazeutisch akzeptables" Salz, im vorliegenden Zusammenhang eine erfindungsgemäße Verbindung zu verstehen ist, die in der Form eines ihrer Salze vorliegt, insbesondere dann, wenn diese Salzform der Verbindung im Vergleich zu ihrer freien Form verbesserte pharmakokinetische Eigenschaften verleiht. Die pharmazeutisch unbedenkliche Salzform der Verbindung kann dieser Verbindung auch erst eine gewünschte pharmakokinetische Eigenschaft verleihen und kann sogar die Pharmakodynamik der Verbindung in Bezug auf ihre therapeutische Wirksamkeit im Körper positiv beeinflussen.

Es ist allgemein bekannt, dass Atome atomare Massen oder Massenzahlen haben können, die von den üblicherweise natürlich vorkommenden atomaren Massen oder Massenzahlen abweichen können. Beispiele für Isotope, die kommerziell verfügbar sind und die durch bekannte Methoden in eine erfindungsgemäße Verbindung inkorporiert werden können, sind Isotope von Wasserstoff, Kohlenstoff, Stickstoff, Sauerstoff, Phosphor, Fluor und Chor, bspw. ²H, ³H, ¹³C, ¹⁴C, ¹⁵N, ¹⁸O, ¹⁷O, ³¹P, ³²P, ³⁵S, ¹⁸F und ³⁶Cl. Der Einbau von schwereren Isotopen, insbesondere Deuterium (²H) in eine erfindungsgemäße Verbindung hat therapeutische Vorteile, die in der höheren metabolischen Stabilität dieser isotopenmarkierten Verbindung begründet liegen. Höhere metabolische Stabilität führt unmittelbar zu einer erhöhten *in vivo* Halbwertszeit, was eine geringere Dosierung ermöglicht.

Daher beziehen sich die Definitionen der Atome H, C, N usw., wie in den erfindungsgemäßen Verbindungen benutzt, auch auf die schwereren Isotope dieser Atome.

Erfindungsgemäß besonders bevorzugt ist die Verwendung von D (Deuterium, ²H) anstelle von Wasserstoff (¹H). Deuterium kann beispielsweise mittels eines geeigneten Substituenten an HET in den erfindungsgemäßen Verbindungen eingebaut werden, z.B.Trideuteriomethyl oder Trideuteriomethoxy.

### Verwendung

Es wurde überraschend gefunden, dass die erfindungsgemäßen Verbindungen eine spezifische Hemmung von ATM Kinase, bewirken. Entsprechend stellt die vorliegende Erfindung ATM Inhibitoren bereit, welche aus den erfindungsgemäßen Verbindungen ausgewählt sind.

Im Rahmen der hier vorgestellten Erfindung wurden erstmals neuartige 2,3-Dihydro-*1H-*imidazol[4,5-c]chinolin-Verbindungen bereitgestellt. Die erfindungsgemäßen Verbindungen steuern affin und/oder selektiv ATM Kinase an. Die erfindungsgemäßen Verbindungen zeichnen sich durch eine hohe Spezifität und Stabilität, niedrige Herstellungskosten und leichte Handhabung aus. Solche Eigenschaften bilden die Grundlage für eine reproduzierbaren Wirkungsweise und die verlässliche und sichere Wechselwirkung mit den entsprechenden Zielstrukturen. Die Erfindung beinhaltet auch die Verwendung der vorliegenden 2,3-Dihydro-1H-imidazol[4,5-c]chinolin-Derivate zur Hemmung, Regulation und/oder Modulation der Signalkaskade von ATM Kinase und bietet damit neuartige Werkzeuge für Forschung und/oder Diagnostik.

Die vorliegende Erfindung betrifft die erfindungsgemäßen Verbindungen und/oder ihrer physiologisch unbedenklichen Solvate, Salze, und/oder Tautomere zur Verwendung als Medikament.

Die erfindungsgemäßen Verbindungen und/oder ihre physiologisch unbedenklichen Solvate, Salze, und/oder Tautomere können bei der Behandlung von Erkrankungen, die durch die Aktivität von ATM Kinase, hervorgerufen, vermittelt und/oder verbreitet werden, Verwendung finden.

Zur Identifizierung eines entsprechenden Signalübertragungswegs und zum Nachweis von Wechselwirkungen zwischen verschiedenen Signalübertragungswegen sind geeignete Modelle oder Modellsysteme entwickelt worden, z.B. Zellkulturmodelle (Khwaja et al. (1997) EMBO 16: 2783) und Modelle transgener Tiere (White et al. (2001) Oncogene 20: 7064). Zur Bestimmung bestimmter Stufen in der Signalübertragungskaskade können wechselwirkende Verbindungen genutzt werden, um das Signal zu modulieren (Stephens et al. (2000) Biochemical J 351: 95). Darüber hinaus können die erfindungsgemäßen Verbindungen auch als Reagenzien zur Testung kinaseabhängiger Signalübertragungswege in Tieren und/oder Zellkulturmodellen oder in den in dieser Anmeldung genannten klinischen Erkrankungen verwendet werden. Wie hierin besprochen, sind diese Signalwege für verschiedene Erkrankungen relevant. Dementsprechend sind die erfindungsgemäßen Verbindungen nützlich bei der Prophylaxe, Therapie und/oder Verlaufskontrolle von Erkrankungen, die von Signalwegen mit Beteiligung von ATM Kinase, abhängig sind.

Gegenstand der vorliegenden Erfindung sind auch die erfindungsgemäßen Verbindungen und/oder ihre physiologisch unbedenklichen Solvate, Salze und/oder Tautomere zur Verwendung bei der Behandlung von Krebs, Tumoren, und/oder Metastasen bzw. deren Verwendung bei der Herstellung eines Arzneimittels für ebendiese Verwendungen.

Der Tumor ist insbesondere ausgewählt aus der Gruppe von Erkrankungen von Plattenepithel, Blase, Magen, Nieren, Kopf, Hals, Ösophagus, Gebärmutterhals, Schilddrüse, Darm, Leber, Gehirn, Prostata, Urogenitaltrakts, lymphatisches System, Kehlkopf, Lunge, Haut, Blut und Immunsystem, und/oder der Krebs ist ausgewählt aus der Gruppe von Monozytenleukämie, Lungenadenokarzinom, kleinzelliges Lungenkarzinom, Bauchspeicheldrüsenkrebs, Glioblastom, Darmkarzinom, Brustkarzinom, akute myelotische Leukämie, chronische myelotische Leukämie, akute lymphatische Leukämie, chronische lymphatische Leukämie, Hodgkin-Lymphom und non-Hodgkin-Lymphom.

Erfindungsgemäße Verbindungen und/oder ihre physiologisch unbedenklichen Solvate, Salze und/oder Tautomere können auch zur Verwendung bei der Verlangsamung von Alterungsprozessen geeignet sein, wobei die Verlangsamung anhand des Vergleichs der Lebenspanne des behandelten Wirts oder dessen Zellen, Zellkulturen, Geweben oder Organen mit entsprechenden Positiv- oder Negativkontrollen und/oder Statistiken erfolgt.

Offenbart wird ferner die Behandlung von Krebs, Tumoren, und/oder Metastasen, wobei eine wirksame Menge mindestens einer erfindungsgemäßen Verbindung und/oder ihrer physiologisch unbedenklichen Solvate, Salze und/oder Tautomere einem zu behandelnden Probanden verabreicht wird. Bevorzugte Probanden sind Mensch oder Tier, besonders bevorzugt der Mensch. Die wirksame Menge und die Art der Applikation können vom Fachmann durch Routineversuche bestimmt werden.

Die erfindungsgemäßen Verbindungen, ihre Salze, und/oder Tautomere, sind nicht nur bei den genannten klinischen Krankheitsbildern wirkungsvoll, sondern ebenso in der Diagnose und Therapie sämtlicher Erkrankungen in Zusammenhang mit der ATM-Signalkaskade, vor allem im Hinblick auf die Hemmung von Zellproliferation und -migration.

Darüber hinaus sind die erfindungsgemäßen Inhibitoren in der Behandlung von retroviralen Erkrankungen durch ein Zurückdrängen der retroviralen Integration nutzbar (R. Daniel (1999) Science 284: 644). Schließlich können die erfindungsgemäßen Hemmstoffe als Immunmodulatoren sowie Modulatoren der telomeren Wartung eingesetzt werden.

Offenbart wird weiterhin die Verwendung einer erfindungsgemäßen Verbindung und/oder pharmazeutisch verwendbarem Derivat, Salz, Solvat, Tautomer oder Stereoisomer davon, zur Hemmung von ATM Kinase *in vitro.*

Die genannte Verwendung von erfindungsgemäßen Verbindungen und/oder ihrer pharmazeutisch verwendbaren Solvate, Salze, Tautomere zur Hemmung von ATM Kinase kann in *in vitro* oder *in vivo* Modellen geschehen. Die Suszeptibilität einer bestimmten Zelle gegenüber der Behandlung mit den erfindungsgemäßen Verbindungen kann durch Testen *in vitro* bestimmt werden. Typischerweise wird eine Kultur der Zelle mit einer erfindungsgemäßen Verbindung bei verschiedenen Konzentrationen für eine Zeitdauer inkubiert, die ausreicht, um den aktiven Mitteln zu ermöglichen, Zelltod zu induzieren oder Zellproliferation, Zellvitalität oder Migration zu inhibieren, gewöhnlich zwischen ungefähr einer Stunde und einer Woche. Zum Testen *in vitro* können kultivierte Zellen aus einer Biopsieprobe verwendet werden. Die Menge der nach der Behandlung zurückbleibenden Zellen wird dann bestimmt. Die Verwendung *in vitro* erfolgt insbesondere an Proben von Säugerspezies, die an Krebs, Tumoren, Metastasen, Störungen der Angiogenese, retroviralen Erkrankungen, Immunerkrankungen und/oder pathogenen Alterungsprozessen leiden. Der Wirt oder Patient kann jeglicher Säugerspezies angehören, z. B. einer Primatenspezies, insbesondere Menschen, aber auch Nagetieren (einschließlich Mäusen, Ratten und Hamstern), Kaninchen, Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Das Testen mehrerer spezifischer Verbindungen ermöglicht die Auswahl der Verbindung bzw. des Wirkstoffs, der am besten für die Behandlung des Patienten geeignet erscheint. Die *in vivo* Dosis der gewählten Verbindung wird vorteilhaft auf die Suszeptibilität der Kinase und/oder Schwere der Erkrankung des Patienten mit Blick auf die *in vitro* Daten abgestimmt, wodurch die therapeutische Wirksamkeit merklich erhöht ist. Die Dosis variiert abhängig von der verwendeten spezifischen Verbindung, der spezifischen Erkrankung, dem Patientenstatus usw. Typischerweise ist eine therapeutische Dosis ausreichend, um die unerwünschte Zellpopulation im Zielgewebe erheblich zu vermindern, während die Lebensfähigkeit des Patienten aufrechterhalten wird.

Die hierin offenbarte Lehre der Erfindung und deren Ausführungsformen betreffend die Verwendung von Verbindungen als bzw. für die Herstellung eines Arzneimittels zur Behandlung ist gültig und ohne Einschränkungen auf die Verwendung der Verbindungen zur Hemmung der Kinaseaktivität anwendbar, sofern es sinnvoll erscheint.

Die Behandlung wird im Allgemeinen fortgesetzt, bis eine erhebliche Reduktion vorliegt, z.B. mindestens ca. 50 % Verminderung der Zelllast und kann fortgesetzt werden, bis im Wesentlichen keine unerwünschten Zellen mehr im Körper nachgewiesen werden. In derartigen Tests zeigen und bewirken die erfindungsgemäßen Verbindungen einen inhibierenden Effekt, der gewöhnlich durch IC₅₀-Werte in einem geeigneten Bereich, bevorzugt im mikromolaren Bereich und bevorzugter im nanomolaren Bereich dokumentiert wird. Die Kinase wird insbesondere zu 50 % gehemmt, wenn die Konzentration der Verbindungen kleiner als 1 µM ist, vorzugsweise gleich oder kleiner als 0,5 µM, besonders bevorzugt kleiner als 0,1 µM, 0,05 µM oder 0,001 µM. Diese Konzentration wird als IC₅₀-Wert bezeichnet und bevorzugt mit Hilfe des hierin beschriebenen Assays ermittelt (IC50 ATM). "µM" steht gemäß der üblichen Nomenklatur für Micromol pro Liter, "nM" für Nanomol pro Liter.

### Assays

Die erfindungsgemäßen Verbindungen zeigen eine vorteilhafte biologische Aktivität, die in den hierin beschriebenen Tests nachweisbar ist, wie z.B. auf Enzymen basierenden Assays.

Die Messung der Kinaseaktivität ist eine dem Fachmann wohlbekannte Technik. Generische Testsysteme zur Bestimmung der Kinaseaktivität mit Substraten, z.B. Histon (Alessi et al. (1996) FEBS Lett. 399(3): 333) oder dem basischen Myelinprotein sind in der Literatur beschrieben (Campos-González & Glenney (1992) JBC 267: 14535). Zur Identifikation von Kinase-Inhibitoren stehen verschiedene Assay-Systeme zur Verfügung. Beim Scintillation Proximity Assay (Sorg et al. (2002) J Biomolecular Screening 7: 11) und dem FlashPlate Assay werden die radioaktive Phosphorylierung eines Proteins oder Peptids als Substrat mit ATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al. (2002) J Biomolecular Screening 191). Andere nicht-radioaktive ELISA-Verfahren verwenden spezifische Phospho-Antikörper (Phospho-AK). Der Phospho-AK bindet nur das phosphorylierte Substrat. Diese Bindung ist mit einem zweiten Peroxidase-konjugierten Anti-Schaf-Antikörper durch Chemilumineszens nachweisbar.

Im Rahmen der vorliegenden Erfindung wird die Hemmung (Inhibition) der Aktivität von ATM bevorzugt mit Hilfe des folgenden Assays bestimmt:

### ATM-Kinase Assay - Bestimmung der ATM Inhibition (IC50 ATM):

Der IC₅₀-Wert wurde mit Hilfe eines biochemischen ATM-Kinaseassays bestimmt. Der Assay besteht aus zwei Schritten: der enzymatischen Reaktion und dem Detektionsschritt. Zunächst werden ATM Protein (*Ataxia Telangiectasia* Mutated) und die Testsubstanz in unterschiedlichen Konzentrationen zzgl. Substratprotein p53 und ATP inkubiert. ATM vermittelt die Phosphorylierung von p53 an mehreren Positionen, u.a. an der Aminosäure S15. Die Menge an phosphoryliertem p53 wird mit Hilfe von spezifischen Antikörpern und der TR-FRET-Technik nachgewiesen. Der enzymatische ATM Assay wird als TR-FRET-(HTRF^{™}, Cisbio Bioassays) basierter 384-Well-Assay durchgeführt. Im ersten Schritt wird aufgereinigtes humanes rekombinantes ATM (humane ATM, volle Länge, GenBank-ID NM_000051, exprimiert in einer Säugerzelllinie) in Assaypuffer 15 Minuten lang mit dem ATM Inhibitor in verschiedenen Konzentrationen sowie ohne Testsubstanz als Negativ- bzw. Neutralkontrolle inkubiert. Der Assaypuffer enthält 25 mM HEPES pH 8,0, 10 mM Mg(CH₃COO)₂, 1 mM MnCl₂, 0,1% BSA, 0,01% Brij^{®} 35, 5mM Dithiothreitol (DTT). Die Testsubstanzlösungen wurden mit einem ECHO 555 (Labcyte) in die Mikrotiterplatten dispensiert. Im zweiten Schritt werden aufgereinigtes humanes rekombinantes cmycmarkiertes p53 (humanes p53, volle Länge, GenBank-ID BC003596, exprimiert in Sf21 Insektenzellen) und ATP hinzugefügt und das Reaktionsgemisch 30 - 35 Minuten bei 22°C inkubiert. Das pharmakologisch relevante Assayvolumen beträgt 5 µl. Die finalen Konzentrationen im Assay während der Inkubation des Reaktionsgemisches sind 0,3 - 0,4 nM ATM, 50 - 75 nM p53 und 10 µM ATP. Die enzymatische Reaktion wird durch die Zugabe von EDTA gestoppt. Die Bildung von phosphoryliertem p53 als Resultat der ATMvermittelten Reaktion in Gegenwart von ATP wird über spezifische Antikörper [markiert mit den Fluorophoren Europium (Eu) als Donor und d2 als Akzeptor (Cisbio Bioassays)] nachgewiesen, welche FRET ermöglichen. Hierzu werden 2 µl Antikörper-haltige Stopplösung (12,5 mM HEPES pH 8,0, 125 mM EDTA, 30 mM Natriumchlorid, 300mM Kaliumfluorid, 0,006 % Tween-20, 0,005 % Brij^{®} 35, 0,21 nM anti-phospho-p53(ser15)-Eu Antikörper, 15 nM anti-cmyc-d2 Antikörper) zum Reaktionsgemisch zugegeben. Die Platten werden nach gewöhnlich 2 Stunden Inkubation (zwischen 1,5 und 15h) zur Signalentwicklung in einem Plattenlesegerät (EnVision, PerkinElmer) unter Verwendung des TRF-Modus (sowie mit Laser-Anregung) analysiert. Nach Anregung des Donors Europium bei einer Wellenlänge von 340 nm werden das emittierte Fluoreszenzlicht sowohl des Akzeptors d2 bei 665 nm als auch des Donors Eu bei 615 nm gemessen. Die Menge an phosphoryliertem p53 ist direkt proportional zum Quotienten aus den emittierten Lichtmengen, d.h. den relativen Fluoreszenzeinheiten (RFU) bei 665 nm und 615 nm. Die Messdaten wurden mittels der Genedata Screener Software verarbeitet. IC₅₀ Bestimmungen werden insbesondere durch Anpassen einer Dosiswirkungskurve an die Datenpunkte mittels nichtlinearer Regressionsanalyse bestimmt.
IC₅₀ = halbmaximale inhibitorische Konzentration
ATP = Adenosintriphosphat
TR-FRET = Time-Resolved Fluorescence Resonance Energy Transfer
HTRF^{®} = Homogeneous Time Resolved Fluorescence
HEPES = 2-(4-(2-Hydroxyethyl)-1-piperazinyl)-ethansulfonsäure
Mg(CH₃COO)₂= Magnesiumacetat
MnCl₂ = Mangan(II)-chlorid
BSA = Bovines Serum Albumin
EDTA = Ethylendiamintetraacetat
TRF = Time Resolved Fluorescence

Die Aktivität der erfindungsgemäßen Substanzen in zellulärem Umfeld kann mit Hilfe des folgenden Assays bestimmt werden:

### Zellulärer pCHK2 Assay:

Zur Identifizierung von Substanzen, welche die Phosphorylierung der Proteinkinase CHK2 an der Aminosäure Threonin68 hemmen, wurde in HCT116 Zellen ein Immunfluoreszenzbasierter "high content" Analyse Assay verwendet.

*In vitro* zellbasierter Immunfluoreszenz-Assay zur Identifizierung von Inhibitoren der Bleomycin-induzierten Phosphorylierung von CHK2 (phospho-Thr68) in der humanen Kolonkarzinomzelllinie HCT116:
HCT116 Zellen werden in definierter Zelldichte in 384er Multiwell-Platten in Kulturmedium (DMEM high glucose, 2mM GlutaMax, 1mM Na-Pyruvat, 10% FCS) ausgesät und über Nacht bei 37°C und 10% CO₂ inkubiert. Am Folgetag werden die Testsubstanzen in definiertem Konzentrationsbereich (1nM bis 30µM) in Kombination mit 10µM Bleomycin zugegeben, wobei die Konzentration des Lösungsmittels DMSO konstant bei 0.5% gehalten wird. Nach vier Stunden Inkubation bei 37°C und 10% CO₂ werden die Zellen fixiert (15min 4% Formaldehyd in PBS), permeabilisiert (10min 0.2% Triton X-100 in PBS) und nach Blockierung unspezifischer Bindungsstellen (10% Ziegenserum, 1% BSA in PBS) über Nacht bei 4°C mit einem spezifischen anti-pCHK2 Antikörper inkubiert (Cell Signaling #2661). Der Nachweis von pCHK2 (Thr68) erfolgt mit einem Alexa488-markierten sekundären anti-Kaninchen-IgG-Antikörper. Paralleles Anfärben von DNA mit Propidiumjodid ermöglicht die Zellzahlbestimmung. Die Detektion des pCHK2-Signals erfolgt mit einem high-content-Imager (IMX Ultra von Molecular Devices) und automatische Imageanalyse mit der zum Gerät gehörenden MetaXpress Software. Es wird die Anzahl an Zellkernen bestimmt, die ein pCHK2 Signal über einem definierten Hintergrund aufweisen.

Ferner kann die Wirkung, insbesondere Hemmung, anderer Kinasen und damit die Selektivität der erfindungsgemäßen Verbindungen mit Hilfe der folgenden Assays bestimmt werden:

### mTOR (human)

mTOR (human) wurde mit 50 mM HEPES pH 7.5, 1 mM EGTA, 0.01% Tween 20, 2 mg/mL des Substrates, 3 mM MnCl₂ und [γ-³³P-ATP] (spezifische Aktivität ungefähr 500 cpm/pmol, Konzentration wie erforderlich) inkubiert. Die Reaktion wurde durch Zugabe einer MgATP-Lösung initiiert. Nach einer 40 minütigen Inkubation bei Raumtemperatur wurde die Reaktion durch Zugabe von 3% Phosphorsäure gestoppt. 10 µL der Reaktionslösung wurden dann auf ein P30 Filtermat getropft und drei Mal für 5 min in 75 mM Phosphorsäure und einmal in Methanol gewaschen, getrocknet und mittels Flüssigszintillationszählung ausgewertet.

### PI3K p110α/p85α (human), nicht-radioaktiver Assay

PI3K p110α/p85α (human) wurde in einem Assaypuffer aus 10 µM Phosphatidylinositol-4,5-bisphosphat und MgATP (Konzentration wie erforderlich) inkubiert. Die Reaktion wurde durch Zugabe von ATP-Lösung initiiert. Nach einer 40 minütigen Inkubation bei Raumtemperatur wurde die Reaktion durch Zugabe einer Lösung bestehend aus EDTA und biotinyliertem Phosphatidylinositol-3,4,5-trisphosphat zum Stillstand gebracht. Schließlich wurde der Detektionspuffer, bestehend aus einem Europium-gelabelten anti-GST monoklonalen Antikörper, einer GST-markierten GRP1 PH Domäne und Streptavidin Allophycocyanin, zugefügt. Die Platte wurde über homogene zeitaufgelöste Fluoreszenz (HTRF) ausgelesen und die entsprechenden Signale mit Hilfe der Formel HTRF = 10000 x (Em665nm/Em620nm) ausgewertet.

### PI3K p110β/p85α (human), nicht-radioaktiver Assay

PI3K p110β/p85α (human) wurde in einem Assay Puffer aus 10 µM Phosphatidylinositol-4,5-bisphosphat und MgATP (Konzentration wie erforderlich) inkubiert. Die Reaktion wurde durch Zugabe von MgATP-Lösung initiiert. Nach einer Inkubationszeit von 30 min bei Raumtemperatur wurde die Reaktion durch Zugabe einer Lösung bestehend aus EDTA und biotinyliertem Phosphatidylinositol-3,4,5-trisphosphat zum Stillstand gebracht. Letztendlich wurde der Detektion Puffer, bestehend aus einem Europium-gelabelten anti-GST monoklonalen Antikörper, einer GST-markierten GRP1 PH Domäne und Streptavidin Allophycocyanin, zugefügt. Die Platte wurde über homogene zeitaufgelöste Fluoreszenz (HTRF) ausgelesen und die entsprechenden Signale über die Formel HTRF = 10000 x (Em665nm/Em620nm) ausgewertet.

### PI3K p110δ/p85α (human), nicht-radioaktiver Assay

PI3K p110δ/p85α (human) wurde in einem Assay Puffer aus 10 µM Phosphatidylinositol-4,5-bisphosphat und MgATP (Konzentration wie erforderlich) inkubiert. Die Reaktion wurde durch Zugabe von MgATP-Lösung initiiert. Nach einer Inkubationszeit von 30 min bei Raumtemperatur wurde die Reaktion durch Zugabe einer Lösung bestehend aus EDTA und biotinyliertem Phosphatidylinositol-3,4,5-trisphosphat zum Stillstand gebracht. Letztendlich wurde der Detektion Puffer, bestehend aus einem Europium-gelabelten anti-GST monoklonalen Antikörper, einer GST-markierten GRP1 PH Domäne und Streptavidin Allophycocyanin, zugefügt. Die Platte wurde über homogene zeitaufgelöste Fluoreszenz (HTRF) ausgelesen und die entsprechenden Signale über die Formel HTRF = 10000 x (Em665nm/Em620nm) ausgewertet.

### PI3K (p120γ) (human), nicht-radioaktiver Assay

PI3K (p120γ) (human) wurde in einem Assay Puffer aus 10 µM Phosphatidylinositol-4,5-bisphosphat und MgATP (Konzentration wie erforderlich) inkubiert. Die Reaktion wurde durch Zugabe von MgATP-Lösung initiiert. Nach einer Inkubationszeit von 30 min bei Raumtemperatur wurde die Reaktion durch Zugabe einer Lösung bestehend aus EDTA und biotinyliertem Phosphatidylinositol-3,4,5-trisphosphat zum Stillstand gebracht. Letztendlich wurde der Detektion Puffer, bestehend aus einem Europium-gelabelten anti-GST monoklonalen Antikörper, einer GST-markierten GRP1 PH Domäne und Streptavidin Allophycocyanin, zugefügt. Die Platte wurde über homogene zeitaufgelöste Fluoreszenz (HTRF) ausgelesen und die entsprechenden Signale über die Formel HTRF = 10000 x (Em665nm/Em620nm) ausgewertet.
MgATP = Magnesium 5'-O-[hydroxy({[(hydroxyphosphinato)oxy]phosphinato}oxy)-phos-phoryl]adenosine
MgCl₂= Magnesiumdichlorid
EGTA = Ethylenglycol-bis(aminoethylether)-N,N,N',N'-tetraessigsäure
Tween 20 = Polysorbat 20

### Arzneimittel/Pharmazeutische Zusammensetzung

Gegenstand der Erfindung ist auch ein Arzneimittel bzw. Medikament umfassend mindestens eine erfindungsgemäße Verbindung und/oder ihre physiologisch unbedenklichen Solvate, Salze und/oder Tautomere.

Gegenstand der Erfindung ist ferner eine pharmazeutische Zusammensetzung umfassend eine wirksame Menge von mindestens einer erfindungsgemäßen Verbindung und/oder eines ihrer physiologisch unbedenklichen Salze, Solvate und/oder Tautomere zusammen mit mindestens einem pharmazeutisch verträglichen Hilfsstoff, bzw. einem Träger- und/oder Hilfsstoff.

Ein "Arzneimittel", "Medikament" sowie eine "pharmazeutische Zusammensetzung" oder "pharmazeutische Formulierung" ist zu verstehen als jedes Mittel, welches in der Prophylaxe, Therapie, Verlaufskontrolle oder Nachbehandlung von Patienten eingesetzt werden kann, die zumindest zeitweise eine pathogene Modifikation des Gesamtzustandes bzw. des Zustandes einzelner Teile des Patientenorganismus zeigen, vorzugsweise infolge von Krebs, Tumoren und/oder Metastasen.

Um die protektive oder therapeutische Wirkung der erfindungsgemäßen Verbindungen zu erhöhen, können pharmazeutisch verträgliche Adjuvantien zugesetzt werden. Im Sinne der Erfindung ist jede Substanz, die mit den Verbindungen gemäß der Erfindung eine Wirkung ermöglicht, verstärkt oder modifiziert, ein "Adjuvans". Bekannte Adjuvantien sind z.B. Aluminiumverbindungen, wie z.B. Aluminiumhydroxid oder Aluminiumphosphat, Saponine, wie z.B. QS 21, Muramyldipeptid oder Muramyltripeptid, Proteine, wie z.B. Gammainterferon oder TNF, MF 59, Phosphatdibylcholin, Squalen oder Polyole. Ebenfalls kann die Co-Applikation von Ei-Albumin in komplettem Freund'schen Adjuvans eine gesteigerte zellvermittelte Immunität hervorrufen und somit die Wirkung gebildeter neutralisierender Antikörper unterstützen. Des Weiteren kann DNA, die eine immunstimulatorische Eigenschaft hat, oder die ein Protein mit Adjuvanseffekt kodiert, wie z.B. ein Cytokin, parallel oder in einem Konstrukt appliziert werden.

Das Einbringen des pharmazeutischen Mittels in eine Zelle respektive einen Organismus kann erfindungsgemäß auf jede Art und Weise geschehen, die es ermöglicht, dass die Kinasen mit den in der Zusammensetzung enthaltenen Verbindungen in Kontakt gebracht werden, infolgedessen eine Antwort induziert wird. Das pharmazeutische Mittel der vorliegenden Erfindung kann oral, transdermal, transmucosal, transurethal, vaginal, rektal, pulmonal, enteral und/oder parenteral angewendet werden. Die gewählte Art der Verabreichung richtet sich nach der Indikation, der zu verabreichenden Dosis, Individuumsspezifischen Parametern etc. Insbesondere ermöglichen die verschiedenen Arten der Verabreichung eine ortsspezifische Therapie, die Nebenwirkungen minimiert und die Wirkstoffdosis verringert. Ganz besonders bevorzugte Injektionen sind die intradermale, subkutane, intramuskuläre oder intravenöse Injektion. Die Applikation kann z.B. mit Hilfe so genannter Impfpistolen oder mittels Spritzen geschehen. Es ist auch möglich, die Substanz als Aerosol bereitzustellen, welches von dem Organismus, bevorzugt einem humanen Patienten, inhaliert wird.

Die Darreichungsformen des pharmazeutischen Mittels werden mit den üblichen festen oder flüssigen Trägerstoffen und/oder Verdünnungsmitteln und den üblicherweise eingesetzten Hilfsstoffen entsprechend der gewünschten Applikationsart in einer geeigneten Dosierung und in an sich bekannter Weise hergestellt. Grundsätzlich können also pharmazeutisch annehmbare und dem Fachkundigen bekannte Exzipienten einen Teil des erfindungsgemäßen pharmazeutischen Mittels bilden, wobei die Menge des Exzipientenmaterials, die mit dem Wirkstoff kombiniert wird, um eine Einzeldosierung herzustellen, in Abhängigkeit vom zu behandelnden Individuum und der Art der Verabreichung variiert. Diese pharmazeutisch verträglichen Zusätze umfassen Salze, Puffer, Füllstoffe, Stabilisatoren, Komplexbildner, Antioxidantien, Lösungsmittel, Bindemittel, Gleitmittel, Tabletten-überzüge, Geschmacksstoffe, Farbstoffe, Konservierungsmittel, Stellmittel und dergleichen. Beispiele für solche Exzipienten sind Wasser, pflanzliche Öle, Benzylalkohole, Alkylenglycol, Polyethylenglycol, Kolliphor, Glycerintriacetat, Gelatine, Kohlenhydrate, wie z.B. Laktose oder Stärke, Hydroxypropylmethylcellulose (HPMC), Magnesiumstearat, Talk und Vaseline.

Die pharmazeutische Formulierung kann als Tablette, Filmtablette, Dragee, Lutschtablette, Kapsel, Pille, Pulver, Granulat, Sirup, Saft, Tropfen, Lösung, Dispersion, Suspension, Suppositor, Emulsion, Implantat, Creme, Gel, Salbe, Paste, Lotion, Serum, Öl, Spray, Aerosol, Kleber, Pflaster oder Verband vorliegen. Als orale Darreichungsform werden vorzugsweise Tabletten, Filmtabletten, Dragees, Lutschtabletten, Kapseln, Pillen, Pulver, Granulate, Sirupe, Säfte, Tropfen, Lösungen, Dispersionen oder Suspensionen - auch als Depotform - zubereitet. Des Weiteren sind parenterale Arzneiformen, wie z.B. Suppositorien, Suspensionen, Emulsionen, Implantate oder Lösungen, in Betracht zu ziehen, vorzugsweise ölige oder wässrige Lösungen. Zur topischen Applikation wird der Arzneimittelwirkstoff mit mindestens einem pharmazeutisch annehmbaren Trägerstoff, wie z.B. mikrokristalliner Cellulose, und ggf. weiteren Hilfsstoffen, wie z.B. Feuchtigkeitsspendern, zu auf die Haut auftragbaren festen Formulierungen, wie z.B. Cremes, Gelen, Salben, Pasten, Pulver oder Emulsionen bzw. zu auf die Haut auftragbaren flüssigen Formulierungen, wie z.B. Lösungen, Suspensionen, Lotionen, Seren, Ölen, Sprays oder Aerosole in üblicher Weise formuliert. Vorzugsweise liegt das pharmazeutische Mittel als Injektionslösung vor. Für die Herstellung der Injektionslösung können wässrige Medien, wie z.B. destilliertes Wasser oder physiologische Salzlösungen verwendet werden, wobei letztere saure und basische Additionssalze einschließen. Das pharmazeutische Mittel kann auch als feste Zusammensetzung, beispielsweise im lyophilisierten Zustand vorliegen, und dann durch Zugabe eines auflösenden Mittels, wie z.B. destilliertes Wasser, vor der Verwendung bereitet werden. Die Grundlagen der Herstellung von Lyophilisaten sind dem Fachmann vertraut.

Die Konzentration der aktiven Verbindung in der Formulierung kann 0,1 bis 100 Gewichtsprozente betragen. Entscheidend ist, dass die pharmazeutische Zusammensetzung als Wirkstoff eine effektive Menge der Verbindung zusammen mit den pharmazeutisch verträglichen Hilfsstoffen umfasst. Die Begriffe "effektive Menge", "wirksame Menge" oder "wirksame Dosis" werden hierin austauschbar verwendet und bezeichnen eine Menge des pharmazeutischen Wirkstoffs, die eine prophylaktisch oder therapeutisch relevante Wirkung auf eine Krankheit oder pathologische Veränderung in Zelle, Gewebe, Organ oder Säuger hat. Eine "prophylaktische Wirkung" verhindert das Ausbrechen einer Krankheit oder sogar die Infektion mit einem Pathogen nach dem Eindringen einzelner Vertreter derart, dass deren nachfolgende Ausbreitung stark verringert wird oder sie sogar vollständig inaktiviert werden. Eine "prophylaktische Wirkung" umfasst auch die Erhöhung der normalen physiologischen Funktion. Eine Prophylaxe ist insbesondere ratsam, wenn ein Individuum Veranlagungen für den Ausbruch der vorgenannten Krankheiten aufweist, wie z.B. eine familiäre Vorbelastung, einen Gendefekt oder eine kürzlich überstandene Krankheit. Eine "therapeutisch relevante Wirkung" befreit teilweise oder vollständig von einem, mehreren oder allen Krankheitssymptomen oder führt zur teilweisen oder vollständigen Rückkehr eines, mehrerer oder aller physiologischer oder biochemischer Parameter, die mit der Krankheit oder pathologischen Veränderung in Zusammenhang stehen oder ursächlich dafür sind, in den Normalzustand. Auch wird die Verlaufskontrolle als Art der therapeutischen Behandlung verstanden, wenn die Verbindungen in bestimmten Intervallen verabreicht werden, z.B. um die Symptome einer Krankheit vollständig zu beseitigen. Die jeweilige Dosis bzw. der Dosisbereich für die Gabe der erfindungsgemäßen Verbindungen ist groß genug, um den gewünschten prophylaktischen oder therapeutischen Effekt der Induktion einer biologischen oder medizinischen Antwort zu erreichen. Im Allgemeinen wird die Dosis mit dem Alter, der Konstitution und dem Geschlecht des Patienten variieren sowie die Schwere der Erkrankung berücksichtigen. Es versteht sich, dass die spezifische Dosis, Häufigkeit und Dauer der Verabreichung darüber hinaus von einer Vielzahl an Faktoren abhängen, wie z.B. der Zielsteuerungs- und Bindungsfähigkeit der Verbindungen, Ernährungsgewohnheiten des zu behandelnden Individuums, Art der Verabreichung, Ausscheidungsrate und Kombination mit anderen Medikamenten. Die individuelle Dosis kann sowohl in Bezug auf die primäre Erkrankung als auch in Bezug auf das Eintreten eventueller Komplikationen eingestellt werden. Die exakte Dosis ist durch einen Fachmann mit bekannten Mitteln und Methoden feststellbar. Diese Lehre der Erfindung ist gültig und ohne Einschränkungen auf die pharmazeutische Zusammensetzung umfassend die erfindungsgemäßen Verbindungen anwendbar, sofern es sinnvoll erscheint.

In einer Ausführungsform der Erfindung werden die Verbindungen in einer Dosis von 0,01 mg bis 1 g pro Dosierungseinheit verabreicht, vorzugsweise zwischen 1 bis 700 mg, besonders bevorzugt 5 bis 100 mg. Die tägliche Dosierung liegt insbesondere zwischen 0,02 und 100 mg/kg Körpergewicht.

Aufgrund ihrer überraschend starken und/oder selektiven Kinase-Hemmung, insbesondere Hemmung von ATM-Kinase, die über die Reparatur von Doppelstrang-DNA zelluläre Prozesse regeln, können die Verbindungen der Erfindung in vorteilhaft niedriger Dosierung verabreicht werden, während sie im Vergleich mit weniger potenten bzw. weniger selektiven Inhibitoren eine ähnliche oder sogar überlegene biologische Wirksamkeit erzielen. Eine reduzierte Dosis geht typischerweise mit verringerten medizinischen Nebenwirkungen einher. Darüber hinaus wird eine hoch selektive Hemmung generell auch durch eine Verringerung unerwünschter Nebenwirkungen reflektiert.

Sämtliche genannte sowie weitere Bestandteile bzw. Komponenten eines Medikaments bzw. einer pharmazeutischen Formulierung sind dem Fachmann geläufig und können in Routineversuchen eine spezielle Ausgestaltung für die erfindungsgemäße Lehre erfahren.

### Kombinationstherapie

Arzneimittel und pharmazeutische Zusammensetzungen, die die erfindungsgemäßen Verbindungen enthalten, und der Einsatz dieser Verbindungen zur Behandlung Kinasevermittelter Störungen sind ein vielversprechender Ansatz für ein breites Spektrum von Therapien, wodurch sich bei Mensch und Tier eine direkte und unmittelbare Linderung von Symptomen erreichen lässt. Dies ist besonders für die wirksame Bekämpfung schwerer Krankheiten wie Krebs von Vorteil, entweder als Monotherapie, wie oben geschildert, oder in Kombination mit anderen Therapien, wie beispielsweise Chemo- oder Radiotherapie. Die Schlüsselbeteiligung von ATM an DNA-Reparaturprozessen und der Nachweis, dass ATM-Kinase-Mangel Säugerzellen strahlungsempfindlicher werden lässt, ermöglicht eine therapeutische Anwendung der ATM spezifischen Inhibitoren im Rahmen der Behandlung von z.B. soliden Krebstumoren durch Bestrahlungs- und/oder einer vorzugsweise auf DNA-Doppelstrang-Schäden abzielenden Chemotherapie.

Zur Unterstützung der medizinischen Wirkung kann die pharmazeutische Zusammensetzung entsprechend in einer Ausgestaltung der Erfindung auch einen oder mehrere weitere Wirkstoffe umfassen, beispielsweise ein Antikrebsmittel, wobei eine gleichzeitige oder aufeinander folgende Verabreichung denkbar ist. Die therapeutische Wirkung der erfindungsgemäßen pharmazeutischen Zusammensetzung kann beispielsweise darin bestehen, dass durch die Kinasehemmung bestimmte Antikrebsmittel besser wirken oder durch die Verminderung der Dosis die Anzahl der Nebenwirkungen dieser Medikamente reduziert wird. Entsprechend können die erfindungsgemäßen Verbindungen in Kombination mit anderen Wirkstoffen, einschließlich Antikrebsmitteln, verabreicht werden.

In einer bevorzugten Ausführungsform der Erfindung wird die erfindungsgemäße pharmazeutische Zusammensetzung mit einem Antikrebsmittel kombiniert bzw. umfasst dieses. Entsprechend ist Gegenstand der vorliegenden Erfindung auch eine erfindungsgemäße Verbindung und/oder pharmazeutisch verwendbares Salz, Solvat oder Tautomer davon zur Verwendung bei der Behandlung von Krebs, Tumoren und/oder Metastasen in Kombination mit mindestens einem Antikrebsmittel.

Wie hier verwendet, betrifft der Begriff "Antikrebsmittel" jedes Mittel, das einem Patienten mit Krebs, Tumoren und/oder Metastasen zum Zweck der Behandlung des Krebses verabreicht wird.

Das Antikrebsmittel ist besonders bevorzugt ausgewählt aus der Gruppe umfassend Cytokine, Chemokine, pro-apoptotische Mittel, Interferone, radioaktive Verbindungen, Östrogenrezeptor-Modulatoren, Androgenrezeptor-Modulatoren, Retinoidrezeptor-Modulatoren, Zytotoxika, Zytostatika, Prenyl-Proteintransferase-Hemmer und Angiogenese-Hemmer oder Kombinationen davon. Es ist bevorzugt, dass das Antikrebsmittel den Nukleinsäure- und/oder Proteinstoffwechsel, die Zellteilung, DNA-Replikation, Purin-, Pyrimidin- und/oder Aminosäure-Biosynthese, Genexpression, mRNA-Prozessierung, Proteinsynthese, Apoptose oder Kombinationen davon verändert, insbesondere abschwächt.

Erfindungsgemäß bevorzugte Antikrebsmittel sind solche, die die DNA von Tumorzellen schädigen und damit in DNA-Replikation, DNA-Transkription oder die Genexpression eingreifen. Hierfür kommen insbesondere in Frage:
- Alkylierungsagentien, wie Altretamin, Bendamustin, Busulfan, Carmustin, Chlorambucil, Chlormethine, Cyclophosphamid, Dacarbazin, Ifosfamid, Improsulfantosylat, Lomustin, Melphalan, Mitobronitol, Mitolactol, Nimustin, Ranimustin, Temozolomid, Thiotepa, Treosulfan, Mechloretamin, Carboquon, Apaziquon, Fotemustin, Glufosfamid, Palifosfamid, Pipobroman, Trofosfamid, Uramustin,
- Platinverbindungen, wie Carboplatin, Cisplatin, Eptaplatin, Miriplatinhydrat, Oxaliplatin, Lobaplatin, Nedaplatin, Picoplatin, Satraplatin;
- DDR (DNA damage response) Inhibitoren, wie Topoisomerase-Inhibitoren, z.B. Etoposid, Irinotecan, Razoxan, Sobuzoxan, Topotecan, Camptothecin, Doxorubicin, Amsacrin; Poly-(ADP-ribose)-polymerase (PARP) Inhibitoren, z.B. Talazoparib, Olaparib, Veliparib, Rucaparib, CEP 9722, MK4827, BGB-290; ATR (*Ataxia*telangiectasia and Rad3 related) Inhibitoren, z.B. VE-822, AZ20, AZD6738;
- DNA-verändernde Agentien, wie Amrubicin, Bisantren, Decitabin, Mitoxantron, Procarbazin, Trabectedin, Clofarabin, Amsacrin, Brostallicin, Pixantron, Laromustine;
- Antikrebs-Antibiotica, wie Bleomycin, Dactinomycin, Doxorubicin, Epirubicin, Idarubicin, Levamisol, Miltefosin, Mitomycin C,Romidepsin, Streptozocin, Valrubicin, Zinostatin, Zorubicin, Daunurobicin, Plicamycin, Aclarubicin, Peplomycin, Pirarubicin;
- Alpha-Emitter, wie Alpharadin (²²³Ra Dichlorid, Xofgio), ²¹¹At, ²¹³Bi, ²²⁵Ac, ²²⁷Th.

Besonders bevorzugt sind Bleomycin, Alpharadin, und DDR Inhibitoren, z.B. Etoposid, Irinotecan, Razoxan, Sobuzoxan, Topotecan, Camptothecin, Doxorubicin, Amsacrin; Talazoparib, Olaparib, Veliparib, Rucaparib, CEP 9722, MK4827, BGB-290; VE-822, AZ20, AZD6738.

Die Erfindung kann auch als Kit praktiziert werden, das die erfindungsgemäßen Verbindungen enthält. Das Kit besteht aus getrennten Packungen (a) einer wirksamen Menge einer erfindungsgemäßen Verbindung und/oder ein physiologisch unbedenklichen Salzes, Solvats und/oder Tautomers davon und (b) einer wirksamen Menge eines weiteren Wirkstoffs. Der weitere Wirkstoff ist bevorzugt ein Antikrebsmittel.

Das Kit enthält geeignete Behälter, wie z.B. Schachteln oder Kartons, individuelle Flaschen, Beutel oder Ampullen. Das Kit kann z.B. separate Ampullen enthalten, in denen jeweils eine wirksame Menge einer erfindungsgemäßen Verbindung und/oder ihrer pharmazeutisch verwendbaren Solvate, Salze und/oder Tautomere und einer wirksamen Menge eines weiteren Arzneimittelwirkstoffs gelöst oder in lyophilisierter Form vorliegt. Das Kit der Erfindung kann auch einen Artikel beinhalten, der schriftliche Anweisungen enthält oder den Anwender auf schriftliche Anweisungen hinweist, die den Umgang mit den Verbindungen der Erfindung erläutern.

Eine weitere Ausgestaltung der vorliegenden Erfindung betrifft die erfindungsgemäßen Verbindungen in Kombination mit Radiotherapie und/oder mit mindestens einem weiteren Wirkstoff, vorzugsweise in Kombination mit Radiotherapie und/oder einem Antikrebsmittel. Anders ausgedrückt betrifft eine weitere Ausgestaltung der vorliegenden Erfindung die erfindungsgemäßen Verbindungen zur Verwendung bei der Behandlung von Krebs, Tumoren und/oder Metastasen in Kombination mit Radiotherapie. Ferner ist Gegenstand der vorliegenden Erfindung eine erfindungsgemäße Verbindung und/oder pharmazeutisch verwendbares Salz, Solvat oder Tautomer davon, zur Verwendung bei der Sensibilisierung von Krebszellen gegenüber einem Antikrebsmittel und/oder ionisierender Strahlung.

Technische Bestrahlungsmethoden, die klinisch Verwendung finden, umfassen vorzugsweise Photonenbestrahlung (klassische, elektromagnetische Röntgen-/GammaStrahlung), Protonenbestrahlung, Schwerionenbestrahlung (ionisierter Kohlenstoff) sowie Neutronenbestrahlung, ohne hierauf beschränkt zu sein. Dem Fachmann sind diese Radiotherapien und weitere geeignete Bestrahlungstherapien im Sinne der Erfindung bekannt, wie z.B. aus Herrmann et al. (2006) Klinische Strahlenbiologie, Elsevier München, 4. Auflage, 67-68; Bhide & Nutting (2010) BMC Medicine 8: 25; Choi & Hung (2010) Current Urology Reports 11(3): 172. Als häufigste Anwendung ist die Photonenbestrahlung technisch durch die Verfahren IMRT (Intensitäts-modulierte Radiotherapie) sowie durch bildgebende Verfahren (dreidimensional konforme Radiotherapie) bei der Bestrahlungsplanung und -durchführung zur möglichst exakten Fokussierung verfeinert worden. Die erfindungsgemäßen Verbindungen erzielen bei existierenden Therapien und Bestrahlungen synergistische Effekte und/oder stellen die Wirksamkeit existierender Therapien und Bestrahlungen wieder her.

Die Sensibilisierung erfolgt vorzugsweise *ex vivo* oder *in vitro,* indem die Verbindungen Zellen, Zellkulturen, Geweben oder Organen verabreicht werden, die Serin-Threonin-Proteinkinasen umfassen. Die ex vivo-Verwendung wird insbesondere bei tierischen Zellen angewandt, die aus einem tierischen Organismus stammen, der von einer Krankheit betroffen ist, die ausgewählt ist aus der Gruppe von Krebs, Tumoren, Metastasen und/oder Störungen der Angiogenese. Die ex vivo-behandelten Zellen können entweder für Folgeuntersuchungen weiter in Kultur gehalten oder in ein Tier überführt werden, wobei es sich um das Wirtstier oder ein anderes Tier handeln kann. Die erfindungsgemäße *ex vivo-*Sensibilisierung ist insbesondere von Vorteil, um die spezifische Wirkung der Verbindungen zu testen, so dass unter Auswertung dieser *ex vivo-Daten* die *in vivo-Dosis* entsprechend voreingestellt werden kann. Im Ergebnis wird der therapeutische Effekt signifikant erhöht. Alternativ ist die Erfindung auch für die Anwendung *in vivo* ausgestaltet und betrifft mindestens eine erfindungsgemäße Verbindung und/oder ihre physiologisch unbedenklichen Salze und/oder Tautomere zur Verwendung zur Sensibilisierung von Krebszellen gegenüber einem Antikrebsmittel und/oder ionisierender Strahlung. Zusammenfassend ist festzuhalten, dass die erfindungsgemäßen Verbindungen einzeln und/oder in Kombination mit anderen Behandlungsmaßnahmen verwendet werden können, wie z.B. chirurgischen Eingriffen, Immun-, Strahlen- und/oder Chemotherapie. Letztere beziehen sich auf eine zielgerichtete Therapie mit einem beliebigen NME (d.h. NCE und/oder NBE) als Mono- und/oder On-Target-/Off-Target-Kombinationstherapie.

Wie vorliegend in der Spezifikation einschließlich der anhängiger Ansprüche verwendet, schließen Wortformen im Singular, wie z. B. "ein", "eine", "einer", "der" oder "das" die Entsprechung im Plural ein, sofern der Kontext nicht eindeutig etwas anderes vorgibt. Beispielsweise enthält der Bezug auf "eine Verbindung" eine einzelne Verbindung oder mehrere Verbindungen, die wiederum identisch oder verschieden sein können, oder der Bezug auf "ein Verfahren" schließt äquivalente Schritte und Verfahren ein, die dem Fachmann bekannt sind.

### BEISPIELE

Im Folgenden wird die Erfindung anhand konkreter Ausführungsformen näher erläutert.

### Analytische Methoden

NMR (¹H) wurde mit den folgenden Parametern durchgeführt.
   Geräte: Bruker Avance DRX 500, Bruker Avance 400, Bruker DPX 300
   Standartbedingungen (im Einzelfall abweichend)
   Referenz: TMS
   TD (Time Domaine = Anzahl der Datenpunkte oder digitale Auflösung): 65536
   Lösungsmittel: DMSO-d6
   NS (Number of Scans = Häufigkeit der Abtastung): 32
   SF (Spectrometer Frequence = Sendefrequenz): siehe oben
   TE (Temperatur): 297 K
   Kopplungskonstanten (J) werden in Hertz (Hz) angegeben
HPLC-MS wurde mit den folgenden Parametern durchgeführt.
   Geräte:
      - Shimadzu LCMS-2020,
      - Shimadzu SP-M20A 2010EV
      - Shimadzu UFLC-MS 2010EV
   Verwendete Säulen:
      - Shim-pack VP-ODS,
      - Shim-pack XR-ODS,
      - Kinetex XB-C18 100A,
      - Xbridge BEH C18,
      - Gemini-NX 3u C18 110A
      - ACE UltraCore 2.5 SuperC18
   Methoden: Lösungsmittel-Gradienten mit
      - A: Wasser + 0.1% Ameisensäure, B: Acetonitril + 0.1% Ameisensäure;
      - A :Wasser + 0.05% Trifluoressigsäure, B : Acetonitril + 0.05% Trifluoressigsäure
      - A: Wasser + 5mM Ammoniumcarbonat, B: Acetonitrile
   Detektionswellenlänge: 220 nm
   MS-Typ: API-ES

### Beschreibung der Synthesen

### BEISPIEL 1: Synthese von 1-(3-Fluor-2-pyridyl)-7-methoxy-3-methyl-8-(1-methylpyrazol-4-yl)imidazo[4,5-c]chinolin-2-on (Verbindungsbeispiel 14)

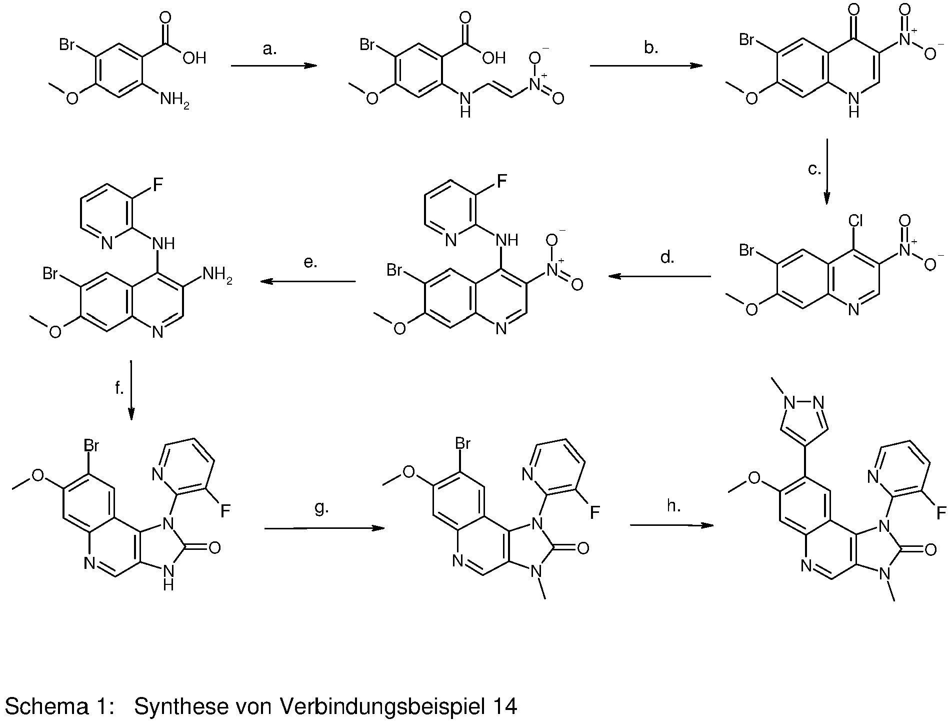

### a. Synthese von 5-Brom-4-methoxy-2-[[(E)-2-nitrovinyl]amino]benzoesäure

Einer Lösung von Natriumhydroxid (14,7 g, 368 mmol) in Wasser (33 mL) wurde unter Rühren Nitromethan (0,63 mL, 11,7 mmol) bei Raumtemperatur zugetropft. Anschließend wurde langsam für 5 Minuten auf 45°C unter Rühren e rwärmt. Die Reaktionslösung wurde danach auf Raumtemperatur abgekühlt und erneut Nitromethan (0,63 mL, 11,7 mmol) zugetropft. Die Reaktionsmischung wurde anschließend für 10 Minuten weitergerührt, wobei eine klare, rötliche Lösung entstand. Nach kurzem Erwärmen (5 Minuten) auf 50°C wurde auf Raumtemperatur abgekühlt und auf Eis (11 g) abdekantiert. Die wässrige Lösung wurde mit konz. Salzsäure vorsichtig auf pH < 2 angesäuert und anschließend sofort einer Lösung aus 2-Amino-5-brom-4-methoxy-benzoesäure (8,00 g, 32,5 mmol) in Wasser (259 mL), angesäuert mit konz. Salzsäure (126 mL, 2,75 mol), unter Rühren zugefügt. Die erhaltene Suspension wurde über Nacht gerührt und anschließend filtriert. Der Rückstand wurde bei 50°getrocknet, wobei 9,60 g (93 %) 5-Brom-4-methoxy-2-[[(E)-2-nitrovinyl]amino]benzoesäure als farbloser Feststoff erhalten wurde.

### b. Synthese von 6-Brom-7-methoxy-3-nitro-1H-chinolin-4-on

5-Brom-4-methoxy-2-((E)-2-nitro-vinylamino)-benzoesäure (4,0 g, 12,6 mmol) wurde in N,N-Dimethylformamid (150 mL) gelöst. Anschließend wurde 1,1'-Carbonyldiimidazol (3,07 g, 18,9 mmol) bei Raumtemperatur unter Rühren zugefügt. Die Reaktionslösung wurde über Nacht bei Raumtemperatur gerührt. Anschließend wurde Acetonitril (120 mL) zugefügt. Die erhaltene Suspension wurde gekühlt und danach abfiltriert. Der gelbe Rückstand wurde mit Diethylether gewaschen und bei 40°C über Nacht getrocknet, wobei 3,0 g (80 %) 6-Brom-7-methoxy-3-nitro-1H-chinolin-4-on als farbloser Feststoff erhalten wurde.

### c. Synthese von 6-Brom-4-chlor-7-methoxy-3-nitro-chinolin

Unter einer trockenen Stickstoffatmosphäre wurde 6-Brom-7-methoxy-3-nitro-1H-chinolin-4-on (2,50 g, 8,36 mmol) vorgelegt. Anschließend wurden Phosphorylchlorid (20 mL, 215 mmol) und N,N-Dimethylformamid (0,13 mL, 1,68 mmol) zugefügt. Die Reaktionslösung wurde 12 Stunden bei 115°C unter Rühren erhitzt. Anschließend wurde im Vakuum eingeengt und der erhaltene Rückstand an Kieselgel chromatographisch (Petrolether/Essigsäureethylester = 87:13, Volumenanteile) aufgereinigt, wobei 2,40 g (90 %) 6-Brom-4-chlor-7-methoxy-3-nitro-chinolin als farbloser Feststoff erhalten wurden.

### d. d. Synthese von 6-Brom-N-(3-fluor-2-pyridyl)-7-methoxy-3-nitro-chinolin-4-amin

Unter einer trockenen Stickstoffatmosphäre wurden 3-Fluorpyridin-2-amin (390 mg, 3,48 mmol), gelöst in N,N-Dimethylformamid (10 mL), vorgelegt. Anschließend wurde der Lösung Natriumhydrid (630 mg, 26,3 mmol) zugefügt und weitere 5 Minuten bei Raumtemperatur gerührt. Danach wurde 6-Brom-4-chlor-7-methoxy-3-nitro-chinolin (1,00 g, 3,15 mmol) zur Reaktionsmischung gegeben, 30 Minuten bei Raumtemperatur gerührt und anschließend durch Zugabe von Eiswasser (100 mL) abgebrochen. Die wässrige Lösung wurde mit Essigsäureethylester dreimal mit je 100 mL extrahiert. Die vereinten organischen Phasen wurden über wasserfreiem Natriumsulfat getrocknet, abfiltriert und im Vakuum zur Trockne eingeengt, wobei 1,0 g (81 %) 6-Brom-N-(3-fluor-2-pyridyl)-7-methoxy-3-nitro-chinolin-4-amin als gelber Feststoff erhalten wurden.

### e. Synthese von 6-Brom-N-4-(3-fluor-2-pyridyl)-7-methoxy-chinolin-3,4-diamin

6-Brom-N-(3-fluor-2-pyridyl)-7-methoxy-3-nitrochinolin-4-amin (1,0 g, 2,54 mmol) wurde unter einer Stickstoffschutzgasatmosphäre in Methanol (50 mL) gelöst vorgelegt. Anschließend wurde der Lösung Raney-Ni (100 mg, 1,17 mmol) zugefügt und die Reaktionsmischung unter einer Wasserstoffatmosphäre 30 Minuten bei Normaldruck gerührt. Nach Belüftung mit Stickstoff, wurde die Suspension filtriert und das Filtrat im Vakuum zur Trockne eingeengt, wobei 0,8 g (87 %) 6-Brom-N-4-(3-fluor-2-pyridyl)-7-methoxy-chinolin-3,4-diamin als gelber Feststoff erhalten wurden.

### f. Synthese von 8-Brom-1-(3-fluor-2-pyridyl)-7-methoxy-3H-imidazo[4,5-c]chinolin-2-on

6-Brom-N-4-(3-fluor-2-pyridyl)-7-methoxy-chinolin-3,4-diamin (0,8 g, 2,20 mmol) wurden in Tetrahydrofuran (25 mL) gelöst vorgelegt. Anschließend wurden 1,1'-Carbonyldiimidazol (1,78 g, 11,0 mmol) und Hünig-Base (1,42 g, 11,0 mmol) zugefügt. Die Reaktionslösung wurde 2 Stunden bei 40°C unter Rühren erwärmt. Danach wurde durch Zugabe von Eiswasser (200 mL) abgebrochen. Die wässrige Phase wurde mit Essigsäureethylester dreimal mit je 50 mL extrahiert. Die vereinten organischen Phasen wurden über wasserfreiem Natriumsulfat getrocknet, abfiltriert und im Vakuum zur Trockne eingeengt, wobei 0,8 g (93 %) 8-Brom-1-(3-fluor-2-pyridyl)-7-methoxy-3H-imidazo[4,5-c]chinolin-2-on als hellgelber Feststoff erhalten wurden.

### g. Synthese von 8-Brom-1-(3-fluor-2-pyridyl)-7-methoxy-3-methyl-imidazo[4,5-c]chinolin-2-on

Unter einer trockenen Stickstoffschutzatmosphäre wurde 8-Brom-1-(3-fluor-2-pyridyl)-7-methoxy-3H-imidazo[4,5-c]chinolin-2-on (0,8 g, 2,06 mmol), gelöst in N,N-Dimethylformamid (10 mL), vorgelegt. Anschließend wurden Natriumhydrid (412 mg, 17,2 mmol) und Methyliodid (1,46 g, 10,3 mmol) zugefügt. Die Reaktionsmischung wurde eine Stunde lang bei Raumtemperatur gerührt. Anschließend wurde durch Zugabe von Eiswasser (100 mL) abgebrochen. Der erhaltene Niederschlag wurde abfiltriert und unter Vakuum getrocknet, wobei 0,6 g (72%) 8-Brom-1-(3-fluor-2-pyridyl)-7-methoxy-3-methyl-imidazo[4,5-c]chinolin-2-on als hellgelber Feststoff erhalten wurden.

### h. Synthese von 1-(3-Fluor-2-pyridyl)-7-methoxy-3-methyl-8-(1-methylpyrazol-4-yl)imidazo[4,5-c]chinolin-2-on (Verbindungsbeispiel 14)

Unter einer Argon-Inertgasatmosphäre wurden in einer geschlossenen Apparatur 8-Brom-1-(3-fluor-2-pyridyl)-7-methoxy-3-methyl-imidazo[4,5-c]chinolin-2-on (150 mg, 0,37 mmol), 1-Methyl-4-(tetramethyl-1,3,2-dioxaborolan-2-yl)-1H-pyrazol (93 mg, 0,45 mmol), Pd(PPh₃)₄ (43 mg, 0,04 mmol) und Kaliumcarbonat (103 mg, 0,75 mmol) in 1,4-Dioxan (10 mL) und Wasser (3 mL) vorgelegt. Die Reaktionsmischung wurde 2 Stunden lang unter Rühren auf 80°C erhitzt. Anschließend wurde auf Raumtemperatur abgekühlt und die Reaktionsmischung im Vakuum zur Trockne eingeengt. Der Rückstand wurde an Kieselgel chromatographisch (Essigsäureethylester/Methanol = 10:1, Volumenanteile) vorgereinigt. Das Eluat wurde zur Trockne eingeengt und das erhaltene Rohprodukt mittels präparativer RP-HPLC (Wasser/Acetonitril) endgereinigt. Nach Einengen der Produktfraktionen wurde 1-(3-Fluor-2-pyridyl)-7-methoxy-3-methyl-8-(1-methylpyrazol-4-yl)imidazo[4,5-c]chinolin-2-on (60 mg, 40%, Verbindungsbeispiel 14) als farbloser Feststoff erhalten.

### BEISPIEL 2: Synthese von 7-Methoxy-3-methyl-8-(1-methylpyrazol-4-yl)-1-thiazol-2-yl-imidazo[4,5-c]chinolin-2-on (Vergleichsbeispiel 47)

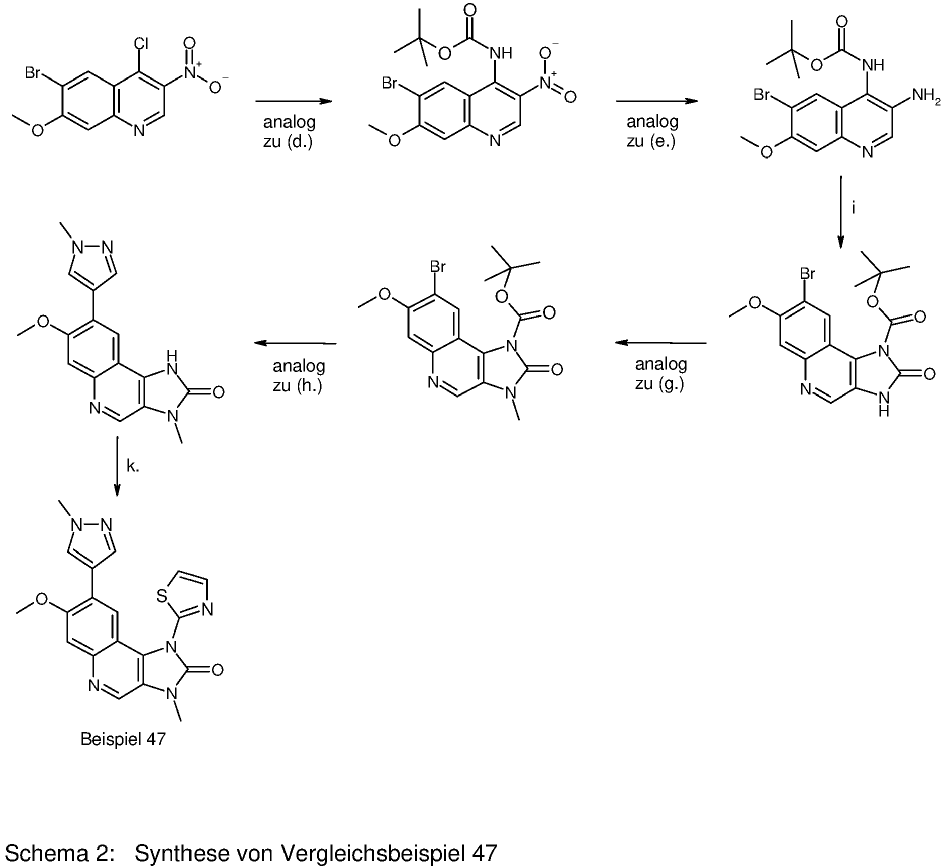

### i. Synthese von tert-Butyl 8-brom-7-methoxy-2-oxo-3H-imidazo[4,5-c]chinolin-1-carboxylat

Tert-Butyl N-(3-amino-6-brom-7-methoxychinolin-4-yl)carbamat (50,0 mg, 0,14 mmol), Ditrichlormethylcarbonat (119 mg, 0,40 mmol) und Triethylamin (0,18 mL, 1,29 mmol) wurden in Dichlormethan (3 mL) gelöst und 2 Stunden lang bei 25°C gerührt. Die Mischung wurde unter Vakuum zur Trockne eingeengt und der Rückstand an Kieselgel chromatographisch aufgereinigt (Essigsäureethylester:Methanol = 10:1, Volumenanteile). Man erhielt 50,0 mg (93 %) tert-Butyl 8-brom-7-methoxy-2-oxo-3H-imidazo[4,5-c]chinolin-1-carboxylat als farblosen Feststoff.

### k. Synthese von 7-Methoxy-3-methyl-8-(1-methylpyrazol-4-yl)-1-thiazol-2-yl-imidazo[4,5-c]chinolin-2-on

Unter Argon wurden in einem verschließbaren Reaktionsgefäß 7-Methoxy-3-methyl-8-(1-methylpyrazol-4-yl)-1H-imidazo[4,5-c]chinolin-2-on (20 mg, 0,06 mmol), 2-Brom-1,3-thiazol (20 mg, 0,12 mmol), Cul (20,00 mg, 0,11 mmol), K₃PO₄ (50 mg, 0,24 mmol) in Toluol (4 mL) aufgenommen. Anschließend wurde bei Raumtemperatur (1S,2S)-1-N,2-N-Dimethylcyclohexan-1,2-diamin (30 mg, 0,21 mmol) zugefügt. Die Mischung wurde unter Rühren 18 Stunden lang auf 100°C erhitzt. Die abgek ühlte Lösung wurde unter Vakuum zur Trockne eingeengt und der Rückstand an Kieselgel chromatographisch aufgereinigt (Wasser/0,05% NH₄HCO₃ : Acetonitril = 10:1). Man erhielt 10 mg (43 %) 7-Methoxy-3-methyl-8-(1-methylpyrazol-4-yl)-1-thiazol-2-yl-imidazo[4,5-c]chinolin-2-on als farblosen Feststoff.

### BEISPIEL 3: Synthese von 1-[5-(Azetidin-3-ylmethoxy)-3-fluor-pyridin-2-yl]-7-methoxy-3-methyl-8-(1-methyl-1H-pyrazol-4-yl)-1,3-dihydro-imidazo[4,5-c]chinolin-2-on

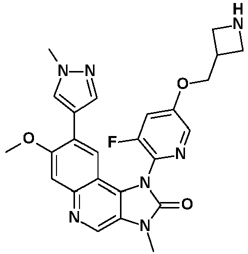

i. In einem 500 mL Rundhalskolben wurden 1-(5-Benzyloxy-3-fluor-2-pyridyl)-7-methoxy-3-methyl-8-(1-methylpyrazol-4-yl)imidazo[4,5-c]chinolin-2-on (1,1 g, 2,15 mmol), MeOH (200 mL), und Pd/C (229 mg, 2,15 mmol) vorgelegt, es wurde mit H₂ gespült und eine H₂-Atmosphäre aufrechterhalten. Die Lösung wurde 8 Stunden lang bei Raumtemperatur gerührt. Feststoff wurde abfiltriert und das Gemisch im Vakuum eingeengt. Man erhielt 740 mg (80%) 1-(3-Fluor-5-hydroxy-2-pyridyl)-7-methoxy-3-methyl-8-(1-methylpyrazol-4-yl)imidazo[4,5-c]chinolin-2-on als weißen Feststoff.
ii. In einem 30 mL Reaktionsgefäß wurden 1-(3-Fluor-5-hydroxy-2-pyridyl)-7-methoxy-3-methyl-8-(1-methylpyrazol-4-yl)imidazo[4,5-c]chinolin-2-on (100 mg, 0,23 mmol, 98%), 3-(Brom-methyl)azetidin-1-tert-butylcarbamat (80 mg, 0,32 mmol), Kaliumcarbonat (66,2 mg, 0,48 mmol) and *N,N*-Dimethylformamid (10 mL) vorgelegt. Die Lösung wurde bei 50°C über Nacht gerührt. Die Reaktion wurde dann durch Zugabe von 10 mL Wasser beendet.
   Die Lösung wurde dann dreimal mit je 10 mL Ethylacetat extrahiert, die vereinten organischen Phasen über wasserfreiem Natriumsulfat getrocknet, abfiltriert und im Vakuum eingeengt. Man erhielt 160 mg (>100%) 3-[[5-fluor-6-[7-methoxy-3-methyl-8-(1-methylpyrazol-4-yl)-2-oxo-imidazo[4,5-c]chinolin-1-yl]-3-pyridyl]oxymethyl]azetidin-1-tert-butylcarbamat als gelbes Öl.
iii. In einem 50 mL Rundkolben wurden 3-[[5-Fluor-6-[7-methoxy-3-methyl-8-(1-methylpyrazol-4-yl)-2-oxo-imidazo[4,5-c]chinolin-1-yl]-3-pyridyl]oxymethyl]azetidin-1-tert-butylcarbamat (160 mg, 0,27 mmol, 99%), Dichlormethan (5 mL) und Trifluoressigsäure (1 mL) vorgelegt. Die Lösung wurde bei 25°C über Nacht gerührt. Das erhaltene Rohprodukt wurde mittels präparativer HPLC aufgereinigt. Man erhielt 30 mg (21%) 1-[5-(Azetidin-3-ylmethoxy)-3-fluor-pyridin-2-yl]-7-methoxy-3-methyl-8-(1-methyl-1H-pyrazol-4-yl)-1,3-dihydro-imidazo[4,5-c]chinolin-2-on als gelben Feststoff.

### BEISPIEL 4: Synthese von 1-(3-Fluor-5-fluormethoxy-pyridin-2-yl)-7-methoxy-3-methyl-8-(1-methyl-1H-pyrazol-4-yl)-1,3-dihydro-imidazo[4,5-c]chinolin-2-on (Verbindungsbeispiel 110)

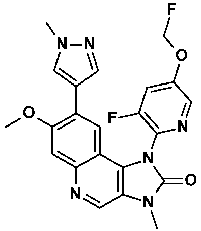

In einem verschlossenen 30 mL Reaktionsgefäß wurden 1-(3-Fluor-5-hydroxy-2-pyridyl)-7-methoxy-3-methyl-8-(1-methylpyrazol-4-yl)imidazo[4,5-c]chinolin-2-on (80 mg, 0,19 mmol, 98%), Kaliumcarbonat (52,4 mg, 0,38 mmol), *N,N*-Dimethylformamid (5 mL) und Bromfluormethan (43,0 mg, 0,36 mmol, 95%) vorgelegt. Die Lösung wurde 2 Stunden lang bei Raumtemperatur gerührt. Dann wurde die Reaktion durch Zugabe von Wasser abgebrochen. Die Lösung wurde dann dreimal mit je 10 mL Ethylacetat extrahiert und die organischen Phasen vereint. Der Rückstand wurde an Kieselgel mit EtOAC/MeOH (97/3) vorgereinigt. Das Rohprodukt wurde mittels präparativer HPLC aufgereinigt. Man erhielt 45 mg (53%) 1-(3-Fluor-5-fluormethoxy-pyridin-2-yl)-7-methoxy-3-methyl-8-(1-methyl-1H-pyrazol-4-yl)-1,3-dihydro-imidazo[4,5-c]chinolin-2-on als weißen Feststoff.

### BEISPIEL 5: Synthese von 1-(5-Difluormethoxy-3-fluor-pyridin-2-yl)-7-methoxy-3-methyl-8-(1-methyl-1H-pyrazol-4-yl)-1,3-dihydro-imidazo[4,5-c]chinolin-2-on (Verbindungsbeispiel 114)

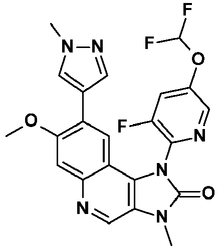

In einem verschlossenen 8 mL Reaktionsgefäß wurden 1-(3-Fluor-5-hydroxy-2-pyridyl)-7-methoxy-3-methyl-8-(1-methylpyrazol-4-yl)imidazo[4,5-c]chinolin-2-on (80 mg, 0,18 mmol, 95%), Kaliumcarbonat (52,5 mg, 0,36 mmol, 95%), *N,N*-Dimethylformamid (5 mL) vorgelegt, es wurde mit Chlordifluormethan gespült und eine Chlordifluormethan-Atmosphäre bei -30°C aufrecht erhalten. Die Lösung wurde 2 Tage lang bei 40°C gerührt. Dann wurde die Reaktion durch Zugabe von 20 mL Eiswasser abgebrochen. Die Lösung wurde dreimal mit je 50 mL Ethylacetat extrahiert und die organischen Phasen vereint. Das Gemisch wurde einmal mit 20 mL Salzlösung gewaschen, über wasserfreiem Natriumsulfat getrocknet und unter Vakuum eingeengt. Das Rohprodukt wurde mittels präparativer HPLC aufgereinigt. Man erhielt 27,7 mg (32%) of 1-(5-Difluormethoxy-3-fluor-pyridin-2-yl)-7-methoxy-3-methyl-8-(1-methyl-1H-pyrazol-4-yl)-1,3-dihydro-imidazo[4,5-c]chinolin-2-on als weißen Feststoff.

### BEISPIEL 6: Synthese von 1-[5-(Dimethyl-phosphinoylmethoxy)-3-fluor-pyridin-2-yl]-8-(1,3-dimethyl-1H-pyrazol-4-yl)-7-methoxy-3-methyl-1,3-dihydro-imidazo[4,5-c]chinolin-2-on

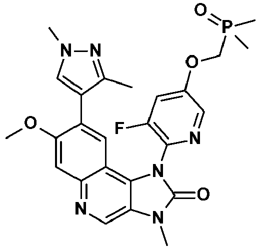

In einem 8 mL Reaktionsgefäß, welches mit Argon gespült und unter Argon-Inertgasatmosphäre gehalten wurde, wurden 8-(1,3-Dimethylpyrazol-4-yl)-1-(3-fluor-5-hydroxy-2-pyridyl)-7-methoxy-3-methyl-imidazo[4,5-c]chinolin-2-on (80 mg, 0,18 mmol, 98%), Chlor-(dimethylphosphoryl)-methan (76,3 mg, 0,54 mmol, 90%), Kaliumcarbonat (50 mg, 0,36 mmol) und *N,N*-Dimethylformamid (5 mL) vorgelegt. Das Reaktionsgemisch wurde 2 Stunden lang bei 180°C erhitzt (Mikrowelle). Die erhaltenen Feststoffe wurden abfiltriert. Das Rohprodukt wurde mittels präparativer HPLC aufgereinigt. Man erhielt 11,7 mg (12%) 1-[5-(Dimethyl-phosphinoylmethoxy)-3-fluor-pyridin-2-yl]-8-(1,3-dimethyl-1H-pyrazol-4-yl)-7-methoxy-3-methyl-1,3-dihydro-imidazo[4,5-c]chinolin-2-on als Feststoff.

### BEISPIEL 7: Synthese von 8-(1,3-Dimethyl-1H-pyrazol-4-yl)-1-(3-fluor-5-methylsulfonylmethoxy-pyridin-2-yl)-7-methoxy-3-methyl-1,3-dihydro-imidazo[4,5-c]chinolin-2-on (Verbindungsbeispiel 201)

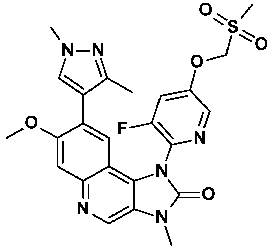

i. In einem 25 mL Rundkolben wurden 8-(1,3-Dimethylpyrazol-4-yl)-1-(3-fluor-5-hydroxy-2-pyridyl)-7-methoxy-3-methyl-imidazo[4,5-c]chinolin-2-on (80 mg, 0,18 mmol, 98%), N,N-Dimethylformamid (5 mL), Kaliumcarbonat (50 mg, 0,36 mmol) und Chlor(methylsulfanyl)methan (110 mg, 1,09 mmol, 95%) vorgelegt. Die Lösung wurde bei 25°C über Nacht gerührt. Dann wurde die Reaktion du rch Zugabe von 50 mL Wasser abgebrochen. Die Lösung wurde viermal mit je 30 mL Ethylacetat extrahiert und die organischen Phasen vereint. Das Gemisch wurde unter Vakuum eingeengt. Der Rückstand wurde an Kieselgel mit MeOH:EtOAc (6:94) vorgereinigt. Man erhielt 80 mg (85%) 8-(1,3-Dimethylpyrazol-4-yl)-1-[3-fluor-5-(methylsulfanylmethoxy)-2-pyridyl]-7-methoxy-3-methyl-imidazo[4,5-c]chinolin-2-on als gelben Feststoff.
ii. In einem 25 mL Rundkolben wurde 8-(1,3-Dimethylpyrazol-4-yl)-1-[3-fluor-5-(methylsulfanylmethoxy)-2-pyridyl]-7-methoxy-3-methyl-imidazo[4,5-c]chinolin-2-on (80 mg, 0,15 mmol, 95%), MeOH (5,00 mL) und eine Lösung von Kaliumperoxodisulfat (220 mg, 0,77 mmol, 95%) in Wasser (0,5 mL) vorgelegt. Die Lösung wurde 3 Stunden lang bei 25°C gerührt. Der Rückstand wurde an Kieselgel mit EtOAc:MeOH (70:30) vorgereinigt und das Rohprodukt mittels präparativer HPLC aufgereinigt. Man erhielt 15 mg (18%) 8-(1,3-Dimethyl-1H-pyrazol-4-yl)-1-(3-fluor-5-methylsulfonylmethoxy-pyridin-2-yl)-7-methoxy-3-methyl-1,3-dihydro-imidazo[4,5-c]chinolin-2-on als weißen Feststoff.

### BEISPIEL 8: Synthese von 1-[3-Fluor-5-(trideuteriomethoxy)-2-pyridyl]-7-methoxy-3-methyl-8-(1-methylpyrazol-4-yl)imidazo[4,5-c]chinolin-2-on (Verbindungsbeispiel 155)

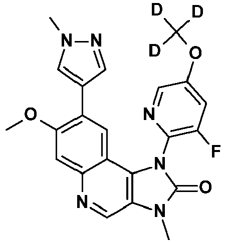

In einem 25 mL Rundkolben wurden 1-(3-Fluor-5-hydroxy-2-pyridyl)-7-methoxy-3-methyl-8-(1-methylpyrazol-4-yl)imidazo[4,5-c]chinolin-2-on (80 mg, 0,19 mmol, 98%), N,N-Dimethylformamid (5,00 mL), Kaliumcarbonat (51,7 mg, 0,37 mmol) und lod(D3)methan (57,1 mg, 0,37 mmol, 95%) vorgelegt. Die Lösung wurde 30 Minuten lang bei Raumtemperatur gerührt. Der Reaktionsfortschritt wurde mittels LCMS verfolgt (Dichlormethan/MeOH = 10:1). Die Reaktion wurde dann durch die Zugabe von 50 mL Eiswasser abgebrochen. Die Lösung wurde dreimal mit je 20 mL Ethylacetat extrahiert und die organischen Phasen vereint. Das Gemisch wurde unter Vakuum eingeengt. Man erhielt 38,5 mg (47%) 1-[3-Fluor-5-(trideuteriomethoxy)-2-pyridyl]-7-methoxy-3-methyl-8-(1-methylpyrazol-4-yl)imidazo[4,5-c]chinolin-2-on als weißen Feststoff.

### BEISPIEL 9: Synthese von 1-(3-Fluor-5-piperazin-1-yl-pyridin-2-yl)-7-methoxy-3-methyl-8-(1-methyl-1H-pyrazol-4-yl)-1,3-dihydro-imidazo[4,5-c]chinolin-2-on (Verbindungsbeispiel 123)

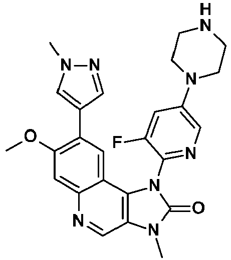

In einem verschlossenen 30 mL Reaktionsgefäß, welches mit Argon gespült und unter Argon-Inertgasatmosphäre gehalten wurde, wurden 1-(5-Chlor-3-fluor-2-pyridyl)-7-methoxy-3-methyl-8-(1-methylpyrazol-4-yl)imidazo[4,5-c]chinolin-2-on (100 mg, 0,22 mmol, 95%), Piperazin-1-tert-butylcarbamat (128 mg, 0,65 mmol, 95%), Pd₂(dba)₃ (21 mg, 0,02 mmol), Xantphos (27 mg, 0,05 mmol), Cs₂CO₃ (150 mg, 0,46 mmol) und Toluol (10 mL) vorgelegt. Das Gemisch wurde bei 90°C über Nacht ge rührt und anschließend unter Vakuum eingeengt. Der Rückstand wurde an Kieselgel mit EtOAc/MeOH (95:5, Volumeneinheiten) gereinigt. Man erhielt 120 mg (94%) 4-[5-Fluor-6-[7-methoxy-3-methyl-8-(1-methylpyrazol-4-yl)-2-oxo-imidazo[4,5-c]chinolin-1-yl]-3-pyridyl]piperazin-1-tert-butylcarbamat als gelben Feststoff.
ii. In einem 25 mL Rundkolben wurden 4-[5-Fluor-6-[7-methoxy-3-methyl-8-(1-methylpyrazol-4-yl)-2-oxo-imidazo[4,5-c]chinolin-1-yl]-3-pyridyl]piperazin-1-tert-butylcarbamat (115 mg, 0,18 mmol, 92%), Dichlormethan (10 mL) und Trifluoressigsäure (4 mL) vorgelegt. Das Gemisch wurde 30 Minuten lang bei 0°C in einem Wasser/Eis-Bad gerührt und unter Vakuum eingeengt. Das Rohprodukt wurde mittels präparativer HPLC aufgereinigt. Man erhielt 50 mg (55%) 1-(3-Fluor-5-piperazin-1-yl-pyridin-2-yl)-7-methoxy-3-methyl-8-(1-methyl-1H-pyrazol-4-yl)-1,3-dihydro-imidazo[4,5-c]chinolin-2-on als weißen Feststoff.

### BEISPIEL 10: Synthese von 5-Fluor-6-[7-methoxy-3-methyl-8-(1-methyl-1H-pyrazol-4-yl)-2-oxo-2,3-dihydro-imidazo[4,5-c]chinolin-1-yl]-N-methyl-nicotinamid (Verbindungsbeispiel 138)

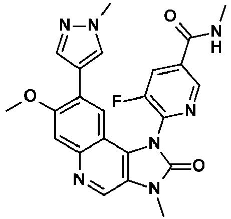

i. In einem 8 mL Reaktionsgefäß wurden eine Lösung von Schwefelsäure (2 mL, 98%) in Wasser (2 mL) und 5-Fluor-6-[7-methoxy-3-methyl-8-(1-methylpyrazol-4-yl)-2-oxo-imidazo[4,5-c]chinolin-1-yl]pyridin-3-nitril (110 mg, 0,26 mmol) vorgelegt. Das Gemisch wurde bei 80°C über Nacht gerührt. Natriumhydroxid (4 mol/L) wurde verwendet, um den pH-Wert auf 8 einzustellen. Das Gemisch wurde unter Vakuum eingeengt und der Rückstand an Kieselgel mit einer 0,5% wäßrigen NH₄HCO₃-Lösung und CH₃CN (82/18) gereinigt. Man erhielt 70 mg (61%) 5-Fluor-6-[7-methoxy-3-methyl-8-(1-methylpyrazol-4-yl)-2-oxo-imidazo[4,5-c]chinolin-1-yl]pyridin-3-carbonsäure als weißen Feststoff.
ii. In einem verschlossenen 30 mL Reaktionsgefäß wurden 5-Fluor-6-[7-methoxy-3-methyl-8-(1-methylpyrazol-4-yl)-2-oxo-imidazo[4,5-c]chinolin-1-yl]pyridin-3-carbonsäure (65,0 mg, 0,14 mmol), eine Lösung von Methylamin (10 mg, 0,32 mmol) in Tetrahydrofuran (0,5 mL), Triethylamin (44 mg, 0,43 mmol), BOP (64,9 mg, 0,15 mmol), und Tetrahydrofuran (5 mL) vorgelegt. Die Lösung wurde 18 Stunden lang bei Raumtemperatur gerührt und unter Vakuum eingeengt, der Rückstand mit 10 mL EtOAc verdünnt. Das Gemisch wurde über wasserfreiem Natriumsulfat getrocknet und unter Vakuum eingeengt. Das Rohprodukt wurde mittels präparativer HPLC aufgereinigt. Man erhielt 20 mg (30%) 5-Fluor-6-[7-methoxy-3-methyl-8-(1-methyl-1H-pyrazol-4-yl)-2-oxo-2,3-dihydro-imidazo[4,5-c]chinolin-1-yl]-N-methyl-nicotinamid als weißen Feststoff.

### BEISPIEL 11: Synthese von 1-[3-Fluor-5-(trideuteriomethoxy)-4-pyridyl]-7-methoxy-3-methyl-8-(1-methylpyrazol-4-yl)imidazo[4,5-c]chinolin-2-on (Vergleichsbeispiel 145)

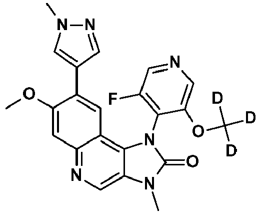

In einem verschlossenen 8 mL Reaktionsgefäß wurden 1-(3-Fluor-5-hydroxy-4-pyridyl)-7-methoxy-3-methyl-8-(1-methylpyrazol-4-yl)imidazo[4,5-c]chinolin-2-on (145 mg, 0,33 mmol, 97%), CD₃OD (0,3 mL), Kaliumcarbonat (143 mg, 1,03 mmol) und *N,N*-Dimethylformamid (3 mL) vorgelegt. Das Gemisch wurde bei 100°C über Nacht gerührt. Die Reaktion wurde dann durch Zugabe von 10 mL Wasser abgebrochen. Die Lösung wurde dreimal mit je 10 mL Ethylacetat extrahiert. Die organischen Phasen wurden vereint, über wasserfreiem Natriumsulfat getrocknet und unter Vakuum eingeengt. Das Rohprodukt wurde mittels präparativer HPLC aufgereinigt. Man erhielt 20 mg (14%) 1-[3-Fluor-5-(trideuteriomethoxy)-4-pyridyl]-7-methoxy-3-methyl-8-(1-methylpyrazol-4-yl)imidazo[4,5-c]chinolin-2-on als weißen Feststoff.

### BEISPIEL 12: Synthese von 1-(3-Fluor-5-methoxy-pyridin-4-yl)-8-(1-fluormethyl-1H-pyrazol-4-yl)-7-methoxy-3-methyl-1,3-dihydro-imidazo[4,5-c]chinolin-2-on (Vergleichsbeispiel 140)

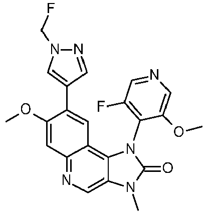

In einem verschlossenen 30 mL Reaktionsgefäß wurden 1-(3-Fluor-5-methoxy-4-pyridyl)-7-methoxy-3-methyl-8-(1H-pyrazol-4-yl)imidazo[4,5-c]chinolin-2-on (60 mg, 0,14 mmol, 97%), Kaliumcarbonat (57,6 mg, 0,42 mmol) und *N,N*-Dimethylformamid (5 mL) vorgelegt. Das Gemisch wurde 5 Minuten lang bei Raumtemperatur gerührt und auf 0°C gekühlt. Bromfluormethan (165 mg, 1.39 mmol, 95%) wurde zugegeben, das Gemisch auf Raumtemperatur erwärmt und eine Stunde lang weiter gerührt. Die Reaktion wurde dann durch die Zugabe von 50 mL Wasser abgebrochen. Die Lösung wurde dreimal mit je 30 mL Ethylacetat extrahiert, die organischen Phasen vereint und unter Vakuum eingeengt. Das Rohprodukt wurde mittels präparativer HPLC aufgereinigt. Man erhielt 5 mg (8%) 1-(3-Fluor-5-methoxy-pyridin-4-yl)-8-(1-fluormethyl-1H-pyrazol-4-yl)-7-methoxy-3-methyl-1,3-dihydro-imidazo[4,5-c]chinolin-2-on als weißen Feststoff.

### BEISPIEL 13: Synthese von 1-(3-Fluor-5-methoxy-4-pyridyl)-7-methoxy-3-methyl-8-(1-methylpyrazol-4-yl)imidazo[4,5-c]chinolin-2-on (Vergleichsbeispiel 4)

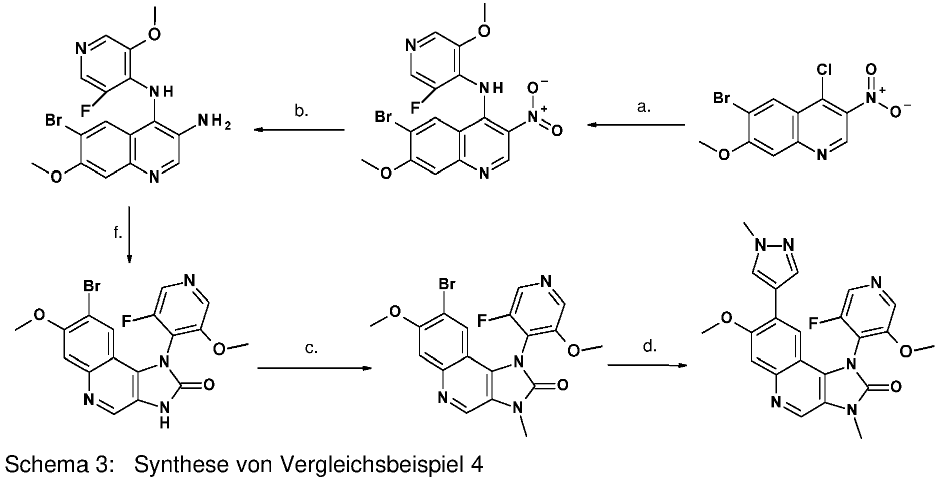

### a. Synthese von 6-Brom-N-(3-fluor-5-methoxy-4-pyridyl)-7-methoxy-3-nitro-chinolin-4-amin

Unter einer trockenen Stickstoffatmosphäre wurden 3-Fluor-5-methoxypyridin-4-amin (447 mg, 3,02 mmol), gelöst in *N,N*-Dimethylformamid (5 mL), vorgelegt. Anschließend wurde der Lösung Natriumhydrid (504 mg, 12,6 mmol, 60%) zugefügt und weitere 5 Minuten bei Raumtemperatur gerührt. Danach wurde 6-Brom-4-chlor-7-methoxy-3-nitro-chinolin (800 mg, 2,52 mmol) zur Reaktionsmischung gegeben, 15 Minuten bei Raumtemperatur gerührt und anschließend durch Zugabe von Eiswasser (100 mL) abgebrochen. Der ausgefallene Niederschlag wurde abfiltriert, mit Eiswasser gewaschen und getrocknet, wobei 1,0 g (94 %) 6-Brom-N-(3-fluor-5-methoxy-4-pyridyl)-7-methoxy-3-nitro-chinolin-4-amin als gelber Feststoff erhalten wurden.

### b. Synthese von 6-Brom-N4-(3-fluor-5-methoxy-4-pyridyl)-7-methoxy-chinolin-3,4-diamin

6-Brom-N-(3-fluor-5-methoxy-4-pyridyl)-7-methoxy-3-nitro-chinolin-4-amin (990 mg, 2,20 mmol) wurde unter einer Stickstoffschutzgasatmosphäre in Methanol (100 mL) gelöst vorgelegt. Anschließend wurde der Lösung Raney-Ni (100 mg, 1,17 mmol) zugefügt und die Reaktionsmischung unter einer Wasserstoffatmosphäre 30 Minuten bei Normaldruck gerührt. Nach Belüftung mit Stickstoff wurde die Suspension filtriert und das Filtrat im Vakuum zur Trockne eingeengt. Der Rückstand wurde aus Ethylacetat/Petrolether kristallisiert, wobei 0,86 g (99 %) 6-Brom-N4-(3-fluor-5-methoxy-4-pyridyl)-7-methoxy-chinolin-3,4-diamin als gelber Feststoff erhalten wurden.

### c. Synthese von 8-Brom-1-(3-fluor-5-methoxy-4-pyridyl)-7-methoxy-3H-imidazo[4,5-c]chinolin-2-on

6-Brom-N4-(3-fluor-5-methoxy-4-pyridyl)-7-methoxy-chinolin-3,4-diamin (0.85 g, 2,20 mmol) wurden in Tetrahydrofuran (20 mL) gelöst vorgelegt. Anschließend wurden 1,1'-Carbonyldiimidazol (1,84 g, 11,3 mmol) und Hünig-Base (1,46 g, 11,3 mmol) zugefügt. Die Reaktionslösung wurde 16 Stunden bei 40°C unter Rüh ren erwärmt. Danach wurde durch Zugabe von Eiswasser (200 mL) abgebrochen. Der ausgefalle Niederschlag wurde abfiltriert, mit Eiswasser gewaschen und getrocknet, wobei 0,87 g (94 %) 8-Brom-1-(3-fluor-5-methoxy-4-pyridyl)-7-methoxy-3H-imidazo[4,5-c]chinolin-2-on als hellgelber Feststoff erhalten wurden.

### d. Synthese von 8-Brom-1-(3-fluor-5-methoxy-4-pyridyl)-7-methoxy-3-methyl-imidazo[4,5-c]chinolin-2-on

Unter einer trockenen Stickstoffschutzatmosphäre wurde 8-Brom-1-(3-fluor-5-methoxy-4-pyridyl)-7-methoxy-3H-imidazo[4,5-c]chinolin-2-on (0,86 g, 1,94 mmol), gelöst in *N,N-*Dimethylformamid (5 mL) vorgelegt. Anschließend wurden Natriumhydrid (388 mg, 9,71 mmol, 60%) und Methyliodid (2,76 g, 19,4 mmol) zugefügt. Die Reaktionsmischung wurde 10 Minuten bei Raumtemperatur gerührt. Anschließend wurde durch Zugabe von Eiswasser (100 mL) abgebrochen. Der erhaltene Niederschlag wurde abfiltriert und unter Vakuum getrocknet, wobei 0,70 g (80%) 8-Brom-1-(3-fluor-5-methoxy-4-pyridyl)-7-methoxy-3-methyl-imidazo[4,5-c]chinolin-2-on als hellgelber Feststoff erhalten wurden.

### e. Synthese von 1-(3-Fluor-5-methoxy-4-pyridyl)-7-methoxy-3-methyl-8-(1-methylpyrazol-4-yl)imidazo[4,5-c]chinolin-2-on (Vergleichsbeispiel 4)

Unter einer Argon-Inertgasatmosphäre wurden in einer geschlossenen Apparatur 8-Brom-1-(3-fluor-5-methoxy-4-pyridyl)-7-methoxy-3-methyl-imidazo[4,5-c]chinolin-2-on (150 mg, 0,33 mmol), 1-Methyl-4-(tetramethyl-1,3,2-dioxaborolan-2-yl)-1H-pyrazol (88,4 mg, 0,44 mmol), Pd(PPh₃)₄ (76,6 mg, 0,07 mmol) und Kaliumcarbonat (91,6 mg, 0,66 mmol) in 1,4-Dioxan (15 mL) und Wasser (5 mL) vorgelegt. Die Reaktionsmischung wurde 2 Stunden unter Rühren auf 80°C erhitzt. Anschließend wurde auf Raumtemperatur abgekühlt und die Reaktionsmischung im Vakuum zur Trockne eingeengt. Der Rückstand wurde an Kieselgel chromatographisch (Essigsäureethylester/Methanol = 97:3, Volumenanteile) vorgereinigt. Das Eluat wurde zur Trockne eingeengt und das erhaltene Rohprodukt mittels präparativer RP-HPLC (Wasser/Acetonitril) endgereinigt. Nach Einengen der Produktfraktionen wurde 1-(3-Fluor-5-methoxy-4-pyridyl)-7-methoxy-3-methyl-8-(1-methylpyrazol-4-yl)imidazo[4,5-c]chinolin-2-on (70 mg, 47%, Verbindungsbeispiel 4) als farbloser Feststoff erhalten.

Die vorstehend benutzten, im Fachbereich üblichen Abkürzungen haben die folgenden Bedeutungen: MeOH: Methanol; Pd(PPh₃)_{4:} Tetrakis(triphenylphosphin)palladium; EtOAc: Ethylacetat; BOP: Benzotriazolyloxytris(dimethylamino)-phosphonium hexafluorophosphat; Pd₂(dba)₃: Tris(dibenzylideneacetone)dipalladium

### BEISPIEL 14: Verbindungsbeispiele, die entsprechend bzw. analog zu den BEISPIELEN 1 bis 13 hergestellt wurden, finden sich in der nachfolgenden Tabelle 2. Die verwendeten Amin-Derivate, Boronsäureester oder Analoga sind im nachstehenden Schema 4 zusammengefasst.

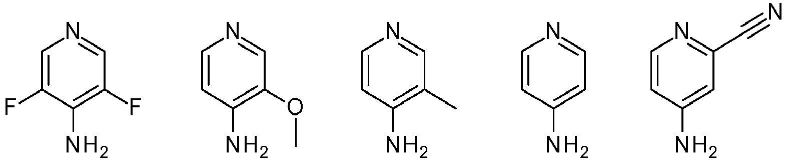

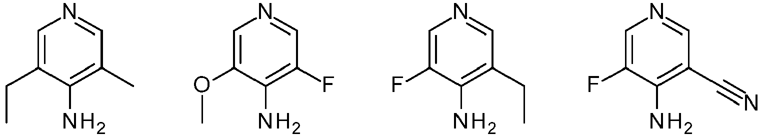

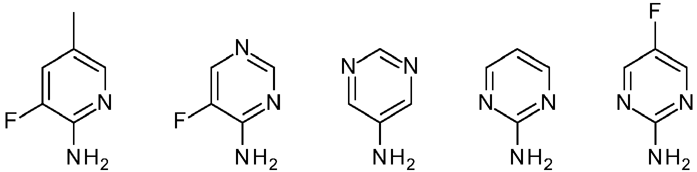

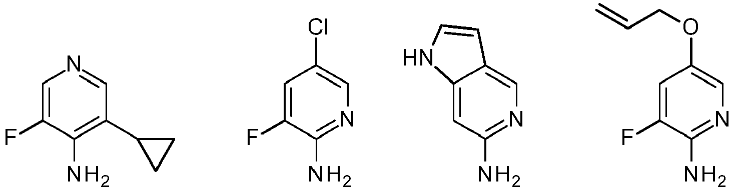

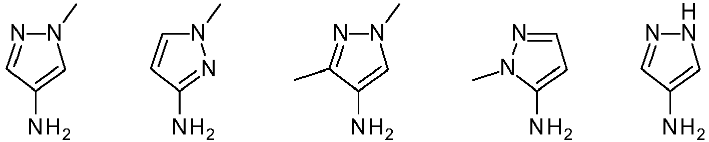

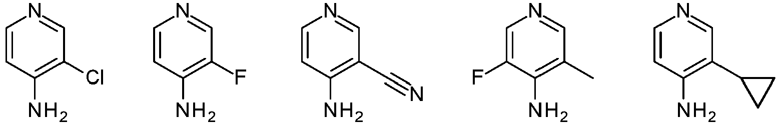

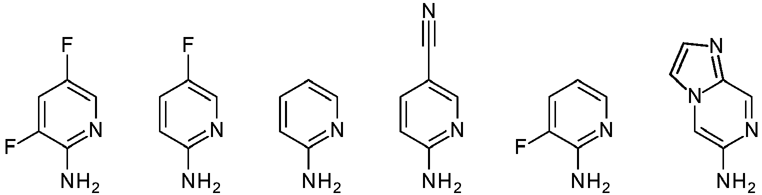

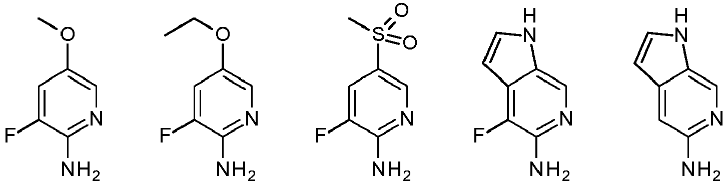

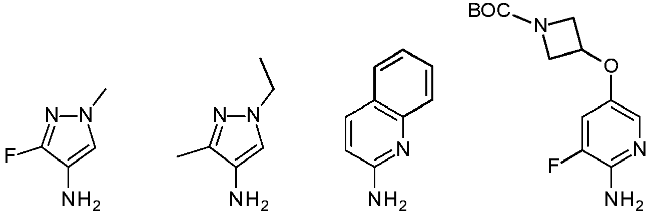

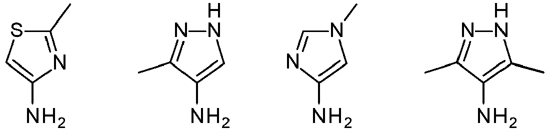

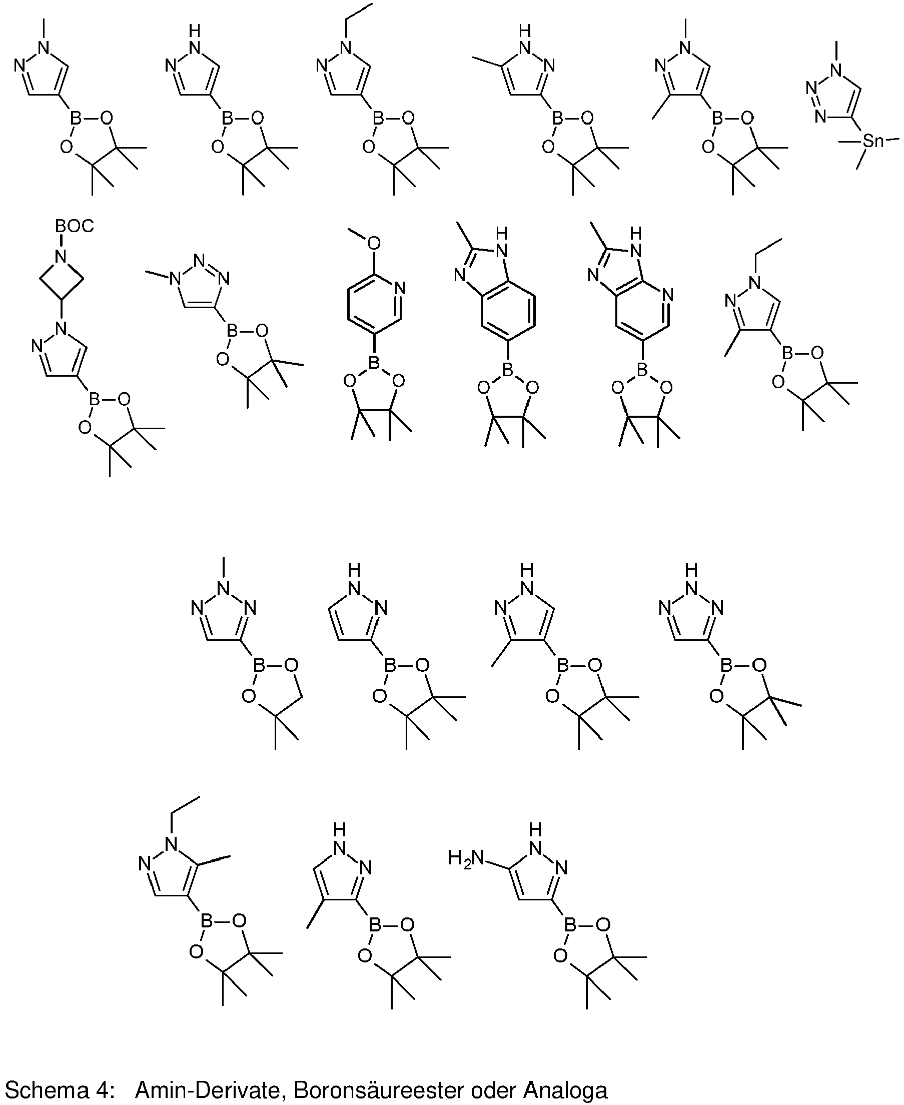

**Tabelle 2**

| Bsp. | Strukturformel | Name | IC₅₀ (pCHK2) | IC₅₀ (ATM) [µM] |
|---|---|---|---|---|
| 1 | 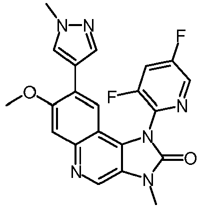 | 1-(3,5-Difluor-pyridin-2-yl)-7-methoxy-3-methyl-8-(1-methyl-1H-pyrazol-4-yl)-1,3-dihydro-imidazo[4,5-c] chinolin-2-on | +++ | < 0,01 |
| | MS: 423 (M+H⁺) | 1H NMR (400 MHz, DMSO-d6, ppm): 8.915 (s, 1H), 8.855-8.849 (d, J=2.4Hz,1H), 8.544-8.495 (m, 1H), 8.016 (s, 1 H), 7.538 (s, 1H), 7.339 (s, 1H), 6.944 (s, 1H), 4.005 (s,3H), 3.864 (s,3H), 3.597 (s,3H) | | |
| 2 | 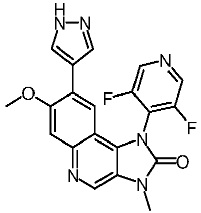 | 1-(3,5-Difluor-pyridin-4-yl)-7-methoxy-3-methyl-8-(1H-pyrazol-4-yl)-1,3-dihydro-imidazo[4,5-c]ch inolin-2-on | +++ | < 0,001 |
| | MS: 409 (M+H⁺) | 1H NMR (400MHz, DMSO, ppm): 13.036 (s, 1H), 9.050 (s,2H), 8.944 (s, 1H), 7.992 (s, 1H), 7.563 (s,1H), 7.440 (s,1H), 7.281(s,1H), 4.012 (s,3H), 3.613 (s,3H) | | |
| 6 | 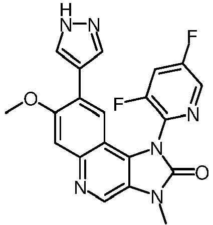 | 1-(3,5-Difluor-pyridin-2-yl)-7-methoxy-3-methyl-8-(1H-pyrazol-4-yl)-1,3-dihydro-imidazo[4,5-c]chinolin-2-on | +++ | < 0,001 |
| | MS: 410 (M+H⁺) | 1H NMR (400MHz,DMSO,ppm): 12.95 (br, 1H), 8.86 (d,2H), 8.51 (m, 1H),7.71 (s,2H), 7.52 (s, 1H), 6.96 (s, 1H), 3.99 (s,3H), 3.29 (s.3H) | | |
| 7 | 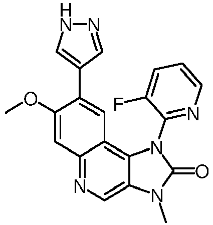 | 1-(3-Fluor-pyridin-2-yl)-7-methoxy-3-methyl-8-(1H-pyrazol-4-yl)-1,3-dihydro-imidazo[4,5-c]ch inolin-2-on | +++ | < 0,001 |
| | MS: 391 (M+H⁺) | 1 H NMR (300MHz,DMSO,ppm): 13.007 (s, 1H), 8.907 (s, 1H), 8.727 (s, 1H), 8.305-8.241 (m, 1H), 7.981-7.939 (m,2H), 7.526 (s, 1H), 7.321 (s, 1H), 6.934 (s, 1H), 3.997 (s,3H), 3.597 (s,3H) | | |
| 8 | 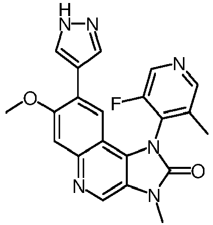 | 1-(3-Fluor-5-methyl-pyridin-4-yl)-7-methoxy-3-methyl-8-(1H-pyrazol-4-yl)-1,3-dihydro-imidazo[4,5-c] chinolin-2-on | +++ | < 0,001 |
| | MS: 405 (M+H⁺) | 1 H NMR (400MHz,DMSO,ppm): 13.035 (s, 1 H), 8.930 (s, 1H), 8.901 (s, 1H), 8.805 (s, 1H), 7.920 (s, 1H), 7.544 (s, 1H), 7.271 (s, 1H), 7.030 (s, 1H), 3.999 (s,3H), 3.615 (s,3H), 2.220 (s,3H) | | |
| 9 | 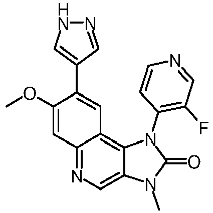 | 1-(3-Fluor-pyridin-4-yl)-7-methoxy-3-methyl-8-(1H-pyrazol-4-yl)-1,3-dihydro-imidazo[4,5-c]ch inolin-2-on | +++ | < 0,001 |
| | MS: 391 (M+H⁺) | 1H NMR (400MHz, DMSO, ppm): 13.023 (s, 1H), 9.075 (s, 1H), 8.908 (s, 1H), 8.838-8.825 (d, J=5.2Hz, 1H), 7.997-7.940 (m,2H), 7.537 (s, 1H), 7.337 (s, 1H), 7.195 (s, 1H), 4.000 (s,3H), 3.589 (s,3H) | | |
| 11 | 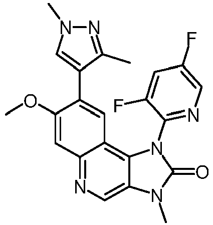 | 1-(3,5-Difluor-pyridin-2-yl)-8-(1 ,3-dimethyl-1H-pyrazol-4-yl)-7-methoxy-3-methyl-1,3-dihydro-imidazo[4,5-c]chinolin-2-on | +++ | < 0,001 |
| | MS: 437 (M+H⁺) | 1H NMR (400MHz, DMSO, ppm): 8.829 (s, 1H), 8.605-8.599 (d, J=2.4Hz,1H), 8.133-8.085 (m, 1H), 7.700 (s, 1H), 7.499 (s, 1H), 6.874 (s, 1H), 3.985 (s,3H), 3.852 (s,3H), 3.680 (s,3H), 1.948 (s,3H) | | |
| 12 | 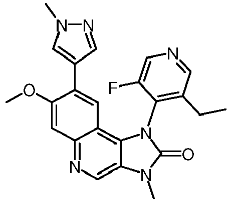 | 1-(3-Ethyl-5-fluor-pyridin-4-yl)-7-methoxy-3-methyl-8-(1-methyl-1H-pyrazol-4-yl)-1,3-dihydro-imidazo[4,5-c]chinolin-2-on | +++ | < 0,001 |
| | MS: 433 (M+H⁺) | 1H NMR (400MHz,DMSO,ppm): 8.935 (s,2H), 8.853 (s, 1H), 7.986 (s, 1H), 7.545 (s, 1H), 7.062 (s, 1H), 6.960 (s, 1H), 3.994 (s,3H), 3.844 (s,3H), 3.618 (s,3H), 2.672-2.600 (m,2H),1.069-1.002 (m,3H) | | |
| 14 | 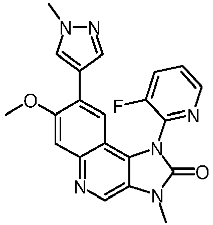 | 1-(3-Fluor-pyridin-2-yl)-7-methoxy-3-methyl-8-(1-methyl-1H-pyrazol-4-yl)-1,3-dihydro-imidazo[4,5-c]chinolin-2-on | +++ | < 0,001 |
| | MS: 405 (M+H⁺) | 1H NMR (400MHz,DMSO,ppm): 8.908 (s, 1H), 8.719-8.708 (m, 1H), 8.295-8.271 (m, 1H), 7.977-7.930 (m, 2H), 7.525 (s,1H), 7.195-7.194 (s,1H), 6.896 (s, 1H), 4.033 (s,3H), 3.994 (s,3H), 3.595 (s,3H) | | |
| 16 | 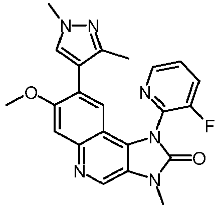 | 8-(1,3-Dimethyl-1H-pyrazol-4-yl)-1-(3-fluor-pyridin-2-yl)-7-methoxy-3-methyl-1,3-dihydro-imidazo[4,5-c] chinolin-2-on | +++ | < 0,001 |
| | MS: 419 (M+H⁺) | 1H NMR (400 MHz, DMSO-d6) ppm = 8.93 (s, 1H), 8.63-8.62 (d, J=4.8Hz, 1 H), 8.25-8.20 (m, 1H), 7.89-7.85 (m, 1H), 7.77 (s, 1H), 7.53 (s, 1H), 6.77 (s, 1H), 3.92 (s, 3H), 3.76 (s, 3H), 3.59 (s, 3H), 1.75 (s, 3H) | | |
| 18 | 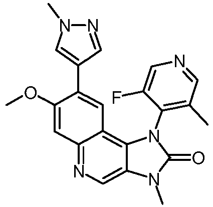 | 1-(3-Fluor-5-methyl-pyridin-4-yl)-7-methoxy-3-methyl-8-(1-methyl-1H-pyrazol-4-yl)-1,3-dihydro-imidazo[4,5-c] chinolin-2-on | +++ | < 0,01 |
| | MS: 419 (M+H⁺) | 1H NMR (400MHz,DMSO,ppm): 8.930 (s, 1H), 8.895 (s, 1H), 8.800 (s, 1H), 7.981 (s, 1H), 7.544 (s, 1H), 7.109 (s, 1H), 6.999 (s, 1H), 3.996 (s,3H), 3.844 (s,3H), 3.613 (s,3H), 2.215 (s,3H) | | |
| 19 | 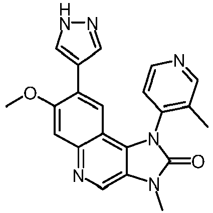 | 7-Methoxy-3-methyl-1-(3-methyl-pyridin-4-yl)-8-(1H-pyrazol-4-yl)-1,3-dihydro-imidazo[4,5-c]ch inolin-2-on | +++ | < 0,01 |
| | MS: 387 (M+H⁺) | 1H NMR (400Hz, DMSO, ppm): 2.141 (s, 3H), 3.596 (s, 3H), 3.991 (s, 3H), 6.970 (s, 1H), 7.202 (s, 1H), 7.517 (s, 1H), 7.682-7.695 (d, J=5.2Hz, 1H), 7.883 (s, 1H),8.777-8.789 (d, J=4.8Hz, 1H), 8.900 (s, 2H), 13.016 (s, 1H) | | |
| 20 | 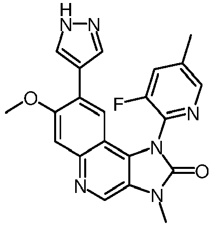 | 1-(3-Fluor-5-methyl-pyridin-2-yl)-7-methoxy-3-methyl-8-(1H-pyrazol-4-yl)-1,3-dihydro-imidazo[4,5-c] chinolin-2-on | +++ | < 0,001 |
| | MS: 405 (M+H⁺) | 1H NMR (400MHz,MeOD,ppm): 8.747 (s, 1H), 8.514 (s,1H), 7.941-7.914 (d,J=10.8,2H), 7.422 (s,2H), 6.947 (s, 1H), 4.019 (s,3H), 3.654 (s,3H), 2.618 (s,3H) | | |
| 22 | 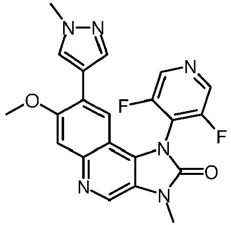 | 1-(3,5-Difluor-pyridin-4-yl)-7-methoxy-3-methyl-8-(1-methyl-1H-pyrazol-4-yl)-1,3-dihydro-imidazo[4,5-c] chinolin-2-on | +++ | < 0,001 |
| | MS: 423 (M+H⁺) | 1H NMR (400MHz,DMSO,ppm): 9.050 (s,2H), 8.499 (s., 1H), 8.030 (s,1H), 7.562 (s, 1H), 7.312-7.254 (d,J=23.2,2H), 4.010 (s,3H), 3.854 (s,3H), 3.611 (s,3H). | | |
| 24 | 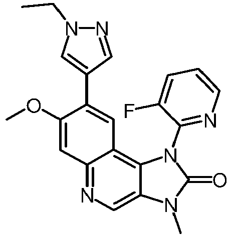 | 8-(1-Ethyl-1H-pyrazol-4-yl)-1-(3-fluor-pyridin-2-yl)-7-methoxy-3-methyl-1,3-dihydro-imidazo[4,5-c] chinolin-2-on | +++ | < 0,001 |
| | MS: 419 (M+H⁺) | 1H NMR (400MHz,DMSO,ppm): 8.907 (s, 1H), 8.732-8.721 (m, 1H), 8.300-8.252 (m, 1H), 7.997-7.936 (m,2H), 7.524 (s, 1H), 7.226 (s, 1H), 6.907 (s, 1H), 4.165-40125 (m,2H), 3.998 (s,3H), 3.596 (s,3H), 1.387-1.350 (m,3H) | | |
| 25 | 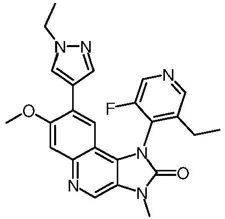 | 1-(3-Ethyl-5-fluor-pyridin-4-yl)-8-(1-ethyl-1H-pyrazol-4-yl)-7-methoxy-3-methyl-1,3-dihydro-imidazo[4,5-c] chinolin-2-on | +++ | < 0,001 |
| | MS: 447 (M+H⁺) | 1H NMR (400MHz,DMSO,ppm): 8.934 (s,2H), 8.860 (s, 1H), 8.001 (s, 1H), 7.544 (s, 1H), 7.098 (s, 1H), 6.973 (s, 1H), 4.165-4.111 (m,2H), 3.999 (s,3H), 3.619 (s,3H), 2.678-2.605 (m,2H), 1.384-1.348 (m,3H), 1.045-1.007 (m,3H) | | |
| 26 | 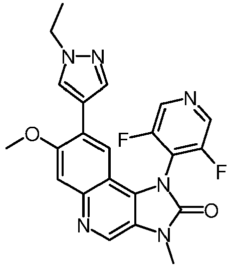 | 1-(3,5-Difluor-pyridin-4-yl)-8-(1-ethyl-1H-pyrazol-4-yl)-7-methoxy-3-methyl-1,3-dihydro-imidazo[4,5-c] chinolin-2-on | +++ | < 0,001 |
| | MS: 437 (M+H⁺) | 1H NMR (300MHz,DMSO,ppm): 9.162 (s, 1H), 9.078 (s,2H), 8.101 (s., 1H), 7.618 (s, 1H), 7.380 (s,2H), 4.192-4.120 (m,2H), 4.062 (s,3H), 3.631 (s,3H), 1.398-1.351 (m,3H). | | |
| 27 | 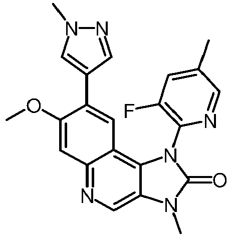 | 1-(3-Fluor-5-methyl-pyridin-2-yl)-7-methoxy-3-methyl-8-(1-methyl-1H-pyrazol-4-yl)-1,3-dihydro-imidazo[4,5-c] chinolin-2-on | +++ | < 0,001 |
| | MS: 419 (M+H⁺) | 1H NMR (400MHz,DMSO,ppm): 8.896 (s, 1H), 8.577 (s, 1H), 8.144-8.118 (d, J=10.4,1H), 7.948 (s, 1 H), 7.517 (s, 1H), 7.218 (s, 1 H), 6.857 (s, 1 H), 3.992 (s,3H), 3.852 (s,3H), 3.588 (s,3H), 2.672 (s,3H) | | |
| 28 | 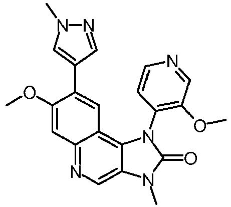 | 7-Methoxy-1-(3-methoxy-pyridin-4-yl)-3-methyl-8-(1-methyl-1H-pyrazol-4-yl)-1,3-dihydro-imidazo[4,5-c] chinolin-2-on | +++ | < 0,01 |
| | MS: 417 (M+H⁺) | 1H NMR (300MHz,DMSO,ppm): 8.855 (m,2H), 8.581 (s, 1H), 7.973 (s., 1H), 7.758 (s, 1H), 7.492 (s, 1H), 7.093 (s,2H), 4.021 (s,3H), 3.843 (m,3H), 3.570 (s,3H), 3.319 (s,3H) | | |
| 29 | 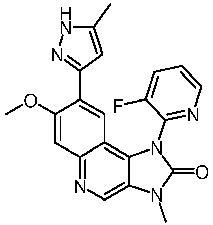 | 1-(3-Fluor-pyridin-2-yl)-7-methoxy-3-methyl-8-(5-methyl-1H-pyrazol-3-yl)-1,3-dihydro-imidazo[4,5-c] chinolin-2-on | +++ | < 0,01 |
| | MS: 405 (M+H⁺) | 1H NMR (400MHz,DMSO,ppm): 8.936 (s, 1H), 8.628 (s, 1H), 8.263-8.239 (m, 1H), 7.891-7.841 (m,2H), 7.541 (s,1H), 7.376 (s,1H), 6.319 (s,1H), 5.980 (s, 1H), 4.016 (s,3H), 3.595 (s,3H), 2.220 (s,3H) | | |
| 31 | 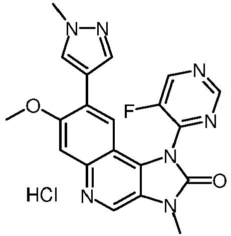 | 1-(5-Fluorpyrimidin-4-yl)-7-methoxy-3-methyl-8-(1-methyl-1H-pyrazol-4-yl)-1H,2H,3H-imidazo[4,5-c] chinolin-2-on hydrochlorid | +++ | < 0,01 |
| | MS: 406 (M+H⁺) | 1H NMR (400MHz,DMSO,ppm): 9.43-9.31 (m,3H), 8.14 (s,1H), 7.75 (s,1H), 7.56 (d,2H), 4.08 (s,3H), 3.88 (s,3H), 3.62 (s,3H) | | |
| 32 | 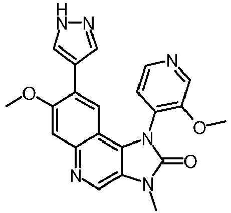 | 7-Methoxy-1-(3-methoxy-pyridin-4-yl)-3-methyl-8-(1H-pyrazol-4-yl)-1,3-dihydro-imidazo[4,5-c]chinolin-2-on | +++ | < 0,01 |
| | MS: 403 (M+H⁺) | 1H NMR (300MHz,DMSO,ppm): 13.016 (s, 1H), 8.856 (s,2H), 8.584 (s., 1H), 7.908 (s, 1H), 7.766 (s, 1H), 7.492 (s, 1H), 7.255 (s, 1H), 7.119 (s, 1H), 3.986 (s,3H), 3.801 (s,3H), 3.571 (s,3H) | | |
| 33 | 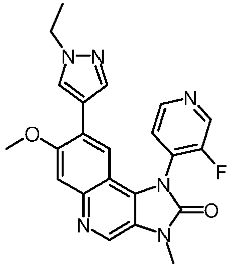 | 8-(1-Ethyl-1H-pyrazol-4-yl)-1-(3-fluor-pyridin-4-yl)-7-methoxy-3-methyl-1,3-dihydro-imidazo[4,5-c] chinolin-2-on | +++ | < 0,001 |
| | MS: 419 (M+H⁺) | 1H NMR (400MHz,DMSO,ppm): 9.076 (s, 1H), 8.908 (s, 1H), 8.842-8.829 (d, J=5.2Hz, 1H), 8.009 (s, 1H), 7.997-7.969 (m, 1H), 7.535 (s, 1H), 7.217 (s, 1H), 7.175 (s, 1H), 4.166-4.112 (m,2H), 4.000 (s,3H), 3.587 (s,3H), 1.383-1.347 (m,3H) | | |
| 34 | 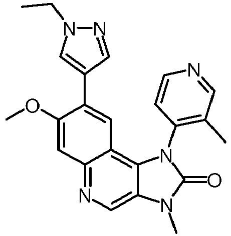 | 8-(1-Ethyl-1H-pyrazol-4-yl)-7-methoxy-3-methyl-1-(3-methyl-pyridin-4-yl)-1,3-dihydro-imidazo[4,5-c] chinolin-2-on | +++ | < 0,01 |
| | MS: 415 (M+H⁺) | 1H NMR (400MHz, DMSO, ppm): 1.349-1.386 (t, J=7.4Hz, 3H), 2.139 (s, 3H), 3.597 (s, 3H), 3.994 (s, 3H), 4.1-4.2 (m, 2H), 6.949 (s, 1H), 7.066 (s, 1H), 7.517 (s, 1H), 7.682-7.695 (d, J=5.2Hz, 1H), 7.968 (s, 1H), 8.780-8.793 (d, J=5.2Hz, 1H), 8.900 (s, 2H) | | |
| 35 | 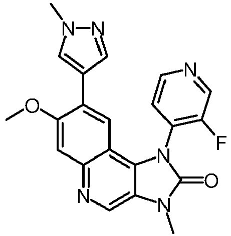 | 1-(3-Fluor-pyridin-4-yl)-7-methoxy-3-methyl-8-(1-methyl-1H-pyrazol-4-yl)-1,3-dihydro-imidazo[4,5-c] chinolin-2-on | +++ | < 0,01 |
| | MS: 405 (M+H⁺) | 1H NMR (400MHz,DMSO,ppm): 9.073-9.070 (d, J=1.2Hz,1H), 8.901 (s, 1H), 8.838-8.825 (d, J=5.2Hz,1H), 7.993-7.964 (m,2H), 7.532 (s, 1H), 7.185 (s, 1H), 7.164 (s, 1H), 3.995 (s,3H), 3.847 (s,3H), 3.583 (s,3H) | | |
| 36 | 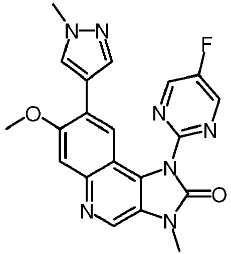 | 1-(5-Fluor-pyrimidin-2-yl)-7-methoxy-3-methyl-8-(1-methyl-1H-pyrazol-4-yl)-1,3-dihydro-imidazo[4,5-c] chinolin-2-on | +++ | < 0,01 |
| | MS: 406 (M+H⁺) | 1H NMR (400MHz,DMSO,ppm): 9.33 (s,2H), 8.89 (s, 1H), 8.08 (s, 1H), 7.51 (s, 1H), 7.42 (s, 1H), 6.92 (s, 1H), 4.11 (s,3H), 3.99 (s,3H), 3.57 (s,3H) | | |
| 38 | 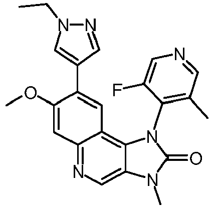 | 8-(1-Ethyl-1H-pyrazol-4-yl)-1-(3-fluor-5-methyl-pyridin-4-yl)-7-methoxy-3-methyl-1,3-dihydro-imidazo[4,5-c] chinolin-2-on | ++ | < 0,001 |
| | MS: 433 (M+H⁺) | 1H NMR (400MHz,DMSO,ppm): 8.929 (s, 1H), 8.904 (s, 1H), 8.806 (s, 1H), 7.993 (s, 1H), 7.543 (s, 1H), 7.144 (s, 1H), 7.010 (s, 1H), 4.165-4.111 (m,2H), 4.000 (s,3H), 3.614 (s,3H), 2.218 (s,3H), 1.384-1.348 (m,3H) | | |
| 39 | 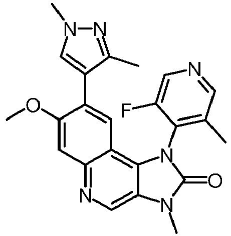 | 8-(1,3-Dimethyl-1H-pyrazol-4-yl)-1-(3-fluor-5-methyl-pyridin-4-yl)-7-methoxy-3-methyl-1,3-dihydro-imidazo[4,5-c]chinolin-2-on | ++ | < 0,001 |
| | MS: 433 (M+H⁺) | 1H NMR (400MHz,DMSO,ppm): 8.948 (s, 1H), 8.833 (s, 1H), 8.732 (s, 1H), 7.824 (s, 1H), 7.545 (s, 1H), 6.874 (s, 1H), 3.929 (s,3H), 3.759 (s,3H) ,3.611 (s,3H), 2.189 (s,3H), 1.702 (s,3H) | | |
| 40 | 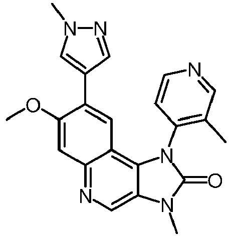 | 7-Methoxy-3-methyl-8-(1-methyl-1H-pyrazol-4-yl)-1-(3-methyl-pyridin-4-yl)-1,3-dihydro-imidazo[4,5-c] chinolin-2-on | ++ | < 0,01 |
| | MS: 401 (M+H⁺) | 1H NMR (400MHz, DMSO, ppm): 2.128 (s, 3H), 3.589 (s, 3H), 3.833 (s, 3H), 3.982 (s, 3H), 6.933 (s, 1H), 7.018 (s, 1H), 7.511 (s, 1H), 7.665-7.678 (d, J=5.2Hz, 1H), 7.953 (s, 1H), 8.764-8.777 (d, J=5.2Hz, 1H), 8.886-8.893 (d, J=2.8Hz, 2H). | | |
| 44 | 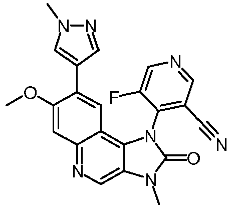 | 5-Fluor-4-[7-methoxy-3-methyl-8-(1-methyl-1H-pyrazol-4-yl)-2-oxo-2,3-dihydro-imidazo[4,5-c]chinolin-1-yl]-nicotinonitril | ++ | < 0,01 |
| | MS: 430 (M+H⁺) | 1H NMR (400MHz,DMSO,ppm): 9.399 (s, 1H), 9.346 (s, 1H), 8.968 (s, 1H), 8.036 (s, 1H), 7.578 (s, 1 H), 7.404 (s, 1H), 7.215 (s, 1 H), 4.020 (s,3H), 3.851 (s,3H), 3.636 (s,3H) | | |
| 45 | 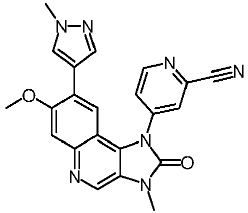 | 4-[7-Methoxy-3-methyl-8-(1-methyl-1H-pyrazol-4-yl)-2-oxo-2,3-dihydro-imidazo[4,5-c]chinolin-1-yl]-pyridin-2-carbonitril | ++ | < 0,05 |
| | MS: 412 (M+H⁺) | 1H NMR (400MHz,DMSO,ppm): 9.099 (s, 1H), 8.913 (s, 1H), 8.497 (s, 1H), 8.106 (s, 1H), 8.097 (s, 1 H), 7.549 (s, 1H), 7.332 (s, 1 H), 7.269 (s, 1 H), 4.007 (s,3H), 3.857 (s,3H), 3.574 (s,3H) | | |
| 46 | 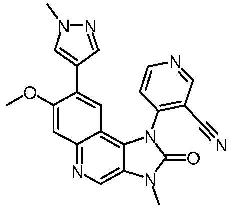 | 4-[7-Methoxy-3-methyl-8-(1-methyl-1H-pyrazol-4-yl)-2-oxo-2,3-dihydro-imidazo[4,5-c]chinolin-1-yl]-nicotinonitril | ++ | < 0,05 |
| | MS: 412 (M+H⁺) | 1H NMR (400MHz,CDCl3,ppm): 9.219 (s, 1H), 9.122 (s,1H), 8.718 (s,1H), 7.767 (s,1H), 7.689 (s,2H), 7.300 (s, 1H), 7.140 (s, 1H), 4.071 (s,3H), 3.924 (s,3H), 3.686 (s,3H) | | |
| 48 | 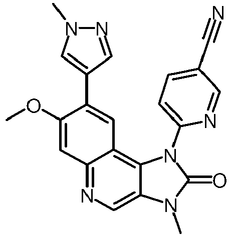 | 6-[7-Methoxy-3-methyl-8-(1-methyl-1H-pyrazol-4-yl)-2-oxo-2,3-dihydro-imidazo[4,5-c]chinolin-1-yl]-nicotinonitril | ++ | < 0,01 |
| | MS: 412 (M+H⁺) | 1H NMR (300MHz,DMSO,ppm): 9.308 (s, 1H), 8.909 (s, 1H), 8.744 (m, 1H), 8.090 (m, 1H), 8.009 (s, 1 H), 7.524 (s, 1 H), 7.402 (s, 1 H), 7.242 (s, 1H), 4.003 (s,3H), 3.865 (s,3H), 3.576 (s,3H) | | |
| 50 | 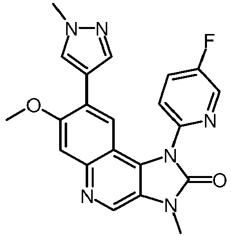 | 1-(5-Fluoro-pyridin-2-yl)-7-methoxy-3-methyl-8-(1-methyl-1H-pyrazol-4-yl)-1,3-dihydro-imidazo[4,5-c]chinolin-2-on | ++ | < 0,01 |
| | MS: 405 (M+H⁺) | 1H NMR (400MHz,DMSO,ppm): 8.916 (m,2H), 8.240 (m, 1H), 7.963 (m,2H), 7.515 (s, 1H), 7.258 (m, 1H), 6.993 (s, 1H), 4.174 (s,3H), 3.854 (s,3H), 3.523 (s,3H) | | |
| 51 | 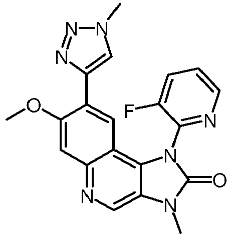 | 1-(3-Fluoro-pyridin-2-yl)-7-methoxy-3-methyl-8-(1-methyl-1H-[1,2,3]triazol-4-yl)-1,3-dihydro-imidazo[4,5-c]chinolin-2-on | ++ | < 0,01 |
| | MS: 406 (M+H⁺) | 1H NMR (400MHz,DMSO,ppm): 8.964 (s, 1H), 8.643 (s,1 H), 8.424 (s, 1H), 8.287-8.239 (m, 1H), 4.057 (s,6H), 3.606 (s,3H) | | |
| 52 | 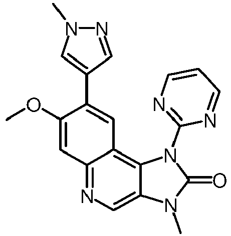 | 7-Methoxy-3-methyl-8-(1-methyl-1H-pyrazol-4-yl)-1-pyrimidin-2-yl-1,3-dihydro-imidazo[4,5-c]ch inolin-2-on | ++ | < 0,01 |
| | MS: 388 (M+H⁺) | 1H NMR (300MHz,DMSO): 3.57 (s,3H), 3.85 (s,3H), 3.99 (s,3H), 6.83 (s, 1H), 7.26 (s, 1H), 7.51 (s, 1H), 7.93 (t, J=4.8,1H), 7.98 (s, 1 H), 8.89 (s, 1H), 9.23 (d, J=4.8,2H). | | |
| 54 | 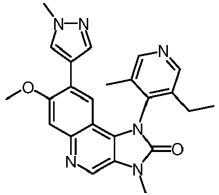 | 1-(3-Ethyl-5-methyl-pyridin-4-yl)-7-methoxy-3-methyl-8-(1-methyl-1H-pyrazol-4-yl)-1,3-dihydro-imidazo[4,5-c]chinolin-2-on | ++ | < 0,01 |
| | MS: 429 (M+H⁺) | 1H NMR (400MHz,DMSO,ppm): 8.918 (s, 1H), 8.751 (s,1H), 8.714 (s,1H), 7.952 (s,1H), 7.514 (s, 1H), 6.963 (s, 1H), 6.831 (s, 1H), 3.982 (s,3H), 3.833 (s,3H), 3.681 (s,3H), 2.469-2.329 (m,3H), 1.977 (s,3H), 0.966-0.928 (m,3H) | | |
| 56 | 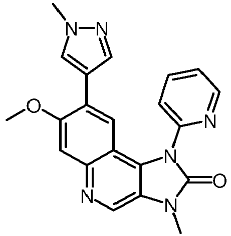 | 7-Methoxy-3-methyl-8-(1-methyl-1H-pyrazol-4-yl)-1-pyridin-2-yl-1,3-dihydro-imidazo[4,5-c]chinolin-2-on | ++ | < 0,01 |
| | MS: 387 (M+H⁺) | 1H NMR (DMSO, 400MHz, ppm): 8.874 (s,1H), 8.816-8.832 (m, 1H), 8.2-8.3 (t, 1H), 7.964 (s, 1H), 7.822-7.842 (d, J=8.0Hz,1H), 7.758-7.789 (m,1H), 7.494 (s, 1H), 7.160 (s, 1H), 6.984 (s, 1H), 3.986 (s,3H), 3.842 (s,3H), 3.575 (s,3H) | | |
| 57 | 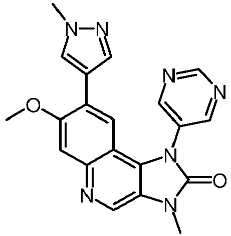 | 7-Methoxy-3-methyl-8-(1-methyl-1H-pyrazol-4-yl)-1-pyrimidin-5-yl-1,3-dihydro-imidazo[4,5-c]ch inolin-2-on | ++ | < 0,05 |
| | MS: 388 (M+H⁺) | 1H NMR (400MHz,DMSO,ppm): 9.512 (s, 1H), 9.241 (s,2H), 8.906 (s, 1H), 7.991 (s, 1H), 7.541 (s, 1H), 7.247 (s, 1H), 7.134 (s, 1H), 4.035-4.004 (s,3H), 3.884-3.857 (s,3H), 3.590 (s,3H) | | |
| 58 | 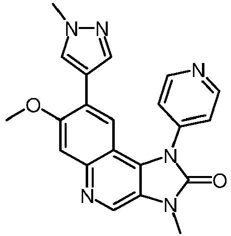 | 7-Methoxy-3-methyl-8-(1-methyl-1H-pyrazol-4-yl)-1-pyridin-4-yl-1,3-dihydro-imidazo[4,5-c]chinolin-2-on | ++ | < 0,01 |
| | MS: 387 (M+H⁺) | 1H NMR (300MHZ,DMSO): 3.57 (s,3H), 3.84 (s,3H), 3.99 (s,3H), 7.11 (s, 1H), 7.20 (s, 1H), 7.50 (s, 1H),7.75 (d, J=6.0Hz,2H), 8.00 (s, 1H), 8.90 (s, 1H), 8.99 (d, J=6.0Hz,2H) | | |
| 60 | 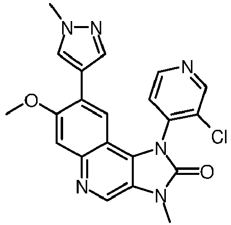 | 1-(3-Chlor-pyridin-4-yl)-7-methoxy-3-methyl-8-(1-methyl-1H-pyrazol-4-yl)-1,3-dihydro-imidazo[4,5-c] chinolin-2-on | + | < 0,01 |
| | MS: 421 (M+H⁺) | 1H NMR (300 MHz, DMSO,ppm): 9.43 (s, 1H), 9.24 (s, 1H), 9.03 (d, J = 5.1 Hz, 1H), 8.10 (d, J = 5.1 Hz, 2H), 7.98 (s,1H), 7.13 (s,1H), 7.06 (s,1H), 4.07 (s,3H), 3.87 (s,3H), 3.67 (s,3H) | | |
| 61 | 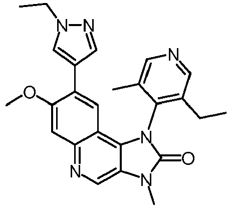 | 1-(3-Ethyl-5-methyl-pyridin-4-yl)-8-(1-ethyl-1H-pyrazol-4-yl)-7-methoxy-3-methyl-1,3-dihydro-imidazo[4,5-c] chinolin-2-on | + | < 0,01 |
| | MS: 443 (M+H⁺) | 1H NMR (400MHz,DMSO,ppm): 8.918 (s, 1H), 8.760 (s,1H), 8.723 (s,1H), 7.963 (s,1H), 7.516 (s, 1H), 7.009 (s,1H), 6.847 (s,1H), 4.156-4.102 (m,2H), 3.989 (s,3H), 3.622 (s,3H), 2.514-2.458 (m,2H), 2.004 (s,3H), 1.382-1.345 (m,3H), 0.973-0.936 (m,3H) | | |
| 62 | 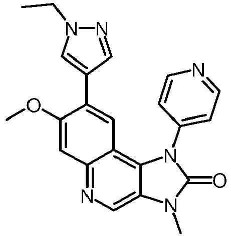 | 8-(1-Ethyl-1H-pyrazol-4-yl)-7-methoxy-3-methyl-1-pyridin-4-yl-1,3-dihydro-imidazo[4,5-c]chinolin-2-on | + | < 0,01 |
| | MS: 401 (M+H⁺) | 1H NMR (400MHZ,DMSO,ppm): 1.35 (t, J=7.6Hz, 3H), 3.56 (s,3H), 3.98 (s,3H), 4.12 (q, J=7.6,2H), 7.17 (d, J=8.8Hz,2H), 7.51 (s, 1H), 7.74 (d, J=6Hz,2H), 7.98 (s, 1H), 8.87 (s, 1H), 8.95 (br,2H) | | |
| 63 | 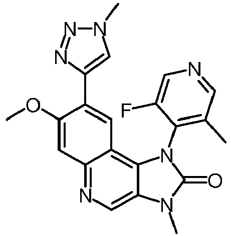 | 1-(3-Fluor-5-methyl-pyridin-4-yl)-7-methoxy-3-methyl-8-(1-methyl-1H-[1,2,3]triazol-4-yl)-1,3-dihydro-imidazo[4,5-c] chinolin-2-on | ++ | < 0,01 |
| | MS: 420 (M+H⁺) | 1H NMR (400 MHz, DMSO-d6) ppm = 8.97 (s, 1H), 8.83 (s, 1H), 8.74 (s, 1H), 8.42 (s, 1H), 7.91 (s,3H) | | |
| 64 | 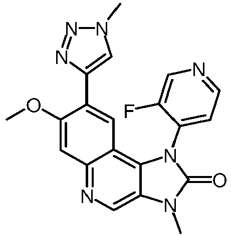 | 1-(3-Fluor-pyridin-4-yl)-7-methoxy-3-methyl-8-(1-methyl-1H-[1,2,3]triazol-4-yl)-1,3-dihydro-imidazo[4,5-c] chinolin-2-on | ++ | < 0,01 |
| | MS: 406 (M+H⁺) | 1H NMR (400 MHz, DMSO-d6) ppm = 9.01 (s, 1H), 8.94 (s, 1H), 8.78-8.77 (m, 1H), 8.42 (s, 1H), 8.04 (s, 1H), 7.94-7.92 (m, 1H), 7.58 (s ,1H), 4.05 (s,6H), 3.58 (s,3H) | | |
| 65 | 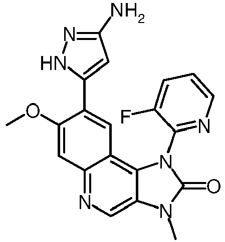 | 8-(3-Amino-1H-pyrazol-5-yl)-1-(3-fluor-2-pyridyl)-7-methoxy-3-methyl-imidazo[4,5-c]chinolin-2-on | ++ | < 0,001 |
| | MS: 406 (M+H⁺) | 1H NMR (400MHz, DMSO) ppm = 11.3 (s, 1H), 8.87 (s, 1H), 8.60-8.59 (m, 1H), 8.17-8.10 (m, 1H), 7.86-7.80 (m, 1H), 7.52 (s, 1H), 7.16 (s, 1H), 5.50 (s, 1H), 4.40 (s,2H), 3.97 (s,3H), 3.58 (s,3H). | | |
| 66 | 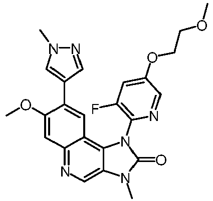 | 1-[3-Fluor-5-(2-methoxyethoxy)-2-pyridyl]-7-methoxy-3-methyl-8-(1-methylpyrazol-4-yl)imidazo[4,5-c]chinolin-2-on | +++ | < 0,001 |
| | MS: 479 (M+H⁺) | 1H NMR (400MHz, DMSO) ppm = 8.90 (s, 1H), 8.48 (s, 1H), 8.02-7.97 (m,2H), 7.52 (s, 1H), 7.29 (s, 1H), 6.92 (s, 1H), 4.43 (d, J = 4,2H), 4.00 (s,3H), 3.86 (s,3H), 3.79-3.77 (m,2H), 3.59 (s,3H), 3.37 (s,3H) | | |
| 67 | 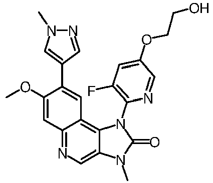 | 1-[3-Fluor-5-(2-hydroxyethoxy)-2-pyridyl]-7-methoxy-3-methyl-8-(1-methylpyrazol-4-yl)imidazo[4,5-c]chinolin-2-on | +++ | < 0,001 |
| | MS: 465 (M+H⁺) | 1H NMR (400MHz, DMSO) ppm = 8.89 (s, 1H), 8.46 (s, 1H), 7.95 (m,2H), 7.51 (s, 1H), 7.29 (s, 1H), 6.92 (s, 1H), 5.07 (m, 1H), 4.30 (m,2H), 3.99 (s,3H), 3.85-3.82 (m,5H), 3.58 (s,3H) | | |
| 68 | 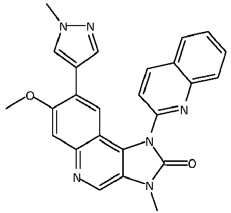 | 7-Methoxy-3-methyl-8-(1-methyl-1H-pyrazol-4-yl)-1-chinolin-2-yl-1,3-dihydro-imidazo[4,5-c]chinolin-2-on | + | < 0,01 |
| | MS. 437 (M+H⁺) | 1H NMR (400 MHz, DMSO-d6) ppm = 8.905 (s, 1H), 8.801-8.780 (m, 1H), 8.254-8.235 (m, 1H), 8.0-032 (m, 1H), 7.949-7.928 (s,2H), 7.850-7.829 (m,1H), 7.829 (s, 1H), 7.700 (s, 1H), 7.113 (s, 1H), 6.674 (s, 1H), 3.965 (s,3H), 3.671 (s,3H), 3.596 (s,3H) | | |
| 69 | 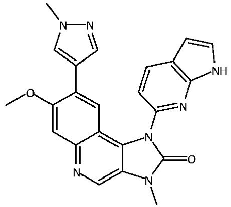 | 7-Methoxy-3-methyl-8-(1-methyl-1H-pyrazol-4-yl)-1-(1H-pyrrolo[2,3-b]pyridin-6-yl)1,3-dihydro-imidazo[4,5-c]chinolin-2-on | ++ | < 0,001 |
| | MS. 426 (M+H⁺) | 1H NMR (400 MHz, DMSO-d6) ppm = 8.85 (s,1H), 8.36 (d, J = 8,1H), 7.76-7.71 (m,2H), 7.46- 7.41 (m,2H), 6.75-6.67 (m,3H), 3.96 (s,3H), 3.74 (s,3H), 3.52 (s,3H) | | |
| 101 | 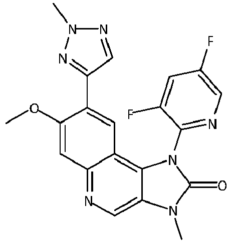 | 1-(3,5-Difluor-pyridin-2-yl)-7-methoxy-3-methyl-8-(2-methyl-2H-[1,2,3]triazol-4-yl)-1,3-dihydro-imidazo[4,5-c] chinolin-2-on | +++ | < 0,05 |
| | MS: 424 (M+H⁺) | 1H NMR (300 MHz, DMSO-d6) ppm = 8.97 (s, 1H), 8.84 (s, 1H), 8.57 -8.51 (m, 1H), 7.59 (s, 1H), 7.45 (s, 1H), 4.13 (s,3H), 4.04 (s,3H), 3.61 (s,3H) | | |
| 103 | 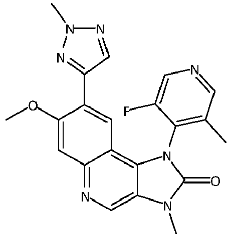 | 1-(3-Fluor-5-methyl-pyridin-4-yl)-7-methoxy-3-methyl-8-(2-methyl-2H-[1,2,3]triazol-4-yl)-1,3-dihydro-imidazo[4,5-c]chinolin-2-on | ++ | < 0,05 |
| | MS: 420 (M+H⁺) | 1H NMR (300 MHz, DMSO-d6) ppm = 9.04 (s, 1H), 8.87 (s, 1H), 8.77 (s, 1H), 8.09 (s, 1H), 7.68 (s, 1H), 7.61 (s, 1H), 4.40 (s,3H), 4.04 (s,3H), 3.62 (s,3H), 2.20 (s,3H) | | |
| 104 | 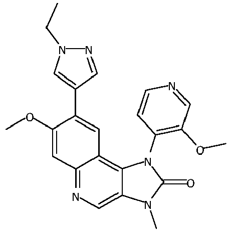 | 8-(1-Ethyl-1H-pyrazol-4-yl)-7-methoxy-1-(3-methoxy-pyridin-4-yl)-3-methyl-1,3-dihydro-imidazo[4,5-c] chinolin-2-on | ++ | < 0,01 |
| | MS: 431 (M+H⁺) | 1H NMR (300MHz,DMSO) ppm = 8.85 -8.84 (d, J=4.5, 2H), 8.58-8.57 (d,J=4.8, 1 , H), 7.98 (s, 1H), 7.76-7.74 (d, J=5.1, 1H), 7.49 (s, 1H), 7.12-7.10 (d, J=6.6, 2H), 4.17 (m,2H), 3.98 (s,3H), 3.57 (s,3H), 3.36 (s,3H), 1.39 (m,3H) | | |
| 105 | 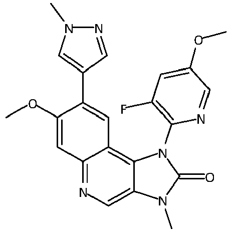 | 1-(3-Fluor-5-methoxy-pyridin-2-yl)-7-methoxy-3-methyl-8-(1-methyl-1H-pyrazol-4-yl)-1,3-dihydro-imidazo[4,5-c]chinolin-2-on | ++ | < 0,001 |
| | MS: 435 (M+H⁺) | 1H NMR (400MHz,DMSO) ppm = 8.89 (s, 1H), 8.45 (s, 1H), 7.98 (m,2H), 7.51 (s, 1H), 7.25 (s, 1H), 6.88 (s, 1H), 4.04 (s,3H), 3.99 (s,3H), 3.85 (s,3H), 3.58 (s,3H) | | |
| 106 | 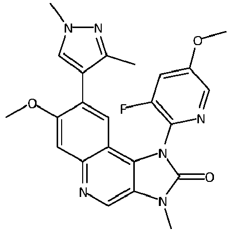 | 8-(1,3-Dimethyl-1H-pyrazol-4-yl)-1-(3-fluor-5-methoxy-pyridin-2-yl)-7-methoxy-3-methyl-1,3-dihydro-imidazo[4,5-c]chinolin-2-on | ++ | < 0,001 |
| | MS: 449 (M+H⁺) | 1H NMR (400MHz,DMSO) ppm = 8.89 (s, 1H), 8.35 (s, 1H), 7.94 (s, 1H), 7.78 (s, 1H), 7.50 (s, 1H), 6.78 (s, 1H), 3.98 (m,3H), 3.90 (s,3H), 3.75 (s,3H), 3.63 (s,3H), 1.78 (s,3H) | | |
| 107 | 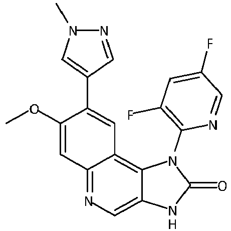 | 1-(3,5-Difluor-pyridin-2-yl)-7-methoxy-8-(1-methyl-1H-pyrazol-4-yl)-1,3-dihydro-imidazo[4,5-c]chinolin-2-on | ++ | < 0,001 |
| | MS: 409 (M+H⁺) | 1H NMR (400MHz,DMSO) ppm = 11.81 (s, 1 H), 8.84 (d ,J= 2.6Hz, 1H), 8.68 (s, 1H), 8.00 (s, 1H), 7.49 (s, 1H), 7.33 (d, J= 0.8Hz, 1H), 6.93 (s, 1H), 3.99 (s,3H), 3.85 (s,3H). | | |
| 108 | 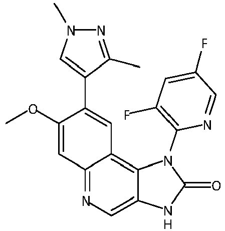 | 1-(3,5-Difluor-pyridin-2-yl)-8-(1,3-dimethyl-1H-pyrazol-4-yl)-7-methoxy-1,3-dihydro-imidazo[4,5-c]chinolin-2-on | ++ | < 0,001 |
| | MS: 423 (M+H⁺) | 1H NMR (400MHz,DMSO) ppm = 11.84 (s, 1H), 8.77 (d,J= 2.5Hz, 1H), 8.70 (s, 1H), 8.48 (m, 1H), 7.79 (s, 1H), 7.50 (s, 1H), 6.76 (s, 1H), 3.91 (s,3H), 3.77 (s,3H), 1.82 (s,3H). | | |
| 109 | 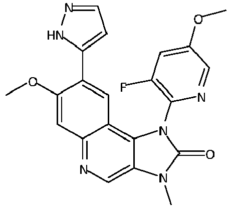 | 1-(3-Fluor-5-methoxy-pyridin-2-yl)-7-methoxy-3-methyl-8-(2H-pyrazol-3-yl)-1,3-dihydro-imidazo[4,5-c] chinolin-2-on | +++ | < 0,01 |
| | 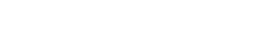 | 1H NMR (400 MHz, DMSO) ppm = 8.91 (s, 1H), 8.36 (d, J= 2.6Hz, 1 H), 7.93 (m, 1H), 7.64 (s, 1 H), 7.53 (s, 1H), 7.40 (s, 1 H), 6.54 (s, 1H), 4.01 (s,3H), 3.97 (s,3H), 3.57 (s,3H). | | |
| | MS: 421 (M+H⁺) | | | |
| 110 | 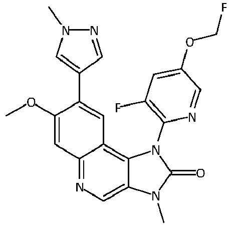 | 1-(3-Fluor-5-fluormethoxy-pyridin-2-yl)-7-methoxy-3-methyl-8-(1-methyl-1H-pyrazol-4-yl)-1,3-dihydro-imidazo[4,5-c]chinolin-2-on | +++ | < 0,001 |
| | MS: 453 (M+H⁺) | 1H NMR (400MHz,DMSO) ppm = 8.90 (s, 1H), 8.65-8.62 (s, 1H), 8.23-8.20 (m, 1H), 7.95 (s, 1H), 7.52 (s, 1H), 7.30 (s, 1H), 6.90 (s, 1H), 6.23-6.20 (m, 1H), H), 6.10-6.01 (s, 1H), 3.99 (s,3H), 3.84 (s,3H), 3.59 (s,3H) | | |
| 111 | 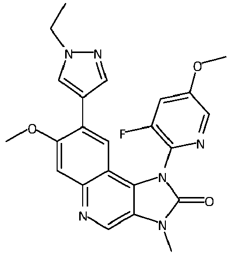 | 8-(1-Ethyl-1H-pyrazol-4-yl)-1-(3-fluor-5-methoxy-pyridin-2-yl)-7-methoxy-3-methyl-1,3-dihydro-imidazo[4,5-c] chinolin-2-on | ++ | < 0,01 |
| | MS: 449 (M+H⁺) | 1H NMR (400MHz,DMSO) ppm = 8.87 (s, 1H), 8.47 (s, 1H), 7.96 (m,2H), 7.50 (s, 1H), 7.29 (s, 1H), 6.88 (s, 1H), 4.16 (m,2H), 4.03 (s,3H), 3.98 (s,3H), 3.57 (s,3H), 1.36 (m,3H) | | |
| 112 | 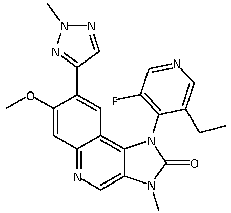 | 1-(3-Ethyl-5-fluor-pyridin-4-yl)-7-methoxy-3-methyl-8-(2-methyl-2H-[1,2,3]triazol-4-yl)-1,3-dihydro-imidazo[4,5-c] chinolin-2-on | ++ | < 0,01 |
| | MS: 434 (M+H⁺) | 1H NMR (400MHz,DMSO) ppm = 9.00 (s, 1H), 8.88 (s, 1H), 8.82 (s, 1H), 8.08 (s, 1H), 7.64-7.06 (d, J=16, 2H), 4.08 (s,3H), 4.03 (s,3H), 3.62 (s,3H), 2.57-2.55 (m,2H), 1.03-1.00 (m,3H) | | |
| 113 | 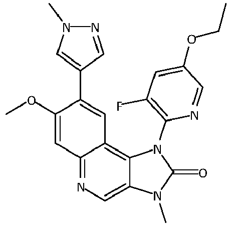 | 1-(5-Ethoxy-3-fluor-pyridin-2-yl)-7-methoxy-3-methyl-8-(1-methyl-1H-pyrazol-4-yl)-1,3-dihydro-imidazo[4,5-c]chinolin-2-on | +++ | < 0,001 |
| | MS: 449 (M+H⁺) | 1H NMR (400MHz,DMSO) ppm = 8.87 (s, 1H), 8.43 (s, 1H), 7.94 (m,2H), 7.50 (s, 1H), 7.24 (s, 1H), 6.87 (s, 1H), 4.32 (m,2 H), 3.98 (s,3H), 3.84 (s,3H), 3.57 (s,3H), 1.44 (m,3H) | | |
| 114 | 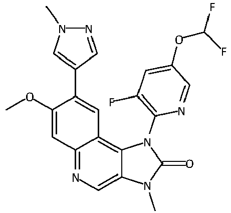 | 1-(5-Difluormethoxy-3-fluor-pyridin-2-yl)-7-methoxy-3-methyl-8-(1-methyl-1H-pyrazol-4-yl)-1,3-dihydro-imidazo[4,5-c]chinolin-2-on | +++ | < 0,001 |
| | MS: 471 (M+H⁺) | 1H NMR (400MHz,DMSO) ppm = 8.92 (s, 1H), 8.71 (s, 1H), 8.35 (m, 1H), 7.96 (s, 1H), 7.78 (s,2H), 7.34 (s, 1H), 6.94 (s, 1H), 4.00 (s,3H), 3.84 (s,3H), 3.32 (s,3H) | | |
| 115 | 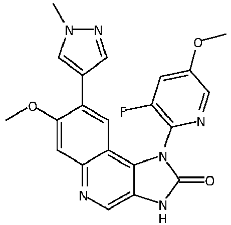 | 1-(3-Fluor-5-methoxy-pyridin-2-yl)-7-methoxy-8-(1-methyl-1H-pyrazol-4-yl)-1,3-dihydro-imidazo[4,5-c]chinolin-2-on | ++ | < 0,01 |
| | MS: 421 (M+H⁺) | 1H NMR (400MHz,DMSO) ppm = 11.71 (s, 1H), 8.65 (s, 1H), 8.46 (s, 1H), 7.98 (m,2H), 7.47 (s, 1H), 7.24 (s, 1H), 6.87 (s, 1H), 4.04 (s,3H), 3.98 (s,3H), 3.85 (s,3H) | | |
| 116 | 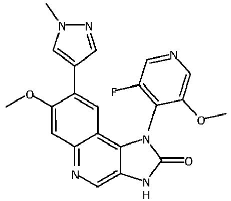 | 1-(3-Fluor-5-methoxy-pyridin-4-yl)-7-methoxy-8-(1-methyl-1H-pyrazol-4-yl)-1,3-dihydro-imidazo[4,5-c]chinolin-2-on | + | < 0,01 |
| | MS: 421 (M+H⁺) | 1H NMR (400 MHz, DMSO-d6) ppm = 11.77 (s, 1H), 8.72 (d,J = 16.7 Hz, 2H), 8.64 (s, 1H), 7.98 (s, 1H), 7.47 (s, 1H), 7.13 (d, J = 14.7 Hz, 2H), 3.97 (s,3H), 3.84 (d, J = 8.0 Hz, 6H). | | |
| 117 | 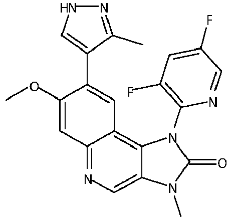 | 1-(3,5-Difluor-pyridin-2-yl)-7-methoxy-3-methyl-8-(3-methyl-1H-pyrazol-4-yl)-1,3-dihydro-imidazo[4,5-c]chinolin-2-on | ++ | < 0,01 |
| | MS: 423 (M+H⁺) | 1H NMR (300MHz, DMSO) ppm = 12.34-12.31 (s, 1H), 8.64-8.41 (s, 1H), 8.78 (s, 1H), 8.67-8.59 (m, 1H), H), 7.52-7.44 (s,2H), 6.74 (s, 1H), 3.91-3.82 (s,3H), 3.57-3.49 (s,3H), 2.02-1.87 (m,3H) | | |
| 119 | 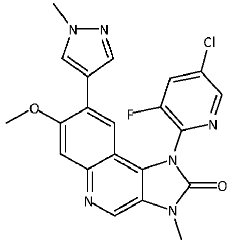 | 1-(5-Chlor-3-fluor-pyridin-2-yl)-7-methoxy-3-methyl-8-(1-methyl-1H-pyrazol-4-yl)-1,3-dihydro-imidazo[4,5-c]chinolin-2-on | +++ | < 0,001 |
| | MS: 440 (M+H⁺) | 1H NMR (400MHz, DMSO) ppm = 8.91 (s, 1H), 8.85 (s, 1H), 8.66 (m, 1H), 8.00 (s, 1H), 7.53 (s, 1H), 7.33 (s, 1H), 6.96 (s, 1H), 4.00 (s,3H), 3.86 (s,3H), 3.59 (s,3H) | | |
| 120 | 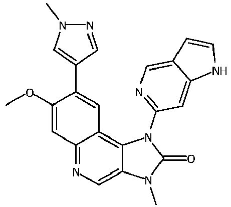 | 7-Methoxy-3-methyl-8-(1-methyl-1H-pyrazol-4-yl)-1-(1H-pyrrolo[3,2-c]pyridin-6-yl)-1,3-dihydro-imidazo[4,5-c]chinolin-2-on | ++ | < 0,001 |
| | MS: 426 (M+H⁺) | 1H NMR (400MHz, DMSO) ppm = 11.97 (s, 1H), 9.00 (s, 1H), 8.84 (s, 1H), 7.78 (s,2H), 7.71 (d, J=2.2Hz, 1H), 7.44 (s, 1H), 6.84 (d, J=2.9Hz, 1H), 6.80 (d, 1H), 6.73 (s, 1H), 3.95 (s,3H), 3.74 (s,3H), 3.57 (s,3H) | | |
| 122 | 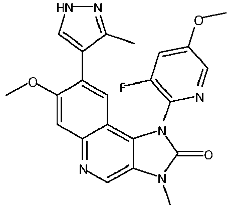 | 1-(3-Fluor-5-methoxy-pyridin-2-yl)-7-methoxy-3-methyl-8-(3-methyl-1H-pyrazol-4-yl)-1,3-dihydro-imidazo[4,5-c] chinolin-2-on | +++ | < 0,001 |
| | MS: 435 (M+H⁺) | 1H NMR (300MHz,DMSO) ppm = 8.84 (s, 1H), 8.31-8.30 (s, 1H), 7.81-7.77 (d, J=12, 1H), 7.50 (s,2H), 6.76 (s, 1H), 3.99 (s,3H), 3.90 (s,3H), 3.56 (s,3H), 1.93 (s,3H) | | |
| 123 | 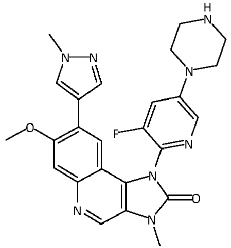 | 1-(3-Fluor-5-piperazin-1-yl-pyridin-2-yl)-7-methoxy-3-methyl-8-(1-methyl-1H-pyrazol-4-yl)-1,3-dihydro-imidazo[4,5-c]chinolin-2-on | ++ | < 0,01 |
| | MS: 490(M+H⁺) | 1H NMR (400MHz, DMSO) ppm = 8.85 (s, 1H), 8.36 (d, J = 2.5Hz, 1H), 7.95 (s, 1H), 7.64 (dd, J = 12.7, 2.6Hz, 1H), 7.48 (s, 1H), 7.28 (s, 1H), 6.89 (s, 1H), 3.97 (s,3H), 3.84 (s,3H), 3.56 (s,3H), 3.36 (dd, J = 6.1 ,3.8Hz, 4H), 2.88 (t, J = 5.0Hz, 4H) | | |
| 125 | 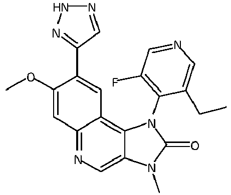 | 1-(3-Ethyl-5-fluor-pyridin-4-yl)-7-methoxy-3-methyl-8-(2H-[1,2,3]triazol-4-yl)-1,3-dihydro-imidazo[4,5-c] chinolin-2-on | ++ | < 0,001 |
| | MS: 420 (M+H⁺) | 1H NMR (400 MHz, DMSO) ppm = 15.25 (s, 1H), 9.00 (s, 1H), 8.88 (s, 1H), 8.82 (s, 1H), 8.26 (s, 1H), 7.83 (s,1H), 7.62 (s,1H), 4.05 (s,3H), 3.64 (s,3H), 2.67 - 2.51 (m,2H), 1.03 (m,3H). | | |
| 126 | 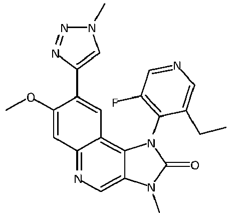 | 1-(3-Ethyl-5-fluor-pyridin-4-yl)-7-methoxy-3-methyl-8-(1-methyl-1H-[1,2,3]triazol-4-yl)-1,3-dihydro-imidazo[4,5-c] chinolin-2-on | ++ | < 0,01 |
| | MS: 434 (M+H⁺) | 1H NMR (400 MHz, DMSO) ppm = 9.00 (s, 1H), 8.87 (s, 1H), 8.82 (s, 1H), 8.44 (s, 1H), 7.92 (s, 1H), 7.62 (s, 1H), 4.06 (d, J = 6.0 Hz, 6H), 3.64 (s,3H), 2.59 (m,2H), 1.03 (t, J = 7.6 Hz, 3H) | | |
| 127 | 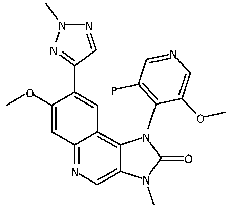 | 1-(3-Fluor-5-methoxy-pyridin-4-yl)-7-methoxy-3-methyl-8-(2-methyl-2H-[1,2,3]triazol-4-yl)-1,3-dihydro-imidazo[4,5-c] chinolin-2-on | ++ | < 0,01 |
| | MS: 436 (M+H⁺) | 1H NMR (400 MHz, DMSO-d6) ppm = 8.94 (s, 1H), 8.76 (s, 1H), 8.68 (s,1H), 8.08 (s,1H), 7.81 (s,1H), 7.57 (s,1H), 4.11 (s,3H), 4.02 (s,3H), 3.86 (s,3H), 3.59 (s,3H). | | |
| 128 | 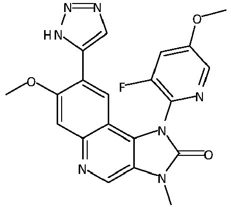 | 1-(3-Fluor-5-methoxy-pyridin-2-yl)-7-methoxy-3-methyl-8-(3H-[1,2,3]triazol-4-yl)-1,3-dihydro-imidazo[4,5-c] chinolin-2-on | ++ | < 0,01 |
| | MS: 422 (M+H⁺) | 1H NMR (400 MHz, DMSO-d6) ppm = 15.16 (s, 1H), 8.93 (s, 1H), 8.38 (d, J = 2.6 Hz, 1H), 8.23 (s, 1H), 7.96 (dd, J = 11.0, 2.6 Hz, 1H), 7.66 (s,1H), 7.57 (s,1H), 4.02 (s,6H), 3.58 (s,3H). | | |
| 129 | 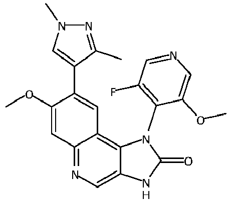 | 8-(1,3-Dimethyl-1H-pyrazol-4-yl)-1-(3-fluor-5-methoxy-pyridin-4-yl)-7-methoxy-1,3-dihydro-imidazo[4,5-c]chinolin-2-on | + | < 0,001 |
| | MS: 435 (M+H⁺) | 1H NMR (400 MHz, DMSO-d6) ppm = 11.8 (s,1H), 8.69 - 8.62 (m,3H), 7.79 (s,1H), 7.46 (s,1H), 6.95 (s,1H), 3.90 (s,3H), 3.84 (s,3H), 3.75 (s,3H), 1.72 (s,3H). | | |
| 131 | 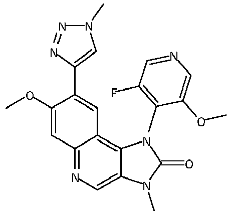 | 1-(3-Fluor-5-methoxy-pyridin-4-yl)-7-methoxy-3-methyl-8-(1-methyl-1H-[1,2,3]triazol-4-yl)-1,3-dihydro-imidazo[4,5-c]chinolin-2-on | ++ | < 0,01 |
| | MS: 436 (M+H⁺) | 1H NMR (400 MHz, DMSO-d6) ppm = 8.93 (s,1H), 8.57 (s,1H), 4.04 (d, 1H), 9.80 (s,1H), 3.84 (s,3H), 3.59 (s,3H). | | |
| 132 | 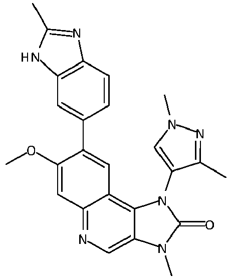 | 1-(1,3-Dimethyl-1H-pyrazol-4-yl)-7-methoxy-3-methyl-8-(2-methyl-3H-benzoimidazol-5-yl)-1,3-dihydro-imidazo[4,5-c]chinolin-2-on | ++ | < 0,001 |
| | MS: 455 (M+H⁺) | 1H NMR (400 MHz, DMSO) ppm = 12.24 (d, J = 6.5 Hz, 1H), 8.85 (d, J = 2.1 Hz, 1H), 8.13 (d, J = 4.9 Hz, 1H), 7.55 - 7.47 (m,2H), 7.45 -7.36 (m,1H), 7.26 (d, J = 2.2 Hz, 1H), 7.10 (m,1H), 3.94 - 3.77 (m,6H), 3.55 (s,3H), 2.49 (s,3H), 1.94 (s,3H). | | |
| 133 | 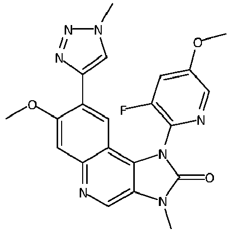 | 1-(3-Fluor-5-methoxy-pyridin-2-yl)-7-methoxy-3-methyl-8-(1-methyl-1H-[1,2,3]triazol-4-yl)-1,3-dihydro-imidazo[4,5-c] chinolin-2-on | ++ | < 0,01 |
| | MS: 436 (M+H⁺) | 1H NMR (400 MHz, DMSO-d6) ppm = 8.92 (s, 1H), 8.43 - 8.34 (m,2H), 7.96 (dd, J = 11.0, 2.6 Hz, 1H), 7.73 (s,1H), 7.56 (s,1H), 4.04 (d, J = 13.4 Hz, 9H), 3.58 (s,3H) | | |
| 134 | 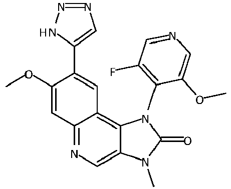 | 1-(3-Fluor-5-methoxy-pyridin-4-yl)-7-methoxy-3-methyl-8-(3H-[1,2,3]triazol-4-yl)-1,3-dihydro-imidazo[4,5-c] chinolin-2-on | ++ | < 0,001 |
| | MS: 422 (M+H⁺) | 1H NMR (400 MHz, DMSO-d6) ppm = 15.11 (s, 1H), 8.94 (s, 1H), 8.70 (d, J = 16.3 Hz, 2H), 8.25 (s, 1H), 7.96 (s, 1H), 7.58 (s, 1H), 4.03 (s,3H), 3.85 (s,3H), 3.59 (s,3H), 2.06 (s,1H). | | |
| 136 | 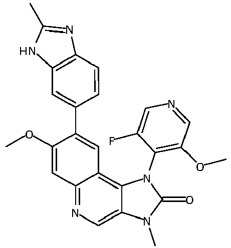 | 1-(3-Fluor-5-methoxy-pyridin-4-yl)-7-methoxy-3-methyl-8-(2-methyl-3H-benzoimidazol-5-yl)-1,3-dihydro-imidazo[4,5-c]chinolin-2-on | +++ | < 0,001 |
| | MS: 485 (M+H⁺) | 1H NMR (400 MHz, DMSO) ppm = 12.23 (s,1H), 8.93 (s, 1H), 8.65 (d, J = 1.9 Hz, 1H), 8.60 (s, 1H), 7.56 (s, 1H), 7.50-7.28 (m,2H), 7.07 - 6.94 (m,2H), 3.89 (d, J = 2.0 Hz, 6H), 3.59 (s,3H), 2.49 (s,3H). | | |
| 137 | 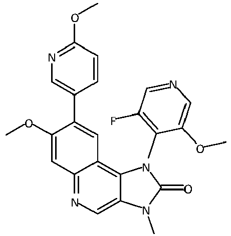 | 1-(3-Fluor-5-methoxy-pyridin-4-yl)-7-methoxy-8-(6-methoxy-pyridin-3-yl)-3-methyl-1,3-dihydro-imidazo[4,5-c]chinolin-2-on | ++ | < 0,01 |
| | MS: 462 (M+H⁺) | 1H NMR (400MHz, DMSO) ppm = 8.95 (s,1H), 8.65 (d, J = 15.8Hz, 2H), 8.00 (d, J = 2.4Hz, 1H), 7.65 (dd, J = 8.6, 2.5Hz, 1H), 7.57 (s, 1H), 6.93 (s, 1H), 6.83 (d, J = 8.6Hz, 1H), 3.87 (d, J = 15.0Hz, 9H), 3.59 (s,3H) | | |
| 138 | 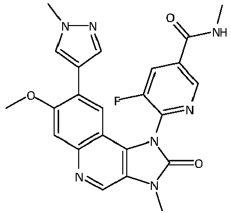 | 5-Fluor-6-[7-methoxy-3-methyl-8-(1-methyl-1H-pyrazol-4-yl)-2-oxo-2,3-dihydro-imidazo[4,5-c]chinolin-1-yl]-N-methyl-nicotinamid | ++ | < 0,01 |
| | MS: 462 (M+H⁺) | 1H NMR (400 MHz, DMSO) ppm = 9.08 - 8.96 (m,2H), 8.93 (s,1H), 8.55 (dd, J = 9.6, 1.9 Hz, 1H), 7.94 (s, 1H), 7.54 (s, 1H), 7.30 (s, 1H), 6.95 (s, 1H), 4.01 (s,3H), 3.84 (s,3H), 3.61 (s,3H), 2.92 (d, J = 4.5 Hz, 3H) | | |
| 139 | 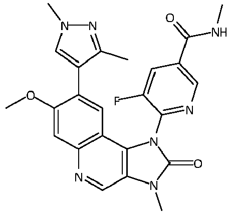 | 6-[8-(1,3-Dimethyl-1H-pyrazol-4-yl)-7-methoxy-3-methyl-2-oxo-2,3-dihydro-imidazo[4,5-c]chinolin-1-yl]-5-fluor-N-methylnicotinamid | ++ | < 0,001 |
| | MS: 476 (M+H⁺) | 1H NMR (400 MHz, DMSO) ppm = 9.02 - 8.93 (m,3H), 8.52 (dd, J = 9.5, 1.9 Hz, 1H), 7.80 (s, 1H), 7.55 (s, 1H), 6.81 (s, 1H), 3.94 (s,3H), 3.76 (s,3H), 3.61 (s,3H), 2.90 (d, J = 4.5 Hz, 3H), 1.76 (s,3H) | | |
| 144 | 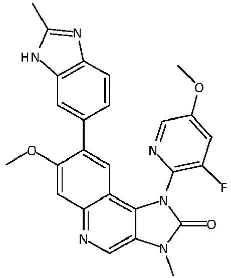 | 1-(3-Fluor-5-methoxy-pyridin-2-yl)-7-methoxy-3-methyl-8-(2-methyl-3H-benzoimidazol-5-yl)-1,3-dihydro-imidazo[4,5-c]chinolin-2-on | ++ | < 0,001 |
| | MS: 485 (M+H⁺) | 1H NMR (400 MHz, DMSO-d6) ppm = 12.21 (s, 1H), 8.93 (s, 1H), 8.35 (d, J = 2.6 Hz, 1H), 7.95 - 7.81 (m, 1H), 7.55 (s, 1H), 7.38 (d, J = 21.1 Hz, 2H), 7.01 (d, J = 32.5 Hz, 1H), 6.72 (s, 1H), 3.93 (d,J = 27.1 Hz, 6H), 3.58 (s,3H), 2.48 (d, J = 1.9 Hz, 3H). | | |
| 152 | 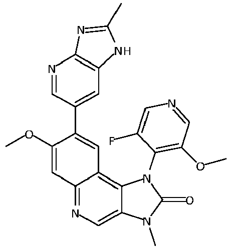 | 1-(3-Fluor-5-methoxy-pyridin-4-yl)-7-methoxy-3-methyl-8-(2-methyl-1H-imidazo[4,5-b]pyridin-6-yl)-1,3-dihydro-imidazo[4,5-c]chinolin-2-on | +++ | < 0,001 |
| | MS: 486 (M+H⁺) | 1H NMR (400 MHz, DMSO-d6) ppm = 12.87 - 12.50 (m,1H), 8.99 (s,1H), 8.65 (d, J = 14.3 Hz, 2H), 8.11 (d, J = 23.8 Hz, 1H), 7.78 (d, J = 12.4 Hz, 1H), 7.62 (d, J = 2.4 Hz, 1H), 7.06 - 6.98 (d, J = 20 Hz, 1H), 3.93 (s,3H), 3.89 (d, J = 6.8 Hz, 3H), 3.63 (s,3H), 2.55 (d, J = 14.3 Hz, 3H). | | |
| 153 | 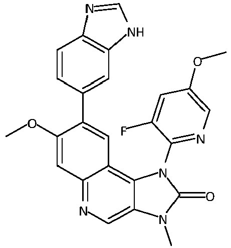 | 8-(3H-Benzimidazol-5-yl)-1-(3-fluor-5-methoxy-pyridin-2-yl)-7-methoxy-3-methyl-1,3-dihydro-imidazo[4,5-c]chinolin-2-on | ++ | < 0,01 |
| | MS: 471 (M+H⁺) | 1H NMR (400 MHz, DMSO-d6) ppm = 12.47 (s, 1H), 8.93 (s, 1H), 8.36 (d, J = 2.6 Hz, 1H), 8.24 (s, 1H), 7.88 (d, J = 10.9 Hz, 1H), 7.56 (s,3H), 7.19 - 7.00 (m,1H), 6.75 (s, 1H), 3.90 (s,3H), 3.59 (s,3H), 3.31 (s,3H). | | |
| 154 | 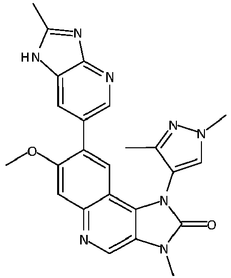 | 1-(1,3-Dimethyl-1H-pyrazol-4-yl)-7-methoxy-3-methyl-8-(2-methyl-1H-imidazo[4,5-b]pyridin-6-yl)-1,3-dihydro-imidazo[4,5-c]chinolin-2-on | + | < 0,001 |
| | MS: 455 (M+H⁺) | 1H NMR (400 MHz, DMSO-d6) ppm =8.91 (s, 1H), 7.29 (s,1H), 3.93 (s,3H), 3.87 (s,3H), 3.58 (s,3H), 2.56 (s,3H), 1.97 (s,3H). | | |
| 155 | 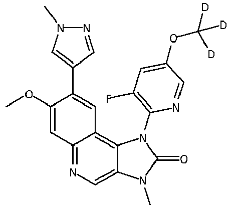 | 1-[3-Fluor-5-(trideuteriomethoxy)-2-pyridyl]-7-methoxy-3-methyl-8-(1-methylpyrazol-4-yl) imidazo[4,5-c]chinolin-2-on | +++ | < 0,001 |
| | MS: 438 (M+H⁺) | 1H NMR (400 MHz,DMSO) ppm = 8.89 (s, 1H), 8.47 (s, 1H), 7.98 (m,2H), 7.52 (s, 1H), 7.25 (s, 1H), 6.88(s,1H), 3.99 (s,3H), 3.85 (s,3H), 3.59 (s,3H) | | |
| 156 | 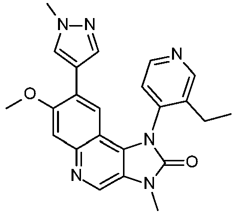 | 1-(3-Ethyl-pyridin-4-yl)-7-methoxy-3-methyl-8-(1-methyl-1H-pyrazol-4-yl)-1,3-dihydro-imidazo[4,5-c] chinolin-2-on | ++ | < 0,001 |
| | MS: 415 (M+H⁺) | 1H NMR (400 MHz, DMSO-d6) ppm = 8.93 (s, 1H), 8.89 (s, 1H), 8.78 (d, 1H), 7.95 (s, 1H), 7.65 (d, 1H), 7.50 (s, 1H), 6.97 (s, 1H), 6.89 (s, 1H), 3.98 (s,3H), 3.83 (s,3H), 3.59 (s,3H), 2.50 (m,2H), 0.99-0.95 (m,3H) | | |
| 157 | 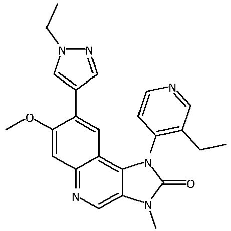 | 8-(1-Ethyl-1H-pyrazol-4-yl)-1 -(3-ethyl-pyridin-4-yl)-7-methoxy-3-methyl-1,3-dihydro-imidazo[4,5-c] chinolin-2-on | +++ | < 0,01 |
| | 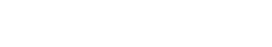 | 1H NMR (400 MHz, DMSO-d6) ppm = 8.88 (s, 1H), 8.14-8.11 (s,2H), 8.04 (s, 1H), 7.54 (s, 1H), 4.19 (s,3H), 4.03-3.99 (d,6H), 3.56 (s,3H), 1.56 (s,3H) | | |
| | MS:429 (M+H⁺) | | | |
| 158 | 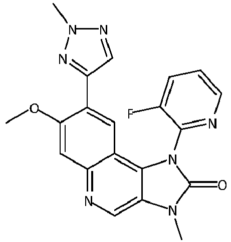 | 1-(3-Fluor-pyridin-2-yl)-7-methoxy-3-methyl-8-(2-methyl-2H-[1,2,3]triazol-4-yl)-1,3-dihydro-imidazo[4,5-c] chinolin-2-on | ++ | < 0,5 |
| | MS: 406 (M+H⁺) | 1H NMR (400 MHz, DMSO-d6) ppm = 8.96 (s, 1H), 8.68 (s, 1H), 8.27-8.23 (m,1H), 8.07 (s, 1H), 7.96-7.92 (m,1H), 7.57 (s,1H), 7.51 (s,1H), 4.10 (s,3H), 4.02 (s,3H), 3.59 (s,3H) | | |
| 159 | 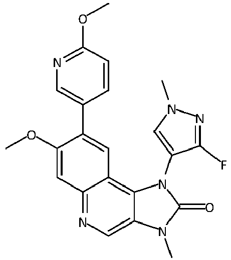 | 1-(3-Fluor-1-methyl-1H-pyrazol-4-yl)-7-methoxy-8-(6-methoxy-pyridin-3-yl)-3-methyl-1,3-dihydro-imidazo[4,5-c]chinolin-2-on | + | < 0,01 |
| | 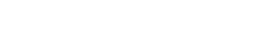 MS: 435 (M+H⁺) | 1H NMR (400 MHz, DMSO-d6) ppm = 8.90 (s,1H), H), 8.19 (dd, J = 2.4, 0.8 Hz, 1H), 7.89 (d, J = 2.4 Hz, 1H), 7.76 (dd, J = 8.6, 2.5 Hz, 1H), 7.56 (s, 1H), 7.42 (s, 1H), 6.92 - 6.84 (m,1H), 3.89 (d, J = 8.2 Hz, 6H), 3.82 (d, J = 1.1 Hz, 3H), 3.55 (s,3H). | | |
| 200 | 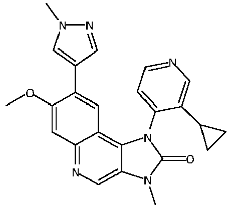 | 1-(3-Cyclopropyl-pyridin-4-yl)-7-methoxy-3-methyl-8-(1-methyl-1H-pyrazol-4-yl)-1,3-dihydro-imidazo[4,5-c] chinolin-2-on | ++ | < 0,05 |
| | 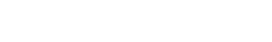 | 1H NMR (400MHz,DMSO) ppm = 8.87 (s, 1H), 8.73 (d, J= 5.1, 1H), 8.61 (s, 1H), 7.95 (s, 1H), 7.64 (d, J= 5.1 Hz, 1H), 7.49 (s, 1H), 6.98-6.92 (m,2H), 3.97 (s,3H), 3.82 (s,3H), 3.58 (s,3H), 1.59 (m,1H), 0.91 (m,1H), 0.82-0.73 (m,1H), 0.62-0.49 (m,2H). | | |
| | MS: 427 (M+H⁺) | | | |
| | 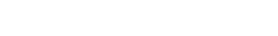 | | | |
| 201 | 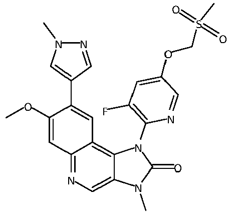 | 1-(3-Fluor-5-methylsulfonylmethoxy-pyridin-2-yl)-7-methoxy-3-methyl-8-(1-methyl-1H-pyrazol-4-yl)-1,3-dihydro-imidazo[4,5-c]chinolin-2-on | +++ | < 0,001 |
| | MS: 514 (M+H⁺) | 1H NMR (400MHz,DMSO) ppm = 8.91 (s, 1H), 8.66 (s, 1H), 8.28 (m, 1H), 7.89 (s, 1H), 7.53 (s, 1H), 7.41 (s, 1H), 6.95 (s, 1H), 5.70 (s,2H), 4.02 (s,3H), 4.00 (s,3H), 3.59 (s,3H), 3.21 (s,3H) | | |
| 202 | 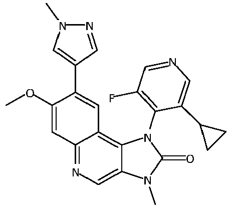 | 1-(3-Cyclopropyl-5-fluor-pyridin-4-yl)-7-methoxy-3-methyl-8-(1-methyl-1H-pyrazol-4-yl)-1,3-dihydro-imidazo[4,5-c]chinolin-2-on | ++ | < 0,001 |
| | MS: 445 (M+H⁺) | 1H NMR (400MHz,DMSO) ppm = 8,91 (s, 1H), 8.83 (s, 1H), 8.50 (s, 1H), 7.98 (s, 1H), 7.52 (s, 1H), 7.08-6.99 (m,2H), 3.98 (s,3H), 3.83 (s,3H), 3.60 (s,3H), 1.71-1.61 (m,1H), 1. 04- 0.9 5 (m,1H), 0.87 (m,1H), 0.37-0.62 (m,2H). | | |
| 203 | 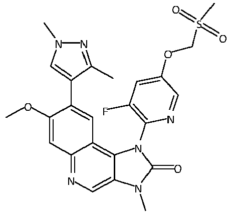 | 8-(1,3-Dimethyl-1H-pyrazol-4-yl)-1-(3-fluor-5-methylsulfonylmethoxy-pyridin-2-yl)-7-methoxy-3-methyl-1,3-dihydro-imidazo[4,5-c]chinolin-2-on | ++ | < 0,001 |
| | MS: 528 (M+H⁺) | 1H NMR (400MHz,DMSO) ppm = 8.92 (s, 1H), 8.56 (s, 1H), 8.22 (m,1H), 7.75 (s, 1H), 7.53 (s, 1H), 6.83 (s, 1H), 5.64 (s,2H), 3.92 (s,3H), 3.76 (s,3H), 3.58 (s,3H), 3.17 (s,3H), 1.826(s,3H) | | |
| 204 | 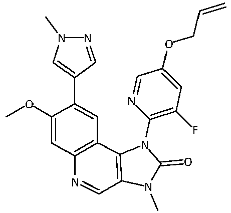 | 1-(5-Allyloxy-3-fluor-pyridin-2-yl)-7-methoxy-3-methyl-8-(1-methyl-1H-pyrazol-4-yl)-1,3-dihydro-imidazo[4,5-c]chinolin-2-on | ++ | < 0,001 |
| | MS: 461 (M+H⁺) | 1H NMR (400 MHz,DMSO) ppm = 8.87 (s, 1H), 8.46 (d, J = 2.6Hz, 1H), 8.01-7.93 (m,2H), 7.50 (s, 1H), 7.24 (d, J = 0.8Hz, 1H), 6.87 (s, 1H), 6.11 (ddt, J = 17.2, 10.6, 5.3Hz, 1H), 5.51 (dq, J = 17.3, 1.6Hz, 1H), 5.38 (dq, J = 10.5, 1.4Hz, 1H), 4.88 | | |
| 205 | 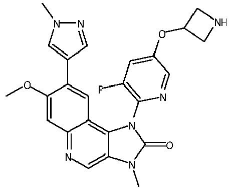 | 1-[5-(Azetidin-3-yloxy)-3-fluor-pyridin-2-yl]-7-methoxy-3-methyl-8-(1-methyl-1H-pyrazol-4-yl)-1,3-dihydro-imidazo[4,5-c]chinolin-2-on | ++ | < 0,001 |
| | MS: 476 (M+H⁺) | 1H NMR (400MHz,DMSO) ppm = 8.87 (s, 1H), 8.40 (s,1H), 7.98 (s, 1H), 7.84 (m, 1H), 7.50 (s, 1H), 7.27 (s, 1H), 6.89 (s,1H), 5.28 (s, 1H), 3.98 (s,3H), 3.85 (s,2H), 3.84 (s,3H), 3.62 (s,2H), 3.57 (s,3H) | | |
| 206 | 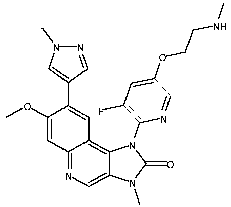 | 1-[3-Fluor-5-(2-methylamino-ethoxy)-pyridin-2-yl]-7-methoxy-3-methyl-8-(1-methyl-1H-pyrazol-4-yl)-1,3-dihydro-imidazo[4,5-c] chinolin-2-on | ++ | < 0,01 |
| | MS: 478 (M+H⁺) | 1H NMR (400 MHz, DMSO-d6) ppm = 8.87 (s,1H), H), 8.44 (d, J = 2.6 Hz, 1H), 7.97 (d, J = 13.0 Hz, 2H), 7.50 (s, 1H), 7.26 (d, J = 0.8 Hz, 1H), 6.89 (s,1H), H), 4.36 - 4.27 (m,2H), 3.97 (s,3H), 3.84 (s,3H), 3.57 (s,3H), 2.97 - 2.89 (m,2H), 2.37 (s,3H). | | |
| 207 | 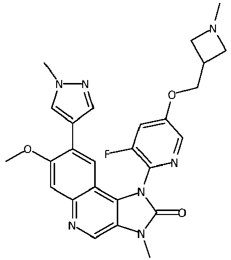 | 1-[3-Fluor-5-(1-methyl-azetidin-3-ylmethoxy)-pyridin-2-yl]-7-methoxy-3-methyl-8-(1-methyl-1H-pyrazol-4-yl)-1,3-dihydro-imidazo[4,5-c]chinolin-2-on | ++ | <0,01 |
| | MS: 505 (M+H⁺) | 1H NMR (400 MHz, DMSO) ppm = 8.87 (s, 1H), 8.45 (d, J = 2.5 Hz, 1H), 7.98 (d, J = 12.0 Hz, 2H), 7.50 (s, 1H), 7.25 (s, 1H), 6.86 (s, 1H), 4.42 - 4.35 (m,2H), 3.97 (s,3H), 3.84 (s,3H), 3.57 (s,3H), 3.35 (s,2H), 3.02 (t, J = 6.20 Hz, 2H), 2.84 (m,1H), 2.22 (s,3H). | | |
| 208 | 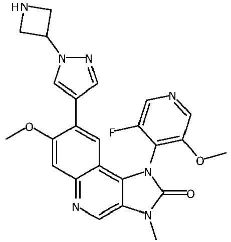 | 8-[1-(Azetidin-3-yl)-1H-pyrazol-4-yl]-1-(3-fluor-5-methoxypyridin-4-yl)-7-methoxy-3-methyl-1H,2H,3H-imidazo[4,5-c]chinolin-2-on | + | < 0,01 |
| | MS: 476 (M+H⁺) | H NMR (400 MHz, DMSO-d6) ppm = 8.90 (s, 1H), 8.78 (s, 1H), 8.74 (s, 1H), 8.11 (s, 1H), 7.53 (s, 1H), 7.28 (s,1H), H), 7.17 (s,1H), H), 5.21 (m,1H), 4.00 (s,3H), 3.96 - 3.84 (m,5H), 3.71 (t, J = 7.9 Hz, 2H), 3.59 (s,3H) | | |

| | | | | |
|---|---|---|---|---|
| +++: 0,2 µM oder weniger ++: > 0,2 µM bis 1 µM +: > 1 µM bis 2 µM | | | | |

Erfindungsgemäße Verbindungen weisen nicht nur eine sehr gute Hemmung von ATM Kinase auf, sie sind auch zusätzlich noch sehr selektiv gegenüber anderen Kinasen, wie beispielsweise mTOR, PIK3 alpha, PI3K beta, PI3K delta und PI3K gamma, was anhand der in der nachfolgenden Tabelle 3 aufgeführten experimentellen Daten belegt wird:

**Tabelle 3**

| Bsp. | IC₅₀ (ATM) [µM] | IC50 (PI3Kalpha) [µM] | IC50 (PI3Kbeta) [µM] | IC50 (PI3Kdelta) [µM] | IC50 (PI3Kgamm a) [µM] | IC50 (mTOR) [µM] |
|---|---|---|---|---|---|---|
| 1 | < 0,01 | > 30 | > 30 | > 30 | > 30 | > 30 |
| 2 | < 0,001 | > 30 | > 30 | > 30 | > 30 | >5 |
| 6 | < 0,001 | > 30 | > 30 | > 30 | > 30 | > 30 |
| 7 | < 0,001 | > 30 | > 30 | > 30 | > 30 | > 30 |
| 8 | < 0,001 | > 30 | > 30 | > 30 | > 30 | >5 |
| 9 | < 0,001 | > 30 | > 30 | > 30 | > 30 | > 10 |
| 11 | < 0,001 | > 30 | > 30 | > 30 | > 30 | > 30 |
| 12 | < 0,001 | > 30 | > 30 | > 30 | > 30 | > 30 |
| 14 | < 0,001 | >30 | > 30 | > 30 | > 30 | > 30 |
| 16 | < 0,001 | > 30 | > 30 | > 30 | > 30 | > 30 |
| 18 | < 0,001 | > 10 | > 30 | > 30 | > 5 | > 5 |
| 19 | < 0,01 | > 30 | | > 30 | > 30 | > 5 |
| 20 | < 0,001 | > 30 | > 30 | > 30 | > 30 | > 30 |
| 22 | < 0,001 | > 30 | | > 30 | > 30 | > 30 |
| 24 | < 0,001 | > 30 | > 30 | > 30 | > 30 | > 30 |
| 25 | < 0,001 | > 30 | > 30 | > 30 | > 30 | > 30 |
| 26 | < 0,001 | > 30 | | > 30 | > 30 | > 30 |
| 27 | < 0,001 | > 30 | > 30 | > 30 | > 30 | > 30 |
| 28 | < 0,01 | > 30 | > 30 | > 30 | > 30 | > 30 |
| 29 | < 0,01 | > 30 | > 30 | > 30 | > 30 | > 30 |
| 31 | < 0,01 | > 30 | > 30 | > 30 | > 30 | > 30 |
| 32 | < 0,01 | > 30 | | > 30 | > 30 | > 10 |
| 33 | < 0,001 | > 30 | > 30 | > 30 | > 30 | > 30 |
| 34 | < 0,01 | > 30 | > 30 | > 30 | > 30 | > 30 |
| 35 | < 0,01 | > 30 | > 30 | > 30 | > 30 | > 30 |
| 36 | < 0,001 | > 30 | > 30 | > 30 | > 30 | > 30 |
| 38 | < 0,001 | > 30 | > 30 | > 30 | > 30 | > 30 |
| 39 | < 0,001 | > 30 | | > 30 | > 30 | > 30 |
| 40 | < 0,01 | > 30 | > 30 | > 30 | > 30 | > 30 |
| 44 | < 0,01 | > 30 | > 30 | > 30 | > 30 | > 30 |
| 45 | < 0,05 | > 30 | > 30 | > 30 | > 30 | > 30 |
| 46 | < 0,05 | > 30 | > 30 | > 30 | > 30 | > 30 |
| 51 | < 0,01 | > 30 | > 30 | > 30 | > 30 | > 30 |
| 54 | < 0,01 | > 30 | | > 30 | > 30 | > 30 |
| 57 | < 0,05 | > 30 | > 30 | > 30 | > 30 | > 30 |
| 58 | < 0,01 | > 30 | > 30 | > 30 | > 30 | > 30 |
| 60 | < 0,01 | > 30 | > 30 | > 30 | > 30 | > 30 |
| 61 | < 0,01 | > 30 | | > 30 | | > 30 |
| 62 | < 0,01 | > 30 | > 30 | > 30 | > 30 | > 30 |

Für die übrigen Verbindungsbeispiele konnten bereits folgende vorteilhaften Werte festgestellt werden:
Bezüglich mTOR weisen die folgenden Verbindungsbeispiele einen mindestens 2000-fach höhren IC50 (ATM) auf (in Bezug auf IC50 (mTOR)), wobei bei einigen Verbindungsbeispielen das Verhältnis IC50 (ATM) : IC50(mTOR) sogar 100.000 übersteigt: 114, 120, 122, 125, 126, 136, 138, 157, 159, 201.

Bezüglich PI3Kalpha weisen die folgenden Verbindungsbeispiele einen mindestens 2000-fach höheren IC50 (ATM) auf: 66, 67, 105, 108, 110, 114, 117, 120, 122, 123, 125, 126, 155, 201, 205.

Bezüglich PI3Kbeta weisen die folgenden Verbindungsbeispiele einen mindestens 2000-fach höheren IC50 (ATM) auf (in Bezug auf IC50 (PI3Kbeta)), wobei bei einigen Verbindungsbeispielen das Verhältnis IC50 (ATM) : IC50(PI3Kbeta) 10.000 übersteigt: 108, 125, 136, 137, 159, 201.

Bezüglich PI3Kdelta weisen die folgenden Verbindungsbeispiele einen mindestens 2000-fach höheren IC50 (ATM) auf: 66, 67, 105, 108, 110, 114, 117, 120, 122, 123, 125, 126, 155, 201, 205.

Bezüglich PI3Kgamma weisen die folgenden Verbindungsbeispiele einen mindestens 2000-fach höheren IC50 (ATM) auf: 66, 67, 105, 108, 110, 114, 117, 120, 122, 123, 125, 126, 136, 155, 201, 205.

### BEISPIEL 15: Weitere Verbindungen, die entsprechend bzw. analog zu den BEISPIELEN 1 bis 12 hergestellt werden können, finden sich in der nachfolgenden Tabelle 4.

**Tabelle 4**

| | | |
|---|---|---|
| 302 | 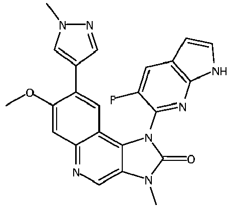 | 1-(5-Fluor-1H-pyrrolo[2,3-b]pyridin-6-yl)-7-methoxy-3-methyl-8-(1-methyl-1H-pyrazol-4-yl)-1,3-dihydro-imidazo[4,5-c]chinolin-2-on |
| 303 | 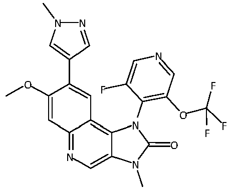 | 1-[3-Fluor-5-(trifluormethoxy)-4-pyridyl]-7-methoxy-3-methyl-8-(1-methyl-1H-pyrazol-4-yl)-1,3-dihydro-imidazo[4,5-c] chinolin-2-on |
| 304 | 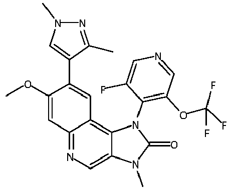 | 1-[3-Fluor-5-(trifluormethoxy)-4-pyridyl]-7-methoxy-3-methyl-8-(1,3-dimethylpyrazol-4-yl)-1,3-dihydro-imidazo[4,5-c]ch inolin-2-on |
| 400 | 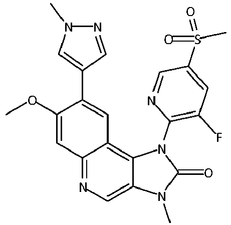 | 1-(3-Fluor-5-methylsulfonyl-pyridin-2-yl)-7-methoxy-3-methyl-8-(1-methyl-1H-pyrazol-4-yl)-1H,2H,3H-imidazo[4,5-c]chinolin-2-on |
| 401 | 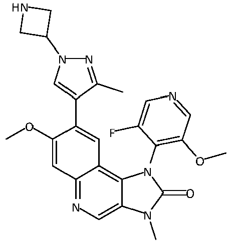 | 8-[1-(Azetidin-3-yl)-3-methyl-1H-pyrazol-4-yl]-1-(3-fluor-5-methoxypyridin-4-yl)-7-methoxy-3-methyl-1H,2H,3H-imidazo[4,5-c]chinolin-2-on |

### BEISPIEL 16: Pharmazeutische Zusammensetzungen

### Beispiel A: Iniektionsgläser

Eine Lösung von 100 g erfindungsgemäßem Wirkstoff und 5 g Dinatriumhydrogenphosphat wird in 3 I zweifach destilliertem Wasser mit 2 N Salzsäure auf pH 6,8 eingestellt, steril filtriert, in Injektionsgläser abgefüllt, unter sterilen Bedingungen lyophilisiert und steril verschlossen. Jedes Injektionsglas enthält 5 mg erfindungsgemäßen Wirkstoff.

### Beispiel B: Suppositorien

Man schmilzt ein Gemisch von 20 g erfindungsgemäßem Wirkstoff mit 100 g Sojalecithin und 1400 g Kakaobutter, gießt es in Formen und lässt es erkalten. Jedes Suppositorium enthält 20 mg erfindungsgemäßen Wirkstoff.

### Beispiel C: Lösung

Man bereitet eine Lösung aus 1 g erfindungsgemäßem Wirkstoff, 9,38 g NaH₂PO₄ *2 H₂O, 28,48 g Na₂HPO₄*12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg erfindungsgemäßen Wirkstoff mit 99,5 g Vaseline unter aseptischen Bedingungen.

### Beispiel E: Tabletten

Ein Gemisch von 1 kg erfindungsgemäßem Wirkstoff, 4 kg Laktose, 1,2 kg Kartoffelstärke, 0,2 kg Talk und 0,1 kg Magnesiumstearat wird in üblicher Weise zu Tabletten verpresst, derart, dass jede Tablette 10 mg erfindungsgemäßen Wirkstoff enthält

### Beispiel F: Dragees

Analog Beispiel E werden Tabletten gepresst, die danach in üblicher Weise mit einem Überzug aus Saccharose, Kartoffelstärke, Talk, Tragant und Farbstoff überzogen werden.

### Beispiel G: Kapseln

2 kg erfindungsgemäßer Wirkstoff werden in üblicher Weise in Hartgelatinekapseln gefüllt, so dass jede Kapsel 20 mg erfindungsgemäßen Wirkstoff enthält.

### Beispiel H: Ampullen

Eine Lösung von 1 kg erfindungsgemäßem Wirkstoff in 60 I zweifach destilliertem Wasser wird steril filtriert, in Ampullen abgefüllt, unter sterilen Bedingungen lyophilisiert und steril verschlossen. Jede Ampulle enthält10 mg erfindungsgemäßen Wirkstoff.

### Beispiel I: Inhalations-Spray

Man löst 14 g erfindungsgemäßen Wirkstoff in 10 I isotonischer NaCl-Lösung und füllt die Lösung in handelsübliche Sprühgefäße mit Pump-Mechanismus. Die Lösung kann in Mund oder Nase gesprüht werden. Ein Sprühstoß (ca. 0,1 ml) entspricht einer Dosis von ca. 0,14 mg.

## Patentansprüche

1. Verbindung, ausgewählt aus:
| | |
|---|---|
| 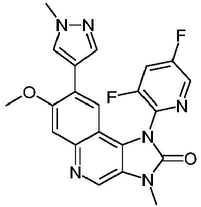 | 1-(3,5-Difluor-pyridin-2-yl)-7-methoxy-3-methyl-8-(1-methyl-1H-pyrazol-4-yl)-1,3-dihydro-imidazo[4,5-c]chinolin-2-on |
| 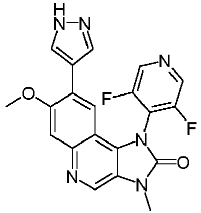 | 1-(3,5-Difluor-pyridin-4-yl)-7-methoxy-3-methyl-8-(1H-pyrazol-4-yl)-1,3-dihydro-imidazo[4,5-c]chinolin-2-on |
| 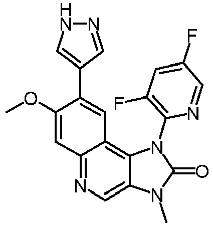 | 1-(3,5-Difluor-pyridin-2-yl)-7-methoxy-3-methyl-8-(1H-pyrazol-4-yl)-1,3-dihydro-imidazo[4,5-c]chinolin-2-on |
| 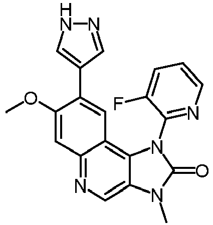 | 1-(3-Fluor-pyridin-2-yl)-7-methoxy-3-methyl-8-(1H-pyrazol-4-yl)-1,3-dihydro-imidazo[4,5-c]chinolin-2-on |
| 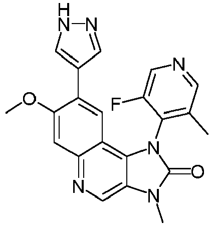 | 1-(3-Fluor-5-methyl-pyridin-4-yl)-7-methoxy-3-methyl-8-(1H-pyrazol-4-yl)-1,3-dihydro-imidazo[4,5-c]chinolin-2-on |
| 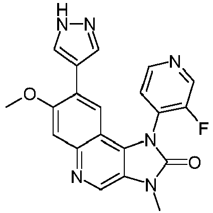 | 1-(3-Fluor-pyridin-4-yl)-7-methoxy-3-methyl-8-(1H-pyrazol-4-yl)-1,3-dihydro-imidazo[4,5-c]chinolin-2-on |
| 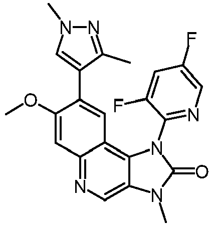 | 1-(3,5-Difluor-pyridin-2-yl)-8-(1,3-dimethyl-1H-pyrazol-4-yl)-7-methoxy-3-methyl-1, 3-dihydro-imidazo[4,5-c]chinolin-2-on |
| 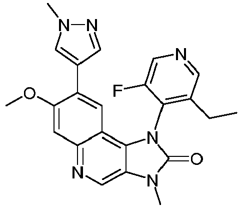 | 1-(3-Ethyl-5-fluor-pyridin-4-yl)-7-methoxy-3-methyl-8-(1-methyl-1H-pyrazol-4-yl)-1,3-dihydro-imidazo[4,5-c]chinolin-2-on |
| 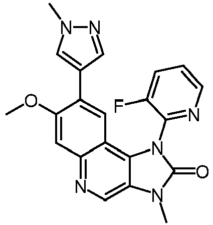 | 1-(3-Fluor-pyridin-2-yl)-7-methoxy-3-methyl-8-(1-methyl-1H-pyrazol-4-yl)-1,3-dihydro-imidazo[4,5-c]chinolin-2-on |
| 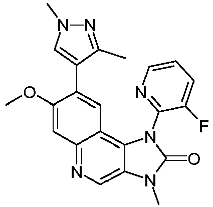 | 8-(1,3-Dimethyl-1H-pyrazol-4-yl)-1-(3-fluor-pyridin-2-yl)-7-methoxy-3-methyl-1,3-dihydro-imidazo[4,5-c]chinolin-2-on |
| 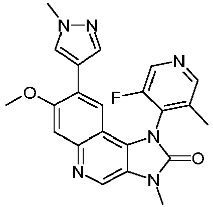 | 1-(3-Fluor-5-methyl-pyridin-4-yl)-7-methoxy-3-methyl-8-(1-methyl-1H-pyrazol-4-yl)-1,3-dihydro-imidazo[4,5-c]chinolin-2-on |
| 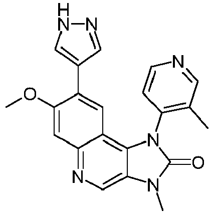 | 7-Methoxy-3-methyl-1-(3-methyl-pyridin-4-yl)-8-(1H-pyrazol-4-yl)-1,3-dihydro-imidazo[4,5-c]chinolin-2-on |
| 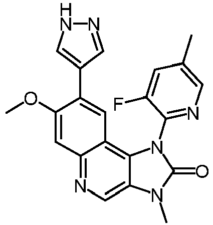 | 1-(3-Fluor-5-methyl-pyridin-2-yl)-7-methoxy-3-methyl-8-(1H-pyrazol-4-yl)-1,3-dihydro-imidazo[4,5-c]chinolin-2-on |
| 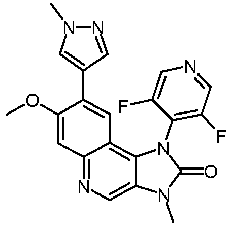 | 1-(3,5-Difluor-pyridin-4-yl)-7-methoxy-3-methyl-8-(1-methyl-1H-pyrazol-4-yl)-1,3-dihydro-imidazo[4,5-c]chinolin-2-on |
| 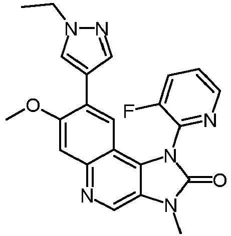 | 8-(1-Ethyl-1H-pyrazol-4-yl)-1-(3-fluor-pyridin-2-yl)-7-methoxy-3-methyl-1,3-dihydro-imidazo[4,5-c]chinolin-2-on |
| 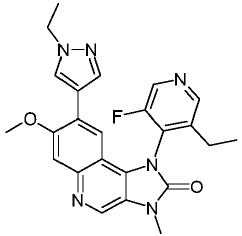 | 1-(3-Ethyl-5-fluor-pyridin-4-yl)-8-(1-ethyl-1H-pyrazol-4-yl)-7-methoxy-3-methyl-1,3-dihydro-imidazo[4,5-c]chinolin-2-on |
| 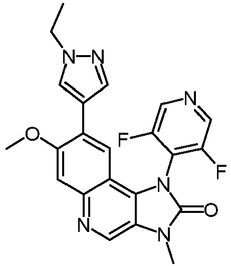 | 1-(3,5-Difluor-pyridin-4-yl)-8-(1-ethyl-1H-pyrazol-4-yl)-7-methoxy-3-methyl-1,3-dihydro-imidazo[4,5-c]chinolin-2-on |
| 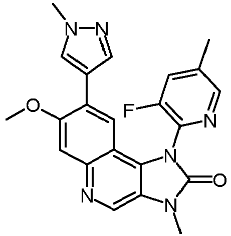 | 1-(3-Fluor-5-methyl-pyridin-2-yl)-7-methoxy-3-methyl-8-(1-methyl-1H-pyrazol-4-yl)-1,3-dihydro-imidazo[4,5-c]chinolin-2-on |
| 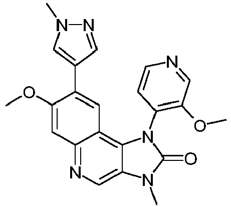 | 7-Methoxy-1-(3-methoxy-pyridin-4-yl)-3-methyl-8-(1-methyl-1H-pyrazol-4-yl)-1,3-dihydro-imidazo[4,5-c]chinolin-2-on |
| 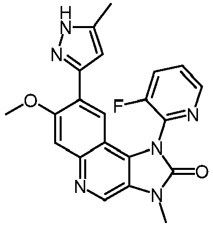 | 1-(3-Fluor-pyridin-2-yl)-7-methoxy-3-methyl-8-(5-methyl-1H-pyrazol-3-yl)-1,3-dihydro-imidazo[4,5-c]chinolin-2-on |
| 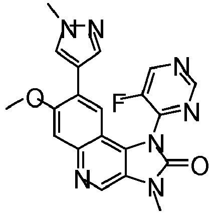 | 1-(5-Fluorpyrimidin-4-yl)-7-methoxy-3-methyl-8-(1-methyl-1H-pyrazol-4-yl)-1H,2H,3H-imidazo[4,5-c]chinolin-2-on |
| 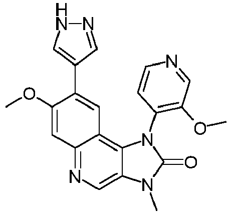 | 7-Methoxy-1-(3-methoxy-pyridin-4-yl)-3-methyl-8-(1H-pyrazol-4-yl)-1,3-dihydro-imidazo[4,5-c]chinolin-2-on |
| 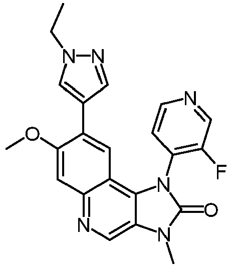 | 8-(1-Ethyl-1H-pyrazol-4-yl)-1-(3-fluor-pyridin-4-yl)-7-methoxy-3-methyl-1,3-dihydro-imidazo[4,5-c]chinolin-2-on |
| 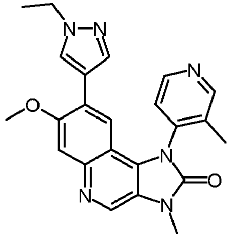 | 8-(1-Ethyl-1H-pyrazol-4-yl)-7-methoxy-3-methyl-1-(3-methyl-pyridin-4-yl)-1,3-dihydro-imidazo[4,5-c]chinolin-2-on |
| 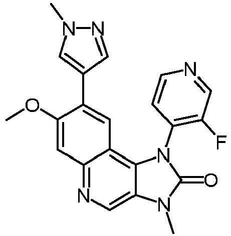 | 1-(3-Fluor-pyridin-4-yl)-7-methoxy-3-methyl-8-(1-methyl-1H-pyrazol-4-yl)-1,3-dihydro-imidazo[4,5-c]chinolin-2-on |
| 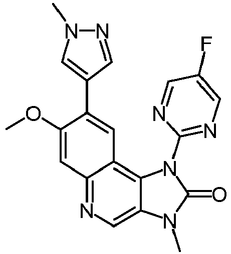 | 1-(5-Fluor-pyrimidin-2-yl)-7-methoxy-3-methyl-8-(1-methyl-1H-pyrazol-4-yl)-1,3-dihydro-imidazo[4,5-c]chinolin-2-on |
| 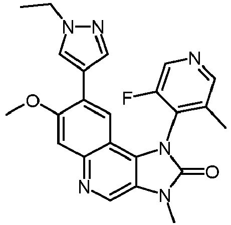 | 8-(1-Ethyl-1H-pyrazol-4-yl)-1-(3-fluor-5-methyl-pyridin-4-yl)-7-methoxy-3-methyl-1,3-dihydro-imidazo[4,5-c]chinolin-2-on |
| 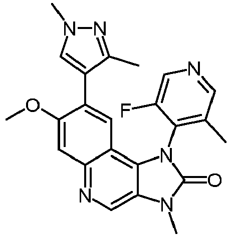 | 8-(1,3-Dimethyl-1H-pyrazol-4-yl)-1-(3-fluor-5-methyl-pyridin-4-yl)-7-methoxy-3-methyl-1,3-dihydro-imidazo[4,5-c]chinolin-2-on |
| 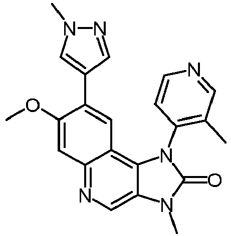 | 7-Methoxy-3-methyl-8-(1-methyl-1H-pyrazol-4-yl)-1-(3-methyl-pyridin-4-yl)-1,3-dihydro-imidazo[4,5-c]chinolin-2-on |
| 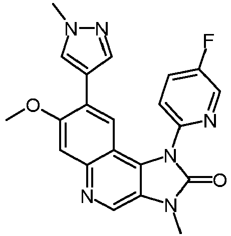 | 1-(5-Fluor-pyridin-2-yl)-7-methoxy-3-methyl-8-(1-methyl-1H-pyrazol-4-yl)-1,3-dihydro-imidazo[4,5-c]chinolin-2-on |
| 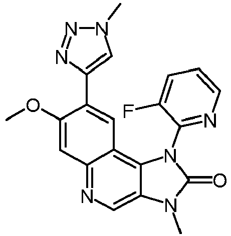 | 1-(3-Fluor-pyridin-2-yl)-7-methoxy-3-methyl-8-(1-methyl-1H-[1,2,3]triazol-4-yl)-1,3-dihydro-imidazo[4,5-c]chinolin-2-on |
| 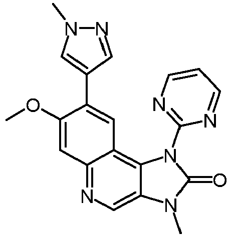 | 7-Methoxy-3-methyl-8-(1-methyl-1H-pyrazol-4-yl)-1-pyrimidin-2-yl-1,3-dihydro-imidazo[4,5-c]chinolin-2-on |
| 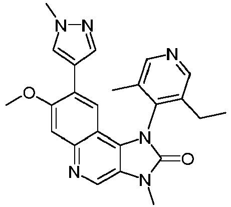 | 1-(3-Ethyl-5-methyl-pyridin-4-yl)-7-methoxy-3-methyl-8-(1-methyl-1H-pyrazol-4-yl)-1,3-dihydro-imidazo[4,5-c]chinolin-2-on |
| 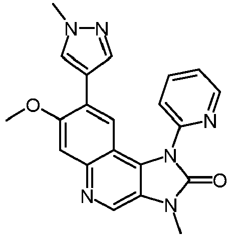 | 7-Methoxy-3-methyl-8-(1-methyl-1H-pyrazol-4-yl)-1-pyridin-2-yl-1,3-dihydro-imidazo[4,5-c]chinolin-2-on |
| 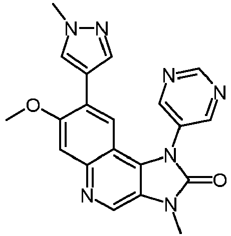 | 7-Methoxy-3-methyl-8-(1-methyl-1H-pyrazol-4-yl)-1-pyrimidin-5-yl-1,3-dihydro-imidazo[4,5-c]chinolin-2-on |
| 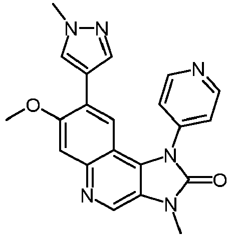 | 7-Methoxy-3-methyl-8-(1-methyl-1H-pyrazol-4-yl)-1-pyridin-4-yl-1,3-dihydro-imidazo[4,5-c]chinolin-2-on |
| 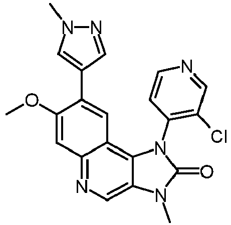 | 1-(3-Chlor-pyridin-4-yl)-7-methoxy-3-methyl-8-(1-methyl-1H-pyrazol-4-yl)-1,3-dihydro-imidazo[4,5-c]chinolin-2-on |
| 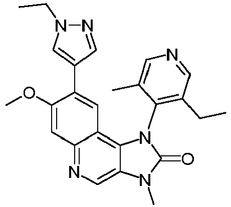 | 1-(3-Ethyl-5-methyl-pyridin-4-yl)-8-(1-ethyl-1H-pyrazol-4-yl)-7-methoxy-3-methyl-1, 3-dihydro-imidazo[4,5-c]chinolin-2-on |
| 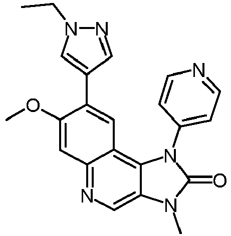 | 8-(1-Ethyl-1H-pyrazol-4-yl)-7-methoxy-3-methyl-1-pyridin-4-yl-1,3-dihydro-imidazo[4,5-c]chinolin-2-on |
| 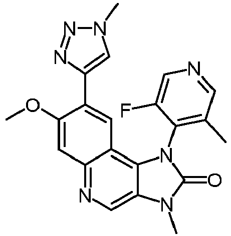 | 1-(3-Fluor-5-methyl-pyridin-4-yl)-7-methoxy-3-methyl-8-(1-methyl-1H-[1,2,3]triazol-4-yl)-1,3-dihydro-imidazo[4,5-c]chinolin-2-on |
| 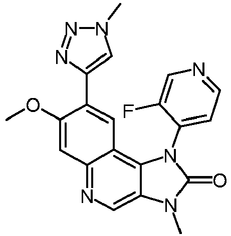 | 1-(3-Fluor-pyridin-4-yl)-7-methoxy-3-methyl-8-(1-methyl-1H-[1,2,3]triazol-4-yl)-1,3-dihydro-imidazo[4,5-c]chinolin-2-on |
| 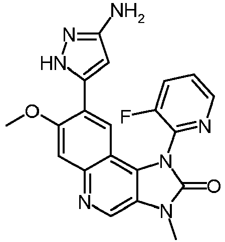 | 8-(3-Amino-1H-pyrazol-5-yl)-1-(3-fluor-2-pyridyl)-7-methoxy-3-methyl-imidazo[4,5-c]chinolin-2-on |
| 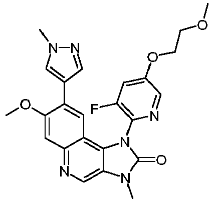 | 1-[3-Fluor-5-(2-methoxyethoxy)-2-pyridyl]-7-methoxy-3-methyl-8-(1-methylpyrazol-4-yl)imidazo[4,5-c]chinolin-2-on |
| 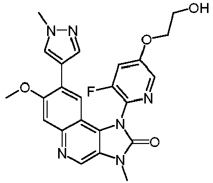 | 1-[3-Fluor-5-(2-hydroxyethoxy)-2-pyridyl]-7-methoxy-3-methyl-8-(1-methylpyrazol-4-yl)imidazo[4,5-c]chinolin-2-on |
| 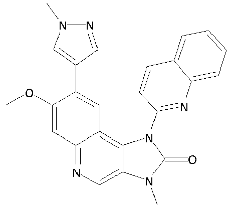 | 7-Methoxy-3-methyl-8-(1-methyl-1H-pyrazol-4-yl)-1-chinolin-2-yl-1,3-dihydro-imidazo[4,5-c]chinolin-2-on |
| 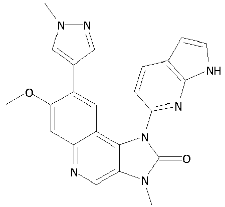 | 7-Methoxy-3-methyl-8-(1-methyl-1H-pyrazol-4-yl)-1-(1H-pyrrolo[2,3-b]pyridin-6-yl)1,3-dihydro-imidazo[4,5-c]chinolin-2-on |
| 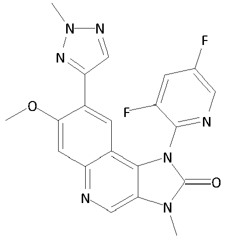 | 1-(3,5-Difluor-pyridin-2-yl)-7-methoxy-3-methyl-8-(2-methyl-2H-[1,2,3]triazol-4-yl)-1,3-dihydro-imidazo[4,5-c] chinolin-2-on |
| 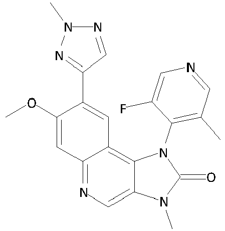 | 1-(3-Fluor-5-methyl-pyridin-4-yl)-7-methoxy-3-methyl-8-(2-methyl-2H-[1,2,3]triazol-4-yl)-1,3-dihydro-imidazo[4,5-c] chinolin-2-on |
| 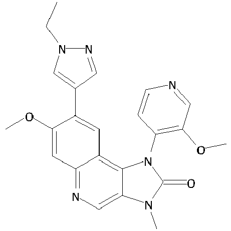 | 8-(1-Ethyl-1H-pyrazol-4-yl)-7-methoxy-1-(3-methoxy-pyridin-4-yl)-3-methyl-1,3-dihydro-imidazo[4,5-c] chinolin-2-on |
| 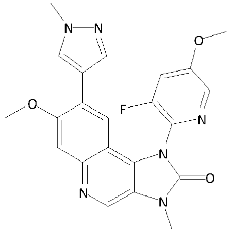 | 1-(3-Fluor-5-methoxy-pyridin-2-yl)-7-methoxy-3-methyl-8-(1-methyl-1H-pyrazol-4-yl)-1,3-dihydro-imidazo[4,5-c]chinolin-2-on |
| 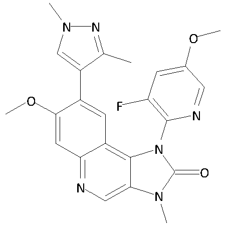 | 8-(1,3-Dimethyl-1H-pyrazol-4-yl)-1-(3-fluor-5-methoxy-pyridin-2-yl)-7-methoxy-3-methyl-1,3-dihydro-imidazo[4,5-c]chinolin-2-on |
| 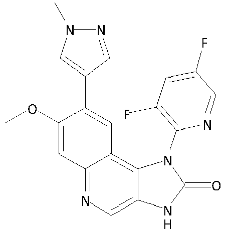 | 1-(3,5-Difluor-pyridin-2-yl)-7-methoxy-8-(1-methyl-1H-pyrazol-4-yl)-1,3-dihydro-imidazo[4,5-c]chinolin-2-on |
| 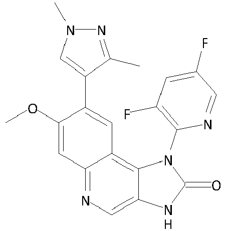 | 1-(3,5-Difluor-pyridin-2-yl)-8-(1,3-dimethyl-1H-pyrazol-4-yl)-7-methoxy-1,3-dihydro-imidazo[4,5-c]chinolin-2-on |
| 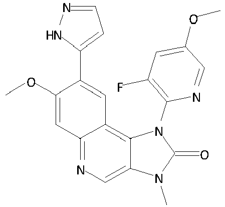 | 1-(3-Fluor-5-methoxy-pyridin-2-yl)-7-methoxy-3-methyl-8-(2H-pyrazol-3-yl)-1 ,3-dihydro-imidazo[4,5-c] chinolin-2-on |
| 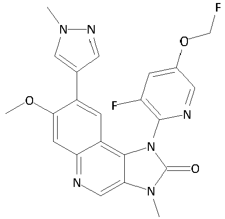 | 1-(3-Fluor-5-fluormethoxy-pyridin-2-yl)-7-methoxy-3-methyl-8-(1-methyl-1H-pyrazol-4-yl)-1,3-dihydro-imidazo[4,5-c]chinolin-2-on |
| 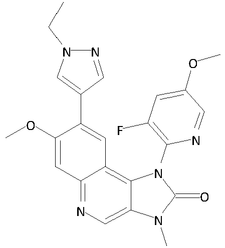 | 8-(1-Ethyl-1H-pyrazol-4-yl)-1-(3-fluor-5-methoxy-pyridin-2-yl)-7-methoxy-3-methyl-1,3-dihydro-imidazo[4,5-c] chinolin-2-on |
| 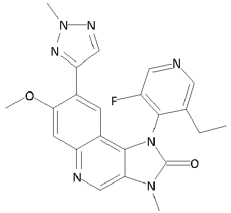 | 1-(3-Ethyl-5-fluor-pyridin-4-yl)-7-methoxy-3-methyl-8-(2-methyl-2H-[1,2,3]triazol-4-yl)-1,3-dihydro-imidazo[4,5-c] chinolin-2-on |
| 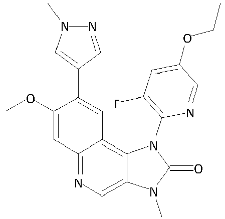 | 1-(5-Ethoxy-3-fluor-pyridin-2-yl)-7-methoxy-3-methyl-8-(1-methyl-1H-pyrazol-4-yl)-1,3-dihydro-imidazo[4,5-c]chinolin-2-on |
| 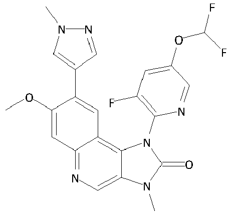 | 1-(5-Difluormethoxy-3-fluor-pyridin-2-yl)-7-methoxy-3-methyl-8-(1-methyl-1H-pyrazol-4-yl)-1,3-dihydro-imidazo[4,5-c]chinolin-2-on |
| 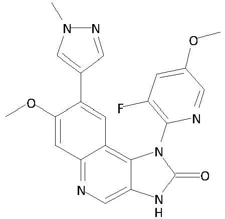 | 1-(3-Fluor-5-methoxy-pyridin-2-yl)-7-methoxy-8-(1-methyl-1H-pyrazol-4-yl)-1,3-dihydro-imidazo[4,5-c]chinolin-2-on |
| 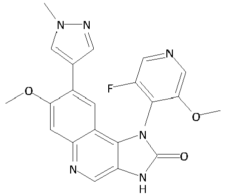 | 1-(3-Fluor-5-methoxy-pyridin-4-yl)-7-methoxy-8-(1-methyl-1H-pyrazol-4-yl)-1,3-dihydro-imidazo[4,5-c]chinolin-2-on |
| 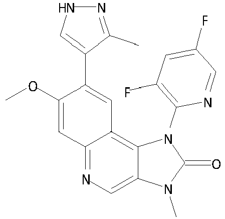 | 1-(3,5-Difluor-pyridin-2-yl)-7-methoxy-3-methyl-8-(3-methyl-1H-pyrazol-4-yl)-1, 3-dihydro-imidazo[4,5-c]chinolin-2-on |
| 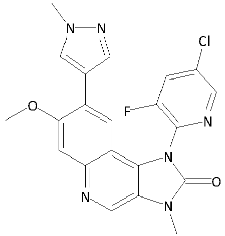 | 1-(5-Chlor-3-fluor-pyridin-2-yl)-7-methoxy-3-methyl-8-(1-methyl-1H-pyrazol-4-yl)-1,3-dihydro-imidazo[4,5-c]chinolin-2-on |
| 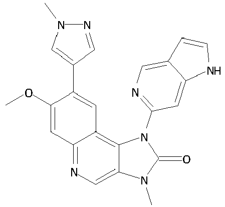 | 7-Methoxy-3-methyl-8-(1-methyl-1H-pyrazol-4-yl)-1-(1H-pyrrolo[3,2-c]pyridin-6-yl)-1,3-dihydro-imidazo[4,5-c]chinolin-2-on |
| 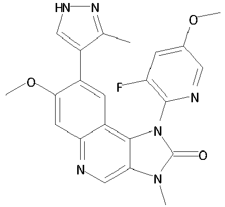 | 1-(3-Fluor-5-methoxy-pyridin-2-yl)-7-methoxy-3-methyl-8-(3-methyl-1H-pyrazol-4-yl)-1,3-dihydro-imidazo[4,5-c] chinolin-2-on |
| 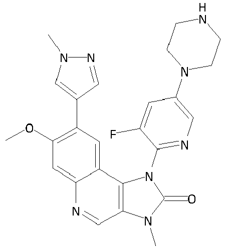 | 1-(3-Fluor-5-piperazin-1-yl-pyridin-2-yl)-7-methoxy-3-methyl-8-(1-methyl-1H-pyrazol-4-yl)-1,3-dihydro-imidazo[4,5-c]chinolin-2-on |
| 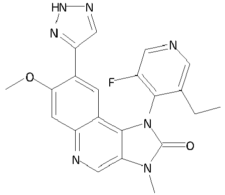 | 1-(3-Ethyl-5-fluor-pyridin-4-yl)-7-methoxy-3-methyl-8-(2H-[1,2,3]triazol-4-yl)-1,3-dihydro-imidazo[4,5-c] chinolin-2-on |
| 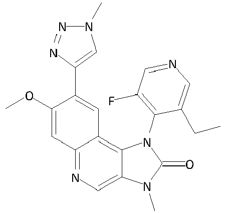 | 1-(3-Ethyl-5-fluor-pyridin-4-yl)-7-methoxy-3-methyl-8-(1-methyl-1H-[1,2,3]triazol-4-yl)-1,3-dihydro-imidazo[4,5-c] chinolin-2-on |
| 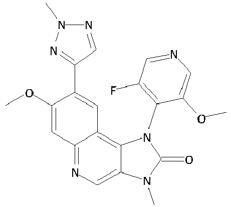 | 1-(3-Fluor-5-methoxy-pyridin-4-yl)-7-methoxy-3-methyl-8-(2-methyl-2H-[1,2,3]triazol-4-yl)-1,3-dihydro-imidazo[4,5-c] chinolin-2-on |
| 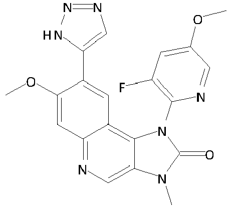 | 1-(3-Fluor-5-methoxy-pyridin-2-yl)-7-methoxy-3-methyl-8-(3H-[1,2,3]triazol-4-yl)-1,3-dihydro-imidazo[4,5-c] chinolin-2-on |
| 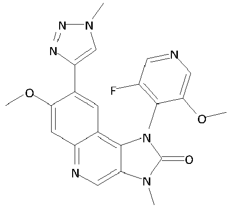 | 1-(3-Fluor-5-methoxy-pyridin-4-yl)-7-methoxy-3-methyl-8-(1-methyl-1H-[1,2,3]triazol-4-yl)-1,3-dihydro-imidazo[4,5-c]chinolin-2-on |
| 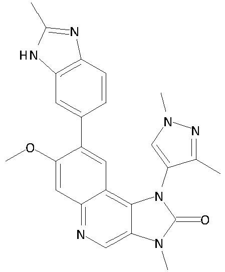 | 1-(1,3-Dimethyl-1H-pyrazol-4-yl)-7-methoxy-3-methyl-8-(2-methyl-3H-benzoimidazol-5-yl)-1,3-dihydro-imidazo[4,5-c]chinolin-2-on |
| 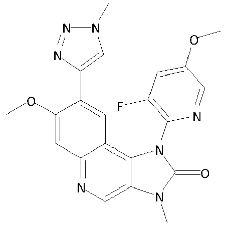 | 1-(3-Fluor-5-methoxy-pyridin-2-yl)-7-methoxy-3-methyl-8-(1-methyl-1H-[1,2,3]triazol-4-yl)-1,3-dihydro-imidazo[4,5-c] chinolin-2-on |
| 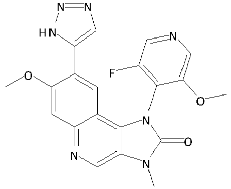 | 1-(3-Fluor-5-methoxy-pyridin-4-yl)-7-methoxy-3-methyl-8-(3H-[1,2,3]triazol-4-yl)-1,3-dihydro-imidazo[4,5-c] chinolin-2-on |
| 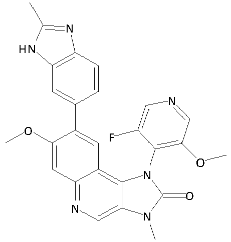 | 1-(3-Fluor-5-methoxy-pyridin-4-yl)-7-methoxy-3-methyl-8-(2-methyl-3H-benzoimidazol-5-yl)-1,3-dihydro-imidazo[4,5-c]chinolin-2-on |
| 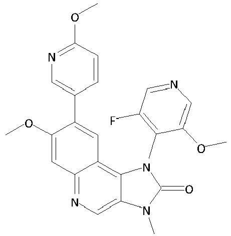 | 1-(3-Fluor-5-methoxy-pyridin-4-yl)-7-methoxy-8-(6-methoxy-pyridin-3-yl)-3-methyl-1,3-dihydro-imidazo[4,5-c]chinolin-2-on |
| 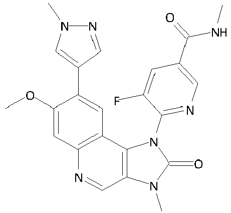 | 5-Fluor-6-[7-methoxy-3-methyl-8-(1-methyl-1 H-pyrazol-4-yl)-2-oxo-2,3-dihydro-imidazo[4,5-c]chinolin-1-yl]-N-methyl-nicotinamid |
| 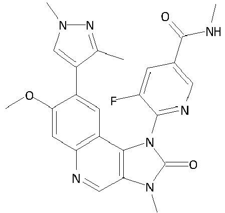 | 6-[8-(1,3-Dimethyl-1H-pyrazol-4-yl)-7-methoxy-3-methyl-2-oxo-2,3-dihydro-imidazo[4,5-c]chinolin-1 -yl]-5-fluor-N-methyl-nicotinamid |
| 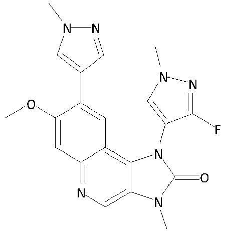 | 1-(3-Fluor-1-methyl-1H-pyrazol-4-yl)-7-methoxy-3-methyl-8-(1-methyl-1H-pyrazol-4-yl)-1,3-dihydro-imidazo[4,5-c]chinolin-2-on |
| 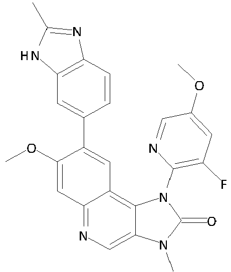 | 1-(3-Fluor-5-methoxy-pyridin-2-yl)-7-methoxy-3-methyl-8-(2-methyl-3H-benzoimidazol-5-yl)-1,3-dihydro-imidazo[4,5-c]chinolin-2-on |
| 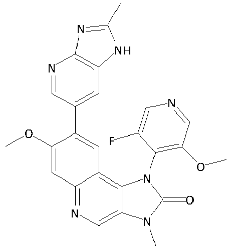 | 1-(3-Fluor-5-methoxy-pyridin-4-yl)-7-methoxy-3-methyl-8-(2-methyl-1H-imidazo[4,5-b]pyridin-6-yl)-1,3-dihydro-imidazo[4,5-c]chinolin-2-on |
| 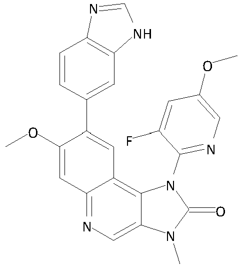 | 8-(3H-Benzimidazol-5-yl)-1-(3-fluor-5-methoxy-pyridin-2-yl)-7-methoxy-3-methyl-1,3-dihydro-imidazo[4,5-c]chinolin-2-on |
| 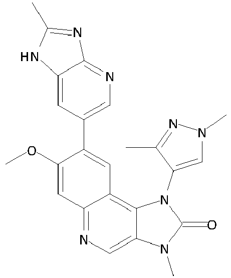 | 1-(1,3-Dimethyl-1H-pyrazol-4-yl)-7-methoxy-3-methyl-8-(2-methyl-1H-imidazo[4,5-b]pyridin-6-yl)-1,3-dihydro-imidazo[4,5-c]chinolin-2-on |
| 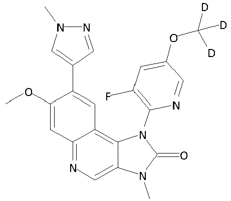 | 1-[3-Fluor-5-(trideuteriomethoxy)-2-pyridyl]-7-methoxy-3-methyl-8-(1-methylpyrazol-4-yl) imidazo[4,5-c]chinolin-2-on |
| 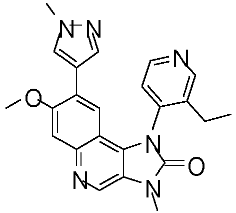 | 1-(3-Ethyl-pyridin-4-yl)-7-methoxy-3-methyl-8-(1-methyl-1H-pyrazol-4-yl)-1,3-dihydro-imidazo[4,5-c] chinolin-2-on |
| 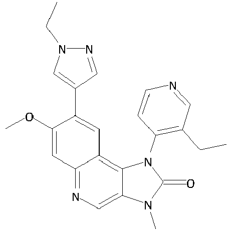 | 8-(1-Ethyl-1H-pyrazol-4-yl)-1-(3-ethyl-pyridin-4-yl)-7-methoxy-3-methyl-1,3-dihydro-imidazo[4,5-c] chinolin-2-on |
| 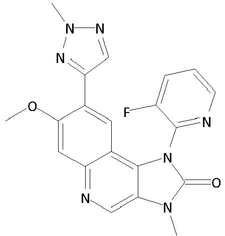 | 1-(3-Fluor-pyridin-2-yl)-7-methoxy-3-methyl-8-(2-methyl-2H-[1,2,3]triazol-4-yl)-1,3-dihydro-imidazo[4,5-c] chinolin-2-on |
| 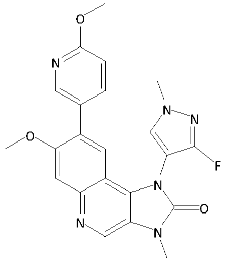 | 1-(3-Fluor-1-methyl-1H-pyrazol-4-yl)-7-methoxy-8-(6-methoxy-pyridin-3-yl)-3-methyl-1,3-dihydro-imidazo[4,5-c]chinolin-2-on |
| 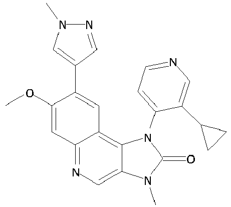 | 1-(3-Cyclopropyl-pyridin-4-yl)-7-methoxy-3-methyl-8-(1-methyl-1H-pyrazol-4-yl)-1,3-dihydro-imidazo[4,5-c] chinolin-2-on |
| 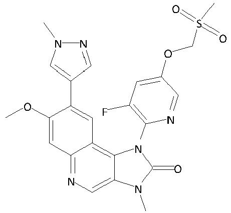 | 1-(3-Fluor-5-methylsulfonylmethoxy-pyridin-2-yl)-7-methoxy-3-methyl-8-(1-methyl-1H-pyrazol-4-yl)-1,3-dihydro-imidazo[4,5-c]chinolin-2-on |
| 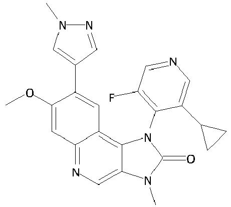 | 1-(3-Cyclopropyl-5-fluor-pyridin-4-yl)-7-methoxy-3-methyl-8-(1-methyl-1H-pyrazol-4-yl)-1,3-dihydro-imidazo[4,5-c]chinolin-2-on |
| 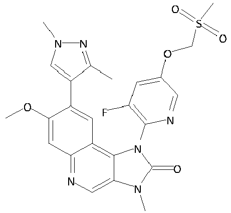 | 8-(1,3-Dimethyl-1H-pyrazol-4-yl)-1-(3-fluor-5-methylsulfonylmethoxy-pyridin-2-yl)-7-methoxy-3-methyl-1,3-dihydro-imidazo[4,5-c]chinolin-2-on |
| 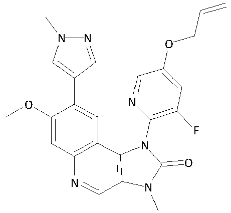 | 1-(5-Allyloxy-3-fluor-pyridin-2-yl)-7-methoxy-3-methyl-8-(1-methyl-1H-pyrazol-4-yl)-1,3-dihydro-imidazo[4,5-c]chinolin-2-on |
| 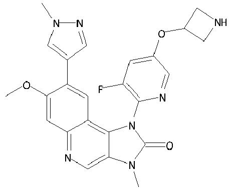 | 1-[5-(Azetidin-3-yloxy)-3-fluor-pyridin-2-yl]-7-methoxy-3-methyl-8-(1-methyl-1H-pyrazol-4-yl)-1,3-dihydro-imidazo[4,5-c]chinolin-2-on |
| 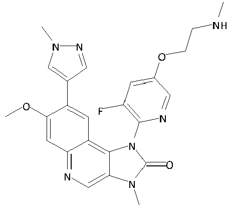 | 1-[3-Fluor-5-(2-methylamino-ethoxy)-pyridin-2-yl]-7-methoxy-3-methyl-8-(1-methyl-1H-pyrazol-4-yl)-1,3-dihydro-imidazo[4,5-c] chinolin-2-on |
| 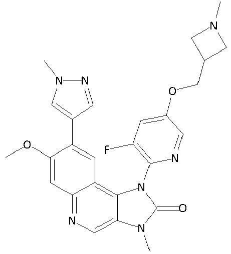 | 1-[3-Fluor-5-(1-methyl-azetidin-3-ylmethoxy)-pyridin-2-yl]-7-methoxy-3-methyl-8-(1-methyl-1H-pyrazol-4-yl)-1,3-dihydro-imidazo[4,5-c]chinolin-2-on |
| 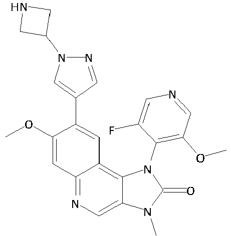 | 8-[1-(Azetidin-3-yl)-1H-pyrazol-4-yl]-1-(3-fluor-5-methoxypyridin-4-yl)-7-methoxy-3-methyl-1H,2H,3H-imidazo[4,5-c]chinolin-2-on |
und/oder pharmazeutisch verwendbares Salz, Solvat oder Tautomer davon.

2. Verbindung nach Anspruch 1 und/oder pharmazeutisch verwendbares Salz, Solvat oder Tautomer davon zur Verwendung als Medikament.

3. Verbindung nach Anspruch 1 und/oder pharmazeutisch verwendbares Salz, Solvat oder Tautomer davon zur Verwendung bei der Behandlung von Krebs, Tumoren und/oder Metastasen.

4. Verbindung und/oder pharmazeutisch verwendbares Salz, Solvat oder Tautomer davon zur Verwendung bei der Behandlung von Krebs, Tumoren und/oder Metastasen nach Anspruch 3 in Kombination mit Radiotherapie.

5. Verbindung und/oder pharmazeutisch verwendbares Salz, Solvat oder Tautomer davon zur Verwendung bei der Behandlung von Krebs, Tumoren und/oder Metastasen nach Anspruch 3 in Kombination mit mindestens einem Antikrebsmittel.

6. Verbindung und/oder pharmazeutisch verwendbares Salz, Solvat oder Tautomer davon zur Verwendung nach einem der Ansprüche 3 bis 5, wobei der Tumor ausgewählt ist aus der Gruppe von Erkrankungen von Plattenepithel, Blase, Magen, Nieren, Kopf, Hals, Ösophagus, Gebärmutterhals, Schilddrüse, Darm, Knochen, Leber, Gehirn, Prostata, Urogenitaltrakts, lymphatisches System, Kehlkopf, Lunge, Haut, Blut und Immunsystem, und/oder der Krebs ausgewählt ist aus der Gruppe von Monozytenleukämie, Lungenadenokarzinom, kleinzelliges Lungenkarzinom, Bauchspeicheldrüsenkrebs, Glioblastom, Darmkarzinom, Brustkarzinom, akute myelotische Leukämie, chronische myelotische Leukämie, akute lymphatische Leukämie, chronische lymphatische Leukämie, Hodgkin-Lymphom und non-Hodgkin-Lymphom.

7. Verbindung nach Anspruch 1 und/oder pharmazeutisch verwendbares Salz, Solvat oder Tautomer davon zur Verwendung in der Sensibilisierung von Krebszellen gegenüber einem Antikrebsmittel und/oder ionisierender Strahlung.

8. Pharmazeutische Zusammensetzung, enthaltend eine wirksame Menge mindestens einer Verbindung nach Anspruch 1 und/oder einem pharmazeutisch verwendbaren Salz, Solvat oder Tautomer davon und mindestens einen pharmazeutisch verträglichen Hilfsstoff.

9. Pharmazeutische Zusammensetzung nach Anspruch 8, ferner enthaltend mindestens einen weiteren Arzneimittelwirkstoff.

10. Pharmazeutische Zusammensetzung nach Anspruch 9, wobei der weitere Arzneimittelwirkstoff ein Antikrebsmittel ist.

11. Kit, bestehend aus getrennten Packungen von
(a) einer wirksamen Menge einer Verbindung nach Anspruch 1 und/oder pharmazeutisch verwendbares Salz, Solvat oder Tautomer, davon, und
(b) einer wirksamen Menge eines weiteren Arzneimittelwirkstoffs.

12. Kit nach Anspruch 11, wobei der weitere Arzneimittelwirkstoff ein Antikrebsmittel ist.

## Claims

1. Compound selected from:
| | |
|---|---|
| 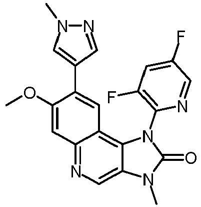 | 1-(3,5-Difluoropyridin-2-yl)-7-methoxy-3-methyl-8-(1-methyl-1H-pyrazol-4-yl)-1,3-dihydroimidazo[4,5-c]quinolin-2-one |
| 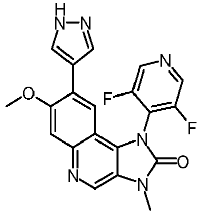 | 1-(3,5-Difluoropyridin-4-yl)-7-methoxy-3-methyl-8-(1H-pyrazol-4-yl)-1,3-dihydro-imidazo[4,5-c]quinolin-2-one |
| 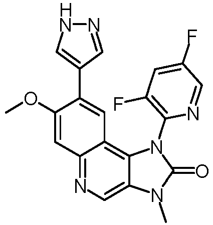 | 1-(3,5-Difluoropyridin-2-yl)-7-methoxy-3-methyl-8-(1H-pyrazol-4-yl)-1,3-dihydro-imidazo[4,5-c]quinolin-2-one |
| 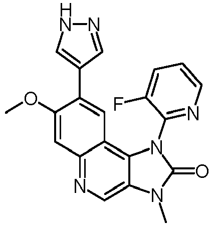 | 1-(3-Fluoropyridin-2-yl)-7-methoxy-3-methyl-8-(1H-pyrazol-4-yl)-1,3-dihydro-imidazo[4,5-c]quinolin-2-one |
| 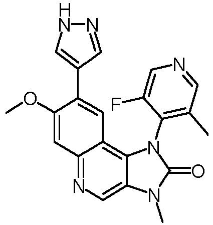 | 1-(3-Fluoro-5-methylpyridin-4-yl)-7-methoxy-3-methyl-8-(1H-pyrazol-4-yl)-1,3-dihydroimidazo[4,5-c]quinolin-2-one |
| 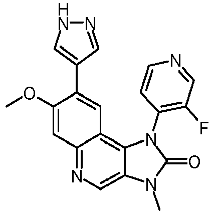 | 1-(3-Fluoropyridin-4-yl)-7-methoxy-3-methyl-8-(1H-pyrazol-4-yl)-1,3-dihydro-imidazo[4,5-c]quinolin-2-one |
| 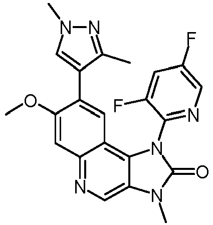 | 1-(3,5-Difluoropyridin-2-yl)-8-(1,3-dimethyl-1H-pyrazol-4-yl)-7-methoxy-3-methyl-1,3-dihydroimidazo[4,5-c]quinolin-2-one |
| 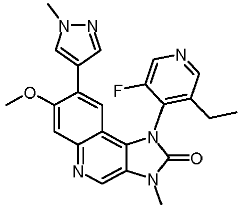 | 1-(3-Ethyl-5-fluoropyridin-4-yl)-7-methoxy-3-methyl-8-(1-methyl-1H-pyrazol-4-yl)-1,3-dihydroimidazo[4,5-c]quinolin-2-one |
| 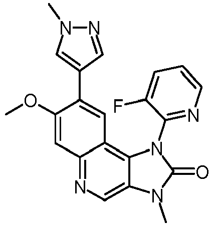 | 1-(3-Fluoropyridin-2-yl)-7-methoxy-3-methyl-8-(1-methyl-1H-pyrazol-4-yl)-1,3-dihydroimidazo[4,5-c]quinolin-2-one |
| 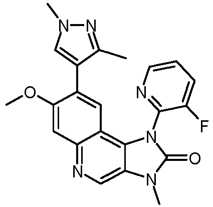 | 8-(1,3-Dimethyl-1H-pyrazol-4-yl)-1-(3-fluoro-pyridin-2-yl)-7-methoxy-3-methyl-1,3-dihydroimidazo[4,5-c]quinolin-2-one |
| 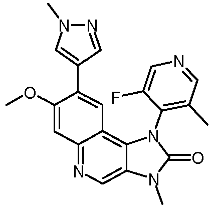 | 1-(3-Fluoro-5-methylpyridin-4-yl)-7-methoxy-3-methyl-8-(1-methyl-1H-pyrazol-4-yl)-1,3-dihydroimidazo[4,5-c]quinolin-2-one |
| 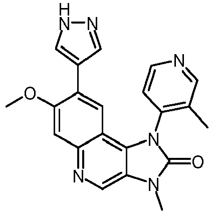 | 7-Methoxy-3-methyl-1-(3-methylpyridin-4-yl)-8-(1H-pyrazol-4-yl)-1,3-dihydroimidazo-[4,5-c]quinolin-2-one |
| 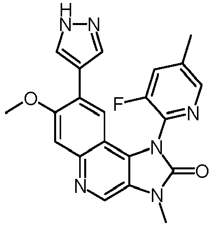 | 1-(3-Fluoro-5-methylpyridin-2-yl)-7-methoxy-3-methyl-8-(1H-pyrazol-4-yl)-1,3-dihydroimidazo[4,5-c]quinolin-2-one |
| 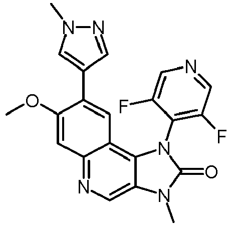 | 1-(3,5-Difluoropyridin-4-yl)-7-methoxy-3-methyl-8-(1-methyl-1H-pyrazol-4-yl)-1,3-dihydroimidazo[4,5-c]quinolin-2-one |
| 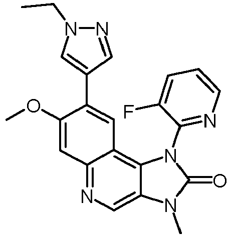 | 8-(1-Ethyl-1H-pyrazol-4-yl)-1-(3-fluoro-pyridin-2-yl)-7-methoxy-3-methyl-1,3-dihydroimidazo[4,5-c]quinolin-2-one |
| 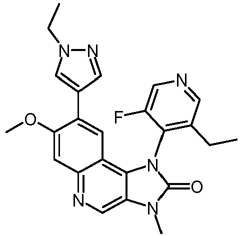 | 1-(3-Ethyl-5-fluoropyridin-4-yl)-8-(1-ethyl-1H-pyrazol-4-yl)-7-methoxy-3-methyl-1,3-dihydroimidazo[4,5-c]quinolin-2-one |
| 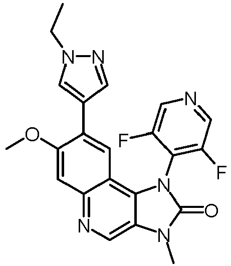 | 1-(3,5-Difluoropyridin-4-yl)-8-(1-ethyl-1H-pyrazol-4-yl)-7-methoxy-3-methyl-1,3-dihydroimidazo[4,5-c]quinolin-2-one |
| 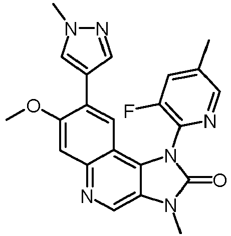 | 1-(3-Fluoro-5-methylpyridin-2-yl)-7-methoxy-3-methyl-8-(1-methyl-1H-pyrazol-4-yl)-1,3-dihydroimidazo[4,5-c]quinolin-2-one |
| 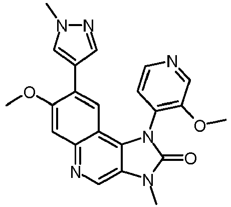 | 7-Methoxy-1-(3-methoxypyridin-4-yl)-3-methyl-8-(1-methyl-1H-pyrazol-4-yl)-1,3-dihydroimidazo[4,5-c]quinolin-2-one |
| 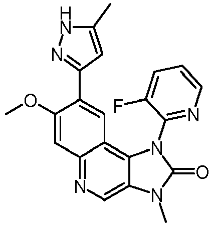 | 1-(3-Fluoropyridin-2-yl)-7-methoxy-3-methyl-8-(5-methyl-1H-pyrazol-3-yl)-1,3-dihydroimidazo[4,5-c]quinolin-2-one |
| 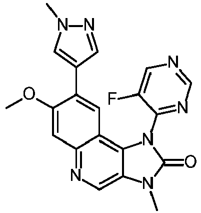 | 1-(5-Fluoropyrimidin-4-yl)-7-methoxy-3-methyl-8-(1-methyl-1H-pyrazol-4-yl)-1H,2H,3H-imidazo[4,5-c]quinolin-2-one |
| 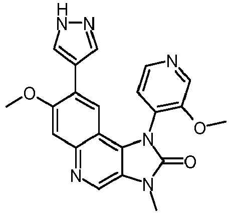 | 7-Methoxy-1-(3-methoxypyridin-4-yl)-3-methyl-8-(1H-pyrazol-4-yl)-1,3-dihydro-imidazo[4,5-c]quinolin-2-one |
| 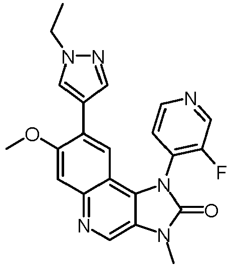 | 8-(1-Ethyl-1H-pyrazol-4-yl)-1-(3-fluoro-pyridin-4-yl)-7-methoxy-3-methyl-1,3-dihydroimidazo[4,5-c]quinolin-2-one |
| 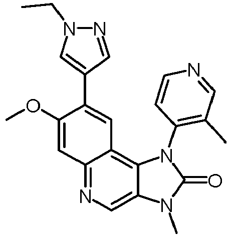 | 8-(1-Ethyl-1H-pyrazol-4-yl)-7-methoxy-3-methyl-1-(3-methylpyridin-4-yl)-1,3-dihydro-imidazo[4,5-c]quinolin-2-one |
| 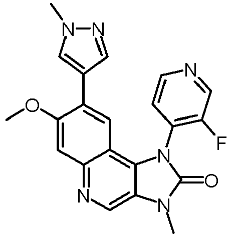 | 1-(3-Fluoropyridin-4-yl)-7-methoxy-3-methyl-8-(1-methyl-1H-pyrazol-4-yl)-1,3-dihydroimidazo[4,5-c]quinolin-2-one |
| 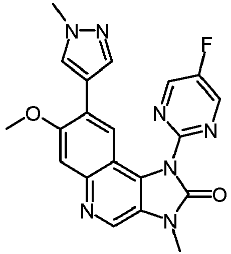 | 1-(5-Fluoropyrimidin-2-yl)-7-methoxy-3-methyl-8-(1-methyl-1H-pyrazol-4-yl)-1,3-dihydroimidazo[4,5-c]quinolin-2-one |
| 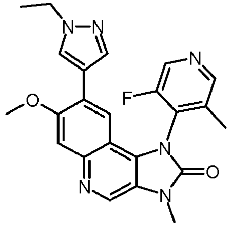 | 8-(1-Ethyl-1H-pyrazol-4-yl)-1-(3-fluoro-5-methylpyridin-4-yl)-7-methoxy-3-methyl-1,3-dihydroimidazo[4,5-c]quinolin-2-one |
| 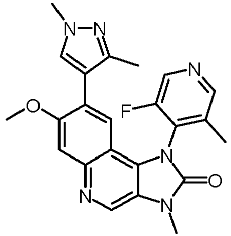 | 8-(1,3-Dimethyl-1H-pyrazol-4-yl)-1-(3-fluoro-5-methylpyridin-4-yl)-7-methoxy-3-methyl-1,3-dihydroimidazo[4,5-c]quinolin-2-one |
| 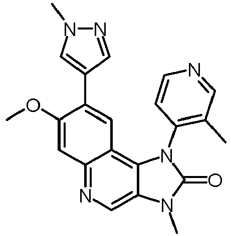 | 7-Methoxy-3-methyl-8-(1-methyl-1H-pyrazol-4-yl)-1-(3-methylpyridin-4-yl)-1,3-dihydroimidazo[4,5-c]quinolin-2-one |
| 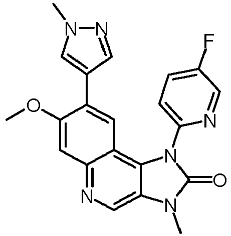 | 1-(5-Fluoropyridin-2-yl)-7-methoxy-3-methyl-8-(1-methyl-1H-pyrazol-4-yl)-1,3-dihydroimidazo[4,5-c]quinolin-2-one |
| 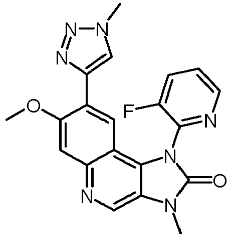 | 1-(3-Fluoropyridin-2-yl)-7-methoxy-3-methyl-8-(1-methyl-1H-[1,2,3]triazol-4-yl)-1,3-dihydroimidazo[4,5-c]quinolin-2-one |
| 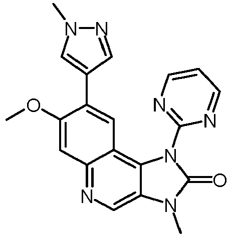 | 7-Methoxy-3-methyl-8-(1-methyl-1H-pyrazol-4-yl)-1-pyrimidin-2-yl-1,3-dihydro-imidazo[4,5-c]quinolin-2-one |
| 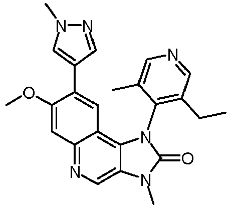 | 1-(3-Ethyl-5-methylpyridin-4-yl)-7-methoxy-3-methyl-8-(1-methyl-1H-pyrazol-4-yl)-1,3-dihydroimidazo[4,5-c]quinolin-2-one |
| 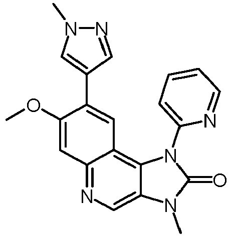 | 7-Methoxy-3-methyl-8-(1-methyl-1H-pyrazol-4-yl)-1-pyridin-2-yl-1,3-dihydro-imidazo[4,5-c]quinolin-2-one |
| 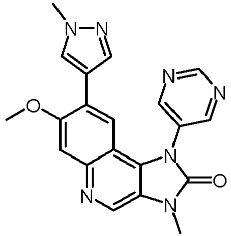 | 7-Methoxy-3-methyl-8-(1-methyl-1H-pyrazol-4-yl)-1-pyrimidin-5-yl-1,3-dihydro-imidazo[4,5-c]quinolin-2-one |
| 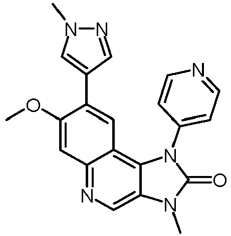 | 7-Methoxy-3-methyl-8-(1-methyl-1H-pyrazol-4-yl)-1-pyridin-4-yl-1,3-dihydro-imidazo[4,5-c]quinolin-2-one |
| 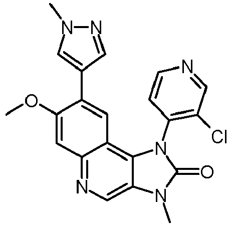 | 1-(3-Chloropyridin-4-yl)-7-methoxy-3-methyl-8-(1-methyl-1H-pyrazol-4-yl)-1,3-dihydroimidazo[4,5-c]quinolin-2-one |
| 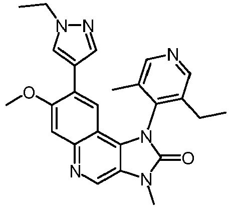 | 1-(3-Ethyl-5-methylpyridin-4-yl)-8-(1-ethyl-1H-pyrazol-4-yl)-7-methoxy-3-methyl-1,3-dihydroimidazo[4,5-c]quinolin-2-one |
| 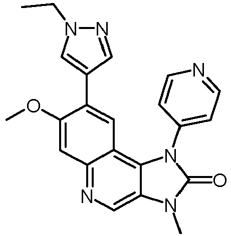 | 8-(1-Ethyl-1H-pyrazol-4-yl)-7-methoxy-3-methyl-1-pyridin-4-yl-1,3-dihydroimidazo-[4,5-c]quinolin-2-one |
| 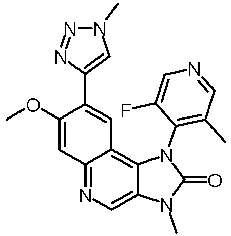 | 1-(3-Fluoro-5-methylpyridin-4-yl)-7-methoxy-3-methyl-8-(1-methyl-1H-[1,2,3]-triazol-4-yl)-1,3-dihydroimidazo[4,5-c]-quinolin-2-one |
| 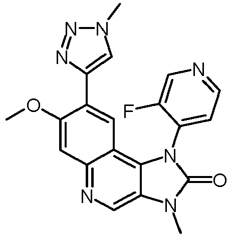 | 1-(3-Fluoropyridin-4-yl)-7-methoxy-3-methyl-8-(1-methyl-1H-[1,2,3]triazol-4-yl)-1,3-dihydroimidazo[4,5-c]quinolin-2-one |
| 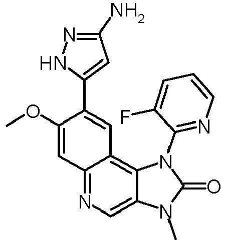 | 3-(3-Amino-1H-pyrazol-5-yl)-1-(3-fluoro-2-pyridyl)-7-methoxy-3-methyl-imidazo[4,5-c]-quinolin-2-one |
| 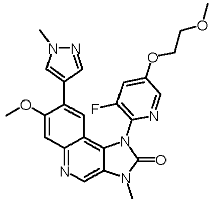 | 1-[3-Fluoro-5-(2-methoxyethoxy)-2-pyridyl]-7-methoxy-3-methyl-8-(1-methylpyrazol-4-yl)imidazo[4,5-c]quinolin-2-one |
| 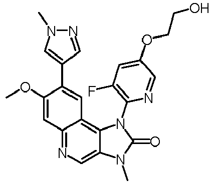 | 1-[3-Fluoro-5-(2-hydroxyethoxy)-2-pyridyl]-7-methoxy-3-methyl-8-(1-methylpyrazol-4-yl)imidazo[4,5-c]quinolin-2-one |
| 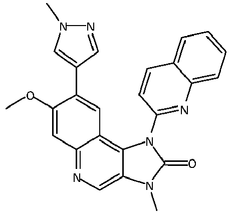 | 7-Methoxy-3-methyl-8-(1-methyl-1H-pyrazol-4-yl)-1-quinolin-2-yl-1,3-dihydro-midazo[4,5-c]quinolin-2-one |
| 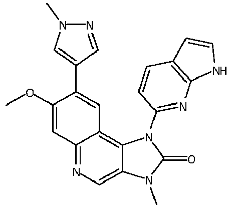 | 7-Methoxy-3-methyl-8-(1-methyl-1H-pyrazol-4-yl)-1-(1H-pyrrolo[2,3-b]pyridin-6-yl)-1,3-dihydroimidazo[4,5-c]quinolin-2-one |
| 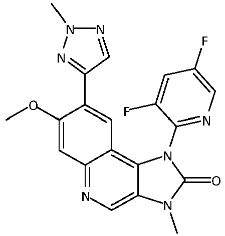 | 1-(3,5-Difluoropyridin-2-yl)-7-methoxy-3-methyl-8-(2-methyl-2H-[1,2,3]triazol-4-yl)-1,3-dihydroimidazo[4,5-c]quinolin-2-one |
| 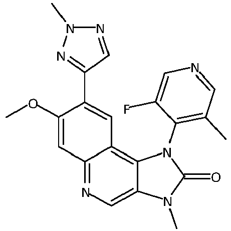 | 1-(3-Fluoro-5-methylpyridin-4-yl)-7-methoxy-3-methyl-8-(2-methyl-2H-[1,2,3]-triazol-4-yl)-1,3-dihydroimidazo[4,5-c]-quinolin-2-one |
| 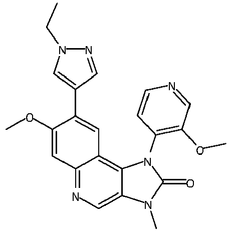 | 8-(1-Ethyl-1H-pyrazol-4-yl)-7-methoxy-1-(3-methoxypyridin-4-yl)-3-methyl-1,3-dihydro-imidazo[4,5-c]quinolin-2-one |
| 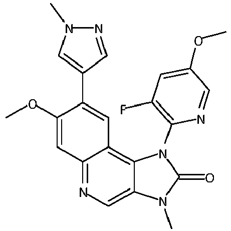 | 1-(3-Fluoro-5-methoxypyridin-2-yl)-7-methoxy-3-methyl-8-(1-methyl-1H-pyrazol-4-yl)-1,3-dihydroimidazo[4,5-c]quinolin-2-one |
| 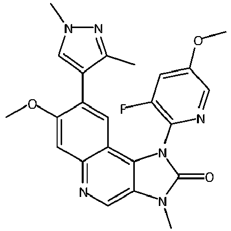 | 8-(1,3-Dimethyl-1H-pyrazol-4-yl)-1-(3-fluoro-5-methoxypyridin-2-yl)-7-methoxy-3-methyl-1,3-dihydroimidazo[4,5-c]quinolin-2-one |
| 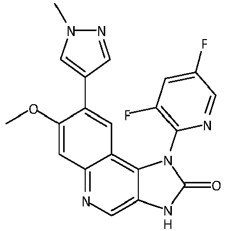 | 1-(3,5-Difluoropyridin-2-yl)-7-methoxy-8-(1-methyl-1H-pyrazol-4-yl)-1,3-dihydroimidazo-[4,5-c]quinolin-2-one |
| 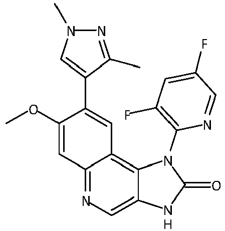 | 1-(3,5-Difluoropyridin-2-yl)-8-(1,3-dimethyl-1H-pyrazol-4-yl)-7-methoxy-1,3-dihydro-imidazo[4,5-c]quinolin-2-one |
| 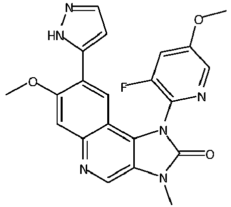 | 1-(3-Fluoro-5-methoxypyridin-2-yl)-7-methoxy-3-methyl-8-(2H-pyrazol-3-yl)-1 ,3-dihydroimidazo[4,5-c]quinolin-2-one |
| 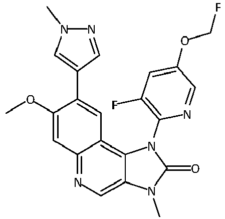 | 1-(3-Fluoro-5-fluoromethoxypyridin-2-yl)-7-methoxy-3-methyl-8-(1-methyl-1H-pyrazol-4-yl)-1,3-dihydroimidazo[4,5-c]quinolin-2-one |
| 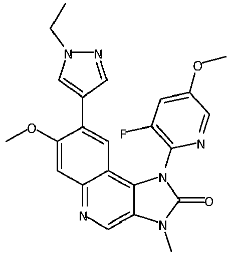 | 8-(1-Ethyl-1H-pyrazol-4-yl)-1-(3-fluoro-5-methoxypyridin-2-yl)-7-methoxy-3-methyl-1,3-dihydroimidazo[4,5-c]quinolin-2-one |
| 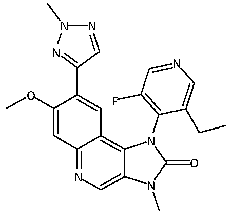 | 1-(3-Ethyl-5-fluoropyridin-4-yl)-7-methoxy-3-methyl-8-(2-methyl-2H-[1,2,3]triazol-4-yl)-1,3-dihydroimidazo[4,5-c]quinolin-2-one |
| 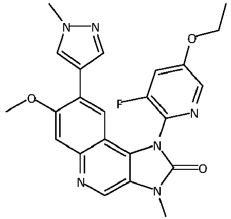 | 1-(5-Ethoxy-3-fluoropyridin-2-yl)-7-methoxy-3-methyl-8-(1-methyl-1H-pyrazol-4-yl)-1,3-dihydroimidazo[4,5-c]quinolin-2-one |
| 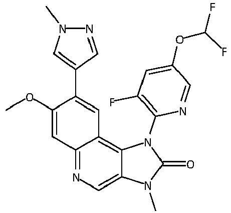 | 1-(5-Difluoromethoxy-3-fluoropyridin-2-yl)-7-methoxy-3-methyl-8-(1-methyl-1H-pyrazol-4-yl)-1,3-dihydroimidazo[4,5-c]quinolin-2-one |
| 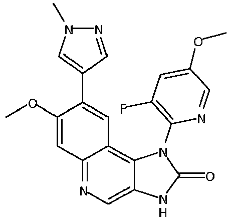 | 1-(3-Fluoro-5-methoxypyridin-2-yl)-7-methoxy-8-(1-methyl-1H-pyrazol-4-yl)-1,3-dihydroimidazo[4,5-c]quinolin-2-one |
| 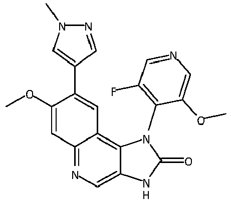 | 1-(3-Fluoro-5-methoxypyridin-4-yl)-7-methoxy-8-(1-methyl-1H-pyrazol-4-yl)-1,3-dihydroimidazo[4,5-c]quinolin-2-one |
| 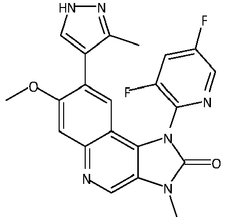 | 1-(3,5-Difluoropyridin-2-yl)-7-methoxy-3-methyl-8-(3-methyl-1H-pyrazol-4-yl)-1,3-dihydroimidazo[4,5-c]quinolin-2-one |
| 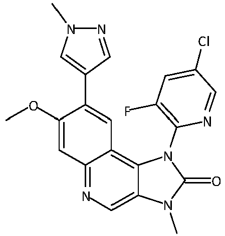 | 1-(5-Chloro-3-fluoropyridin-2-yl)-7-methoxy-3-methyl-8-(1-methyl-1H-pyrazol-4-yl)-1,3-dihydroimidazo[4,5-c]quinolin-2-one |
| 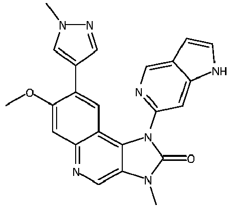 | 7-Methoxy-3-methyl-8-(1-methyl-1H-pyrazol-4-yl)-1-(1H-pyrrolo[3,2-c]pyridin-6-yl)-1,3-dihydroimidazo[4,5-c]quinolin-2-one |
| 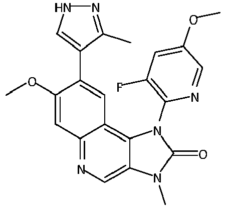 | 1-(3-Fluoro-5-methoxypyridin-2-yl)-7-methoxy-3-methyl-8-(3-methyl-1H-pyrazol-4-yl)-1,3-dihydroimidazo[4,5-c]quinolin-2-one |
| 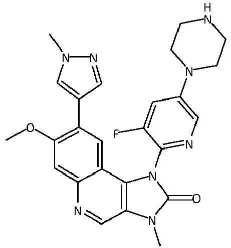 | 1-(3-Fluoro-5-piperazin-1-yl-pyridin-2-yl)-7-methoxy-3-methyl-8-(1-methyl-1H-pyrazol-4-yl)-1,3-dihydroimidazo[4,5-c]quinolin-2-one |
| 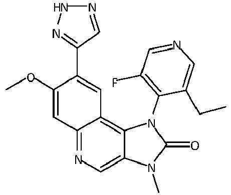 | 1-(3-Ethyl-5-fluoropyridin-4-yl)-7-methoxy-3-methyl-8-(2H-[1,2,3]triazol-4-yl)-1,3-dihydro-imidazo[4,5-c]quinolin-2-one |
| 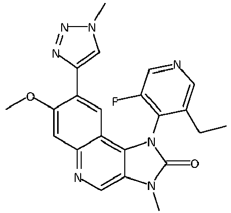 | 1-(3-Ethyl-5-fluoropyridin-4-yl)-7-methoxy-3-methyl-8-(1-methyl-1H-[1,2,3]triazol-4-yl)-1,3-dihydroimidazo[4,5-c]quinolin-2-one |
| 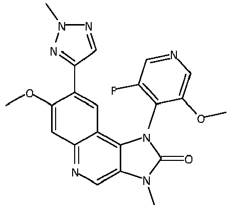 | 1-(3-Fluoro-5-methoxypyridin-4-yl)-7-methoxy-3-methyl-8-(2-methyl-2H-[1,2,3]-triazol-4-yl)-1,3-dihydroimidazo[4,5-c]-quinolin-2-one |
| 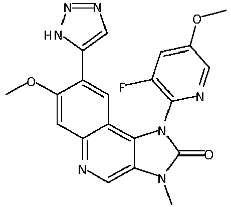 | 1-(3-Fluoro-5-methoxypyridin-2-yl)-7-methoxy-3-methyl-8-(3H-[1,2,3]triazol-4-yl)-1,3-dihydroimidazo[4,5-c]quinolin-2-one |
| 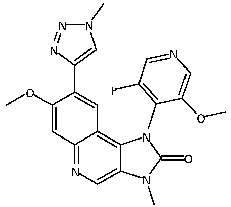 | 1-(3-Fluoro-5-methoxypyridin-4-yl)-7-ethoxy-3-methyl-8-(1-methyl-1H-[1,2,3]-triazol-4-yl)-1,3-dihydroimidazo[4,5-c]-quinolin-2-one |
| 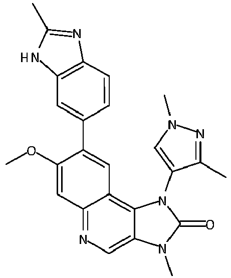 | 1-(1,3-Dimethyl-1H-pyrazol-4-yl)-7-methoxy-3-methyl-8-(2-methyl-3H-benzoimidazol-5-yl)-1,3-dihydroimidazo[4,5-c]quinolin-2-one |
| 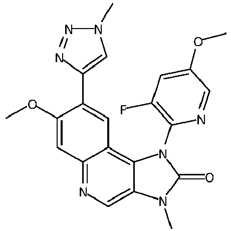 | 1-(3-Fluoro-5-methoxypyridin-2-yl)-7-methoxy-3-methyl-8-(1-methyl-1H-[1,2,3]-triazol-4-yl)-1,3-dihydroimidazo[4,5-c]-quinolin-2-one |
| 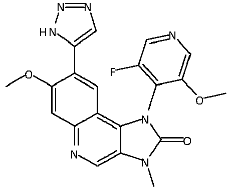 | 1-(3-Fluoro-5-methoxypyridin-4-yl)-7-methoxy-3-methyl-8-(3H-[1,2,3]triazol-4-yl)-1,3-dihydroimidazo[4,5-c]quinolin-2-one |
| 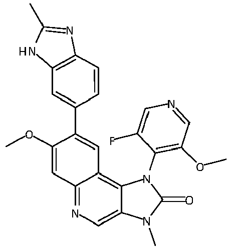 | 1-(3-Fluoro-5-methoxypyridin-4-yl)-7-methoxy-3-methyl-8-(2-methyl-3H-benzo-imidazol-5-yl)-1,3-dihydroimidazo[4,5-c]-quinolin-2-one |
| 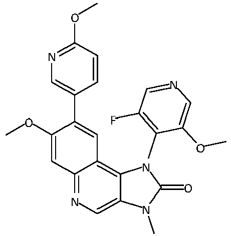 | 1-(3-Fluoro-5-methoxypyridin-4-yl)-7-methoxy-8-(6-methoxypyridin-3-yl)-3-methyl-1,3-dihydroimidazo[4,5-c]quinolin-2-one |
| 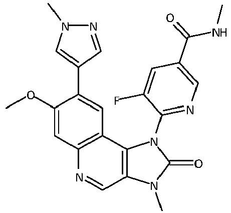 | 5-Fluoro-6-[7-methoxy-3-methyl-8-(1-methyl-1H-pyrazol-4-yl)-2-oxo-2,3-dihydro-imidazo[4,5-c]quinolin-1-yl]-N-methyl-nicotinamide |
| 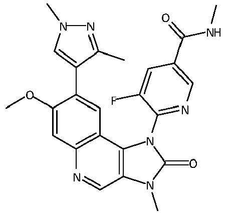 | 6-[8-(1,3-Dimethyl-1H-pyrazol-4-yl)-7-methoxy-3-methyl-2-oxo-2,3-dihydro-imidazo[4,5-c]quinolin-1-yl]-5-fluoro-N-methylnicotinamide |
| 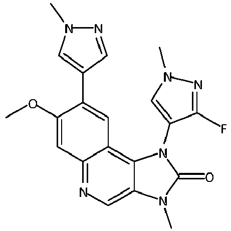 | 1-(3-Fluoro-1-methyl-1H-pyrazol-4-yl)-7-ethoxy-3-methyl-8-(1-methyl- 1H-pyrazol-4-yl)-1,3-dihydroimidazo[4,5-c]quinolin-2-one |
| 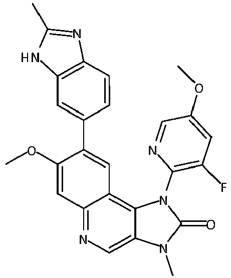 | 1-(3-Fluoro-5-methoxypyridin-2-yl)-7-methoxy-3-methyl-8-(2-methyl-3H-benzo-imidazol-5-yl)-1,3-dihydroimidazo[4,5-c]-quinolin-2-one |
| 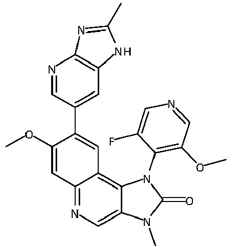 | 1-(3-Fluoro-5-methoxypyridin-4-yl)-7-methoxy-3-methyl-8-(2-methyl-1H-imidazo-[4,5-b]pyridin-6-yl)-1,3-dihydroimidazo-[4,5-c]quinolin-2-one |
| 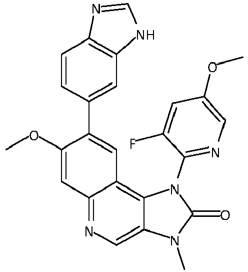 | 8-(3H-Benzimidazol-5-yl)-1-(3-fluoro-5-methoxypyridin-2-yl)-7-methoxy-3-methyl-1,3-dihydroimidazo[4,5-c]quinolin-2-one |
| 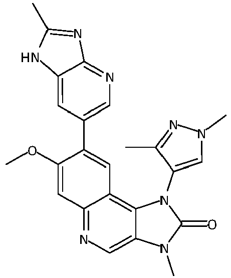 | 1-(1,3-Dimethyl-1H-pyrazol-4-yl)-7-methoxy-3-methyl-8-(2-methyl-1H-imidazo[4,5-b]-pyridin-6-yl)-1,3-dihydroimidazo[4,5-c]-quinolin-2-one |
| 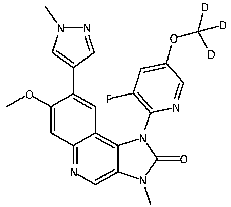 | 1-[3-Fluoro-5-(trideuteriomethoxy)-2-pyridyl]-7-methoxy-3-methyl-8-(1-methyl-pyrazol-4-yl)imidazo[4,5-c]quinolin-2-one |
| 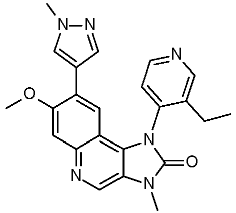 | 1-(3-Ethylpyridin-4-yl)-7-methoxy-3-methyl-8-(1-methyl-1H-pyrazol-4-yl)-1,3-dihydro-imidazo[4,5-c]quinolin-2-one |
| 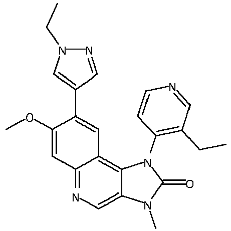 | 8-(1-Ethyl-1H-pyrazol-4-yl)-1-(3-ethyl-pyridin-4-yl)-7-methoxy-3-methyl-1,3-dihydroimidazo[4,5-c]quinolin-2-one |
| 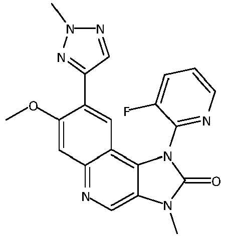 | 1-(3-Fluoropyridin-2-yl)-7-methoxy-3-methyl-8-(2-methyl-2H-[1,2,3]triazol-4-yl)-1,3-dihydroimidazo[4,5-c]quinolin-2-one |
| 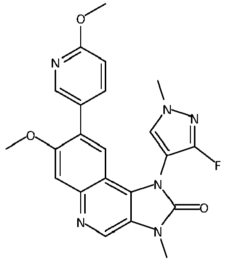 | 1-(3-Fluoro-1-methyl-1H-pyrazol-4-yl)-7-methoxy-8-(6-methoxypyridin-3-yl)-3-methyl-1,3-dihydroimidazo[4,5-c]quinolin-2-one |
| 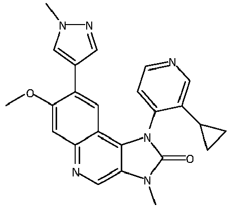 | 1-(3-Cyclopropylpyridin-4-yl)-7-methoxy-3-methyl-8-(1-methyl-1H-pyrazol-4-yl)-1,3-dihydroimidazo[4,5-c]quinolin-2-one |
| 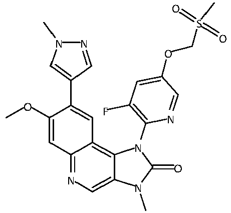 | 1-(3-Fluoro-5-methylsulfonylmethoxypyridin-2-yl)-7-methoxy-3-methyl-8-(1-methyl-1H-pyrazol-4-yl)-1,3-dihydroimidazo[4,5-c]-quinolin-2-one |
| 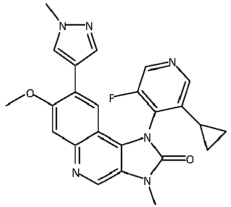 | 1-(3-Cyclopropyl-5-fluoropyridin-4-yl)-7-methoxy-3-methyl-8-(1-methyl-1H-pyrazol-4-yl)-1,3-dihydroimidazo[4,5-c]quinolin-2-one |
| 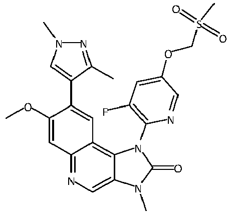 | 8-(1,3-Dimethyl-1H-pyrazol-4-yl)-1-(3-fluoro-5-methylsulfonylmethoxypyridin-2-yl)-7-methoxy-3-methyl-1,3-dihydroimidazo-[4,5-c]quinolin-2-one |
| 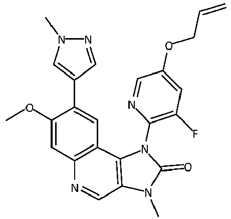 | 1-(5-Allyloxy-3-fluoropyridin-2-yl)-7-methoxy-3-methyl-8-(1-methyl-1H-pyrazol-4-yl)-1,3-dihydroimidazo[4,5-c]quinolin-2-one |
| 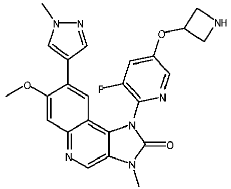 | 1-[5-(Azetidin-3-yloxy)-3-fluoropyridin-2-yl]-7-methoxy-3-methyl-8-(1-methyl-1H-pyrazol-4-yl)-1,3-dihydroimidazo[4,5-c]-quinolin-2-one |
| 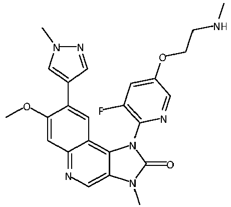 | 1-[3-Fluoro-5-(2-methylaminoethoxy)-pyridin-2-yl]-7-methoxy-3-methyl-8-(1-methyl-1H-pyrazol-4-yl)-1,3-dihydroimidazo-[4,5-c]quinolin-2-one |
| 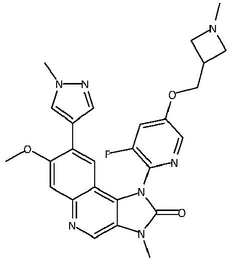 | 1-[3-Fluoro-5-(1-methylazetidin-3-yl-methoxy)pyridin-2-yl]-7-methoxy-3-methyl-8-(1-methyl-1H-pyrazol-4-yl)-1,3-dihydro-imidazo[4,5-c]quinolin-2-one |
| 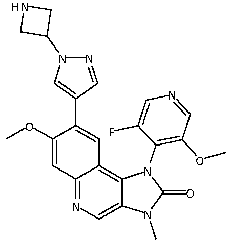 | 8-[1-(Azetidin-3-yl)-1H-pyrazol-4-yl]-1-(3-fluoro-5-methoxypyridin-4-yl)-7-methoxy-3-methyl-1H,2H,3H-imidazo[4,5-c]quinolin-2-one |
and/or pharmaceutically usable salt, solvate or tautomer thereof.

2. Compound according to Claim 1 and/or pharmaceutically usable salt, solvate or tautomer thereof for use as medicament.

3. Compound according to Claim 1 and/or pharmaceutically usable salt, solvate or tautomer thereof for use in the treatment of cancer, tumours and/or metastases.

4. Compound and/or pharmaceutically usable salt, solvate or tautomer thereof for use in the treatment of cancer, tumours and/or metastases according to Claim 3 in combination with radiotherapy.

5. Compound and/or pharmaceutically usable salt, solvate or tautomer thereof for use in the treatment of cancer, tumours and/or metastases according to Claim 3 in combination with at least one anticancer agent.

6. Compound and/or pharmaceutically usable salt, solvate or tautomer thereof for use according to one of Claims 3 to 5, where the tumour is selected from the group of diseases of squamous epithelium, bladder, stomach, kidneys, head, neck, oesophagus, cervix, thyroid, intestine, bone, liver, brain, prostate, urogenital tract, lymphatic system, larynx, lung, skin, blood and immune system, and/or the cancer is selected from the group of monocytic leukaemia, lung adenocarcinoma, small-cell lung carcinoma, pancreatic cancer, glioblastoma, intestinal carcinoma, breast carcinoma, acute myeloid leukaemia, chronic myeloid leukaemia, acute lymphatic leukaemia, chronic lymphatic leukaemia, Hodgkin's lymphoma and non-Hodgkin's lymphoma.

7. Compound according to Claim 1 and/or pharmaceutically usable salt, solvate or tautomer thereof for use in the sensitisation of cancer cells to an anticancer agent and/or ionising radiation.

8. Pharmaceutical composition comprising an effective amount of at least one compound according to Claim 1 and/or a pharmaceutically usable salt, solvate or tautomer thereof and at least one pharmaceutically tolerated assistant.

9. Pharmaceutical composition according to Claim 8, furthermore comprising at least one further medicament active compound.

10. Pharmaceutical composition according to Claim 9, where the further medicament active compound is an anticancer agent.

11. Kit consisting of separate packs of
(a) an effective amount of a compound according to Claim 1 and/or pharmaceutically usable salt, solvate or tautomer thereof, and
(b) an effective amount of a further medicament active compound.

12. Kit according to Claim 11, where the further medicament active compound is an anticancer agent.

## Revendications

1. Composé choisi parmi :
| | |
|---|---|
| 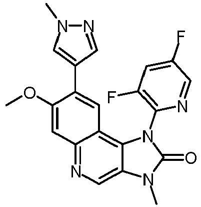 | 1-(3,5-Difluoropyridin-2-yl)-7-méthoxy-3-méthyl-8-(1-méthyl-1H-pyrazol-4-yl)-1,3-dihydroimidazo[4,5-c]quinoléin-2-one |
| 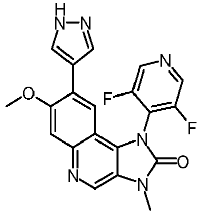 | 1-(3,5-Difluoropyridin-4-yl)-7-méthoxy-3-méthyl-8-(1H-pyrazol-4-yl)-1,3-dihydro-imidazo[4,5-c]quinoléin-2-one |
| 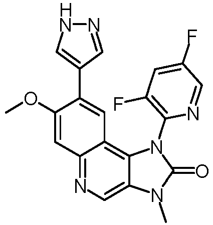 | 1-(3,5-Difluoropyridin-2-yl)-7-méthoxy-3-méthyl-8-(1H-pyrazol-4-yl)-1,3-dihydro-imidazo[4,5-c]quinoléin-2-one |
| 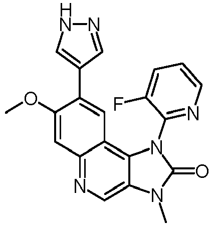 | 1-(3-Fluoropyridin-2-yl)-7-méthoxy-3-méthyl-8-(1H-pyrazol-4-yl)-1,3-dihydro-imidazo[4,5-c]quinoléin-2-one |
| 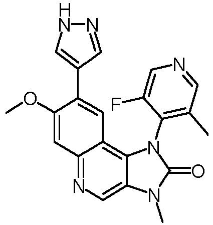 | 1-(3-Fluoro-5-méthylpyridin-4-yl)-7-méthoxy-3-méthyl-8-(1H-pyrazol-4-yl)-1,3-dihydroimidazo[4,5-c]quinoléin-2-one |
| 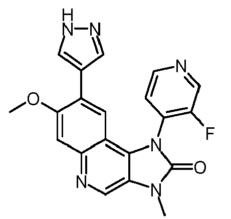 | 1-(3-Fluoropyridin-4-yl)-7-méthoxy-3-méthyl-8-(1H-pyrazol-4-yl)-1,3-dihydro-imidazo[4,5-c]quinoléin-2-one |
| 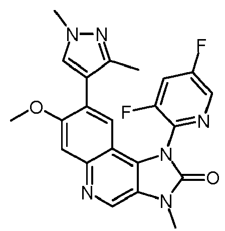 | 1-(3,5-Difluoropyridin-2-yl)-8-(1,3-diméthyl-1H-pyrazol-4-yl)-7-méthoxy-3-méthyl-1,3-dihydroimidazo[4,5-c]quinoléin-2-one |
| 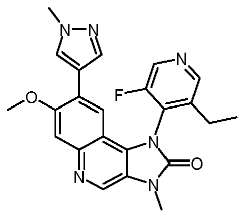 | 1-(3-Éthyl-5-fluoropyridin-4-yl)-7-méthoxy-3-méthyl-8-(1-méthyl-1H-pyrazol-4-yl)-1,3-dihydroimidazo[4,5-c]quinoléin-2-one |
| 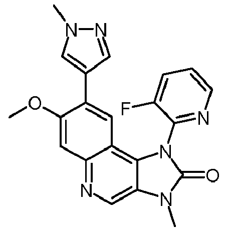 | 1-(3-Fluoropyridin-2-yl)-7-méthoxy-3-méthyl-8-(1-méthyl-1H-pyrazol-4-yl)-1,3-dihydroimidazo[4,5-c]quinoléin-2-one |
| 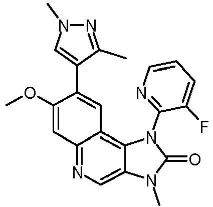 | 8-(1,3-Diméthyl-1H-pyrazol-4-yl)-1-(3-fluoropyridin-2-yl)-7-méthoxy-3-méthyl-1,3-dihydroimidazo[4,5-c]quinoléin-2-one |
| 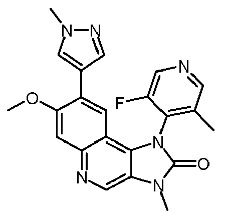 | 1-(3-Fluoro-5-méthylpyridin-4-yl)-7-méthoxy-3-méthyl-8-(1-méthyl-1H-pyrazol-4-yl)-1,3-dihydroimidazo[4,5-c]quinoléin-2-one |
| 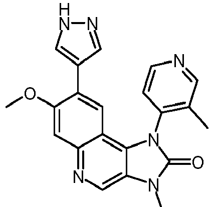 | 7-Méthoxy-3-méthyl-1-(3-méthylpyridin-4-yl)-8-(1H-pyrazol-4-yl)-1,3-dihydro-imidazo[4,5-c]quinoléin-2-one |
| 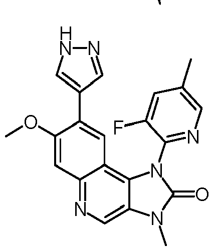 | 1-(3-Fluoro-5-méthylpyridin-2-yl)-7-méthoxy-3-méthyl-8-(1H-pyrazol-4-yl)-1,3-dihydroimidazo[4,5-c]quinoléin-2-one |
| 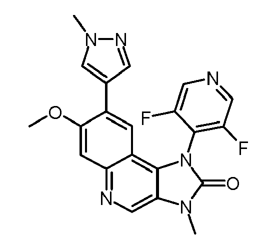 | 1-(3,5-Difluoropyridin-4-yl)-7-méthoxy-3-méthyl-8-(1-méthyl-1H-pyrazol-4-yl)-1,3-dihydroimidazo[4,5-c]quinoléin-2-one |
| 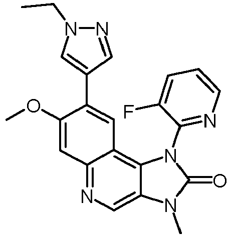 | 8-(1-Éthyl-1H-pyrazol-4-yl)-1-(3-fluoro-pyridin-2-yl)-7-méthoxy-3-méthyl-1,3-dihydroimidazo[4,5-c]quinoléin-2-one |
| 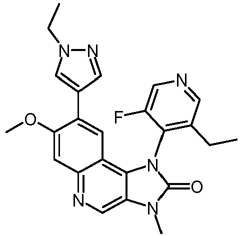 | 1-(3-Éthyl-5-fluoropyridin-4-yl)-8-(1-éthyl-1H-pyrazol-4-yl)-7-méthoxy-3-méthyl-1,3-dihydroimidazo[4,5-c]quinoléin-2-one |
| 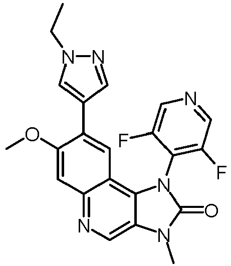 | 1-(3,5-Difluoropyridin-4-yl)-8-(1-éthyl-1H-pyrazol-4-yl)-7-méthoxy-3-méthyl-1,3-dihydroimidazo[4,5-c]quinoléin-2-one |
| 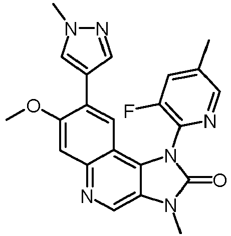 | 1-(3-Fluoro-5-méthylpyridin-2-yl)-7-méthoxy-3-méthyl-8-(1-méthyl-1H-pyrazol-4-yl)-1,3-dihydroimidazo[4,5-c]quinoléin-2-one |
| 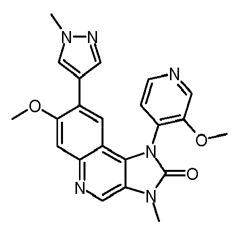 | 7-Méthoxy-1-(3-méthoxypyridin-4-yl)-3-méthyl-8-(1-méthyl-1H-pyrazol-4-yl)-1,3-dihydroimidazo[4,5-c]quinoléin-2-one |
| 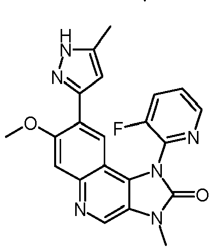 | 1-(3-Fluoropyridin-2-yl)-7-méthoxy-3-méthyl-8-(5-méthyl-1H-pyrazol-3-yl)-1,3-dihydroimidazo[4,5-c]quinoléin-2-one |
| 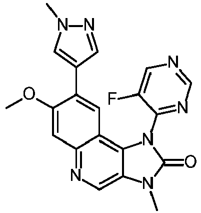 | 1-(5-Fluoropyrimidin-4-yl)-7-méthoxy-3-méthyl-8-(1-méthyl-1H-pyrazol-4-yl)-1H,2H,3H-imidazo[4,5-c]quinoléin-2-one |
| 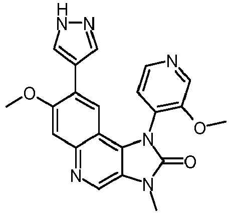 | 7-Méthoxy-1-(3-méthoxypyridin-4-yl)-3-méthyl-8-(1H-pyrazol-4-yl)-1,3-dihydro-imidazo[4,5-c]quinoléin-2-one |
| 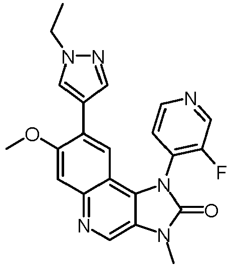 | 8-(1-Éthyl-1H-pyrazol-4-yl)-1-(3-fluoro-pyridin-4-yl)-7-méthoxy-3-méthyl-1,3-dihydroimidazo[4,5-c]quinoléin-2-one |
| 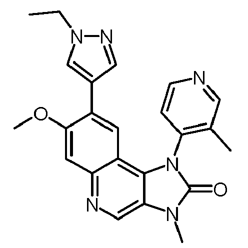 | 8-(1-Éthyl-1H-pyrazol-4-yl)-7-méthoxy-3-méthyl-1-(3-méthylpyridin-4-yl)-1,3-dihydroimidazo[4,5-c]quinoléin-2-one |
| 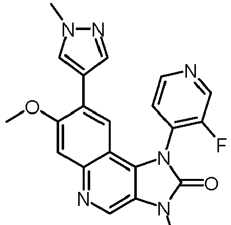 | 1-(3-Fluoropyridin-4-yl)-7-méthoxy-3-méthyl-8-(1-méthyl-1H-pyrazol-4-yl)-1,3-dihydroimidazo[4,5-c]quinoléin-2-one |
| 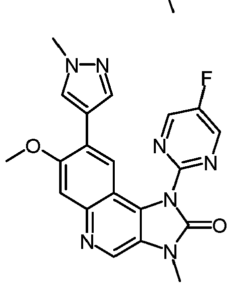 | 1-(5-Fluoropyrimidin-2-yl)-7-méthoxy-3-méthyl-8-(1-méthyl-1H-pyrazol-4-yl)-1,3-dihydroimidazo[4,5-c]quinoléin-2-one |
| 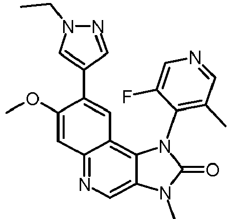 | 8-(1-Éthyl-1H-pyrazol-4-yl)-1-(3-fluoro-5-méthylpyridin-4-yl)-7-méthoxy-3-méthyl-1 ,3-dihydroimidazo[4,5-c]quinoléin-2-one |
| 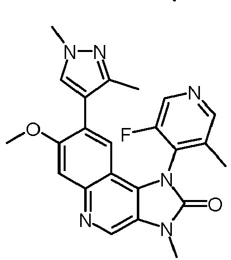 | 8-(1,3-Diméthyl-1H-pyrazol-4-yl)-1-(3-fluoro-5-méthylpyridin-4-yl)-7-méthoxy-3-méthyl-1,3-dihydroimidazo[4,5-c]quinoléin-2-one |
| 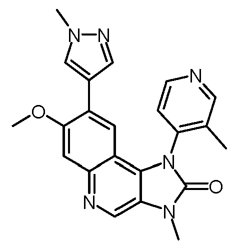 | 7-Méthoxy-3-méthyl-8-(1-méthyl-1H-pyrazol-4-yl)-1-(3-méthylpyridin-4-yl)-1,3-dihydroimidazo[4,5-c]quinoléin-2-one |
| 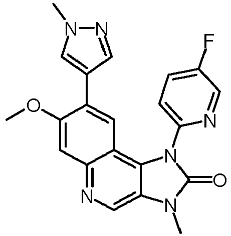 | 1-(5-Fluoropyridin-2-yl)-7-méthoxy-3-méthyl-8-(1-méthyl-1H-pyrazol-4-yl)-1,3-dihydroimidazo[4,5-c]quinoléin-2-one |
| 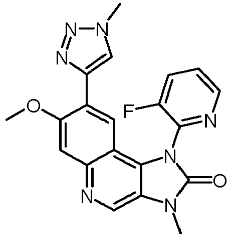 | 1-(3-Fluoropyridin-2-yl)-7-méthoxy-3-méthyl-8-(1-méthyl-1H-[1,2,3]triazol-4-yl)-1,3-dihydroimidazo[4,5-c]quinoléin-2-one |
| 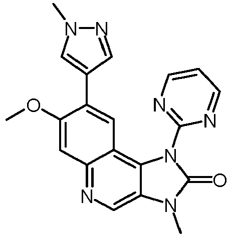 | 7-Méthoxy-3-méthyl-8-(1-méthyl-1H-pyrazol-4-yl)-1-pyrimidin-2-yl-1,3-dihydro-imidazo[4,5-c]quinoléin-2-one |
| 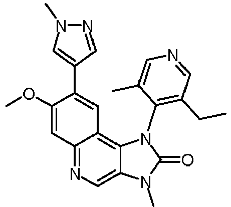 | 1-(3-Éthyl-5-méthylpyridin-4-yl)-7-méthoxy-3-méthyl-8-(1-méthyl-1H-pyrazol-4-yl)-1,3-dihydroimidazo[4,5-c]quinoléin-2-one |
| 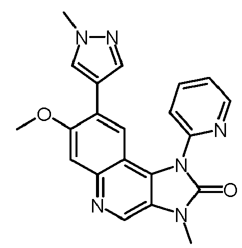 | 7-Méthoxy-3-méthyl-8-(1-méthyl-1H-pyrazol-4-yl)-1-pyridin-2-yl-1,3-dihydro-imidazo[4,5-c]quinoléin-2-one |
| 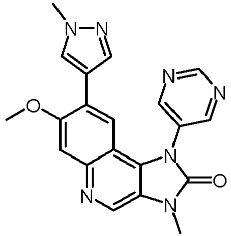 | 7-Méthoxy-3-méthyl-8-(1-méthyl-1H-pyrazol-4-yl)-1-pyrimidin-5-yl-1,3-dihydro-imidazo[4,5-c]quinoléin-2-one |
| 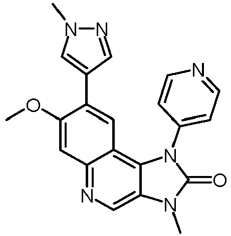 | 7-Méthoxy-3-méthyl-8-(1-méthyl-1H-pyrazol-4-yl)-1-pyridin-4-yl-1,3-dihydro-imidazo[4,5-c]quinoléin-2-one |
| 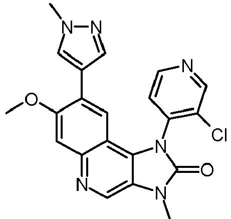 | 1-(3-Chloropyridin-4-yl)-7-méthoxy-3-méthyl-8-(1-méthyl-1H-pyrazol-4-yl)-1,3-dihydroimidazo[4,5-c]quinoléin-2-one |
| 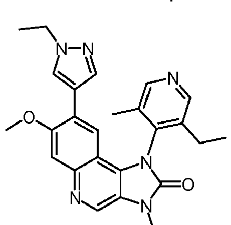 | 1-(3-Éthyl-5-méthylpyridin-4-yl)-8-(1-éthyl-1H-pyrazol-4-yl)-7-méthoxy-3-méthyl-1,3-dihydroimidazo[4,5-c]quinoléin-2-one |
| 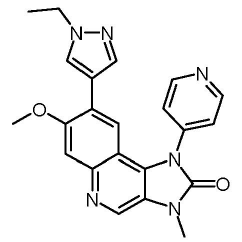 | 8-(1-Éthyl-1H-pyrazol-4-yl)-7-méthoxy-3-méthyl-1-pyridin-4-yl-1,3-dihydro-imidazo[4,5-c]quinoléin-2-one |
| 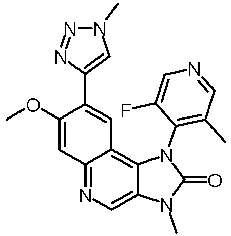 | 1-(3-Fluoro-5-méthylpyridin-4-yl)-7-méthoxy-3-méthyl-8-(1-méthyl-1H-[1,2,3]triazol-4-yl)-1,3-dihydroimidazo-[4,5-c]quinoléin-2-one |
| 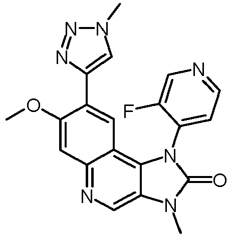 | 1-(3-Fluoropyridin-4-yl)-7-méthoxy-3-méthyl-8-(1-méthyl-1H-[1,2,3]triazol-4-yl)-1,3-dihydroimidazo[4,5-c]quinoléin-2-one |
| 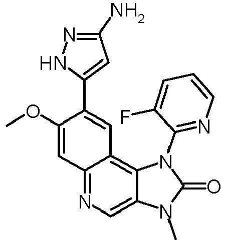 | 8-(3-Amino-1H-pyrazol-5-yl)-1-(3-fluoro-2-pyridyl)-7-méthoxy-3-méthylimidazo-[4,5-c]quinoléin-2-one |
| 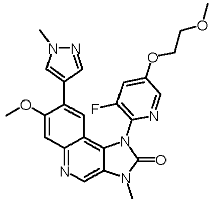 | 1-[3-Fluoro-5-(2-méthoxyéthoxy)-2-pyridyl]-7-méthoxy-3-méthyl-8-(1-méthylpyrazol-4-yl)imidazo[4,5-c]quinoléin-2-one |
| 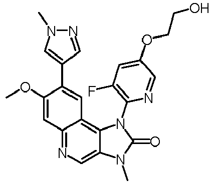 | 1-[3-Fluoro-5-(2-hydroxyéthoxy)-2-pyridyl]-7-méthoxy-3-méthyl-8-(1-méthylpyrazol-4-yl)imidazo[4,5-c]quinoléin-2-one |
| 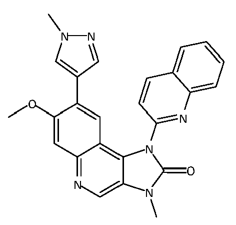 | 7-Méthoxy-3-méthyl-8-(1-méthyl-1H-pyrazol-4-yl)-1-quinoléin-2-yl-1,3-dihydro-imidazo[4,5-c]quinoléin-2-one |
| 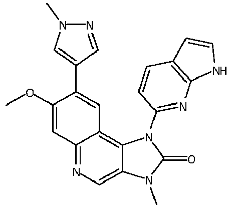 | 7-Méthoxy-3-méthyl-8-(1-méthyl-1H-pyrazol-4-yl)-1-(1H-pyrrolo[2,3-b]pyridin-6-yl)-1,3-dihydroimidazo[4,5-c]quinoléin-2-one |
| 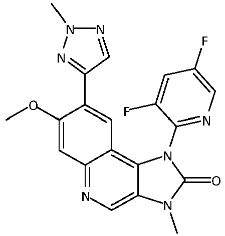 | 1-(3,5-Difluoropyridin-2-yl)-7-méthoxy-3-méthyl-8-(2-méthyl-2H-[1,2,3]triazol-4-yl)-1,3-dihydroimidazo[4,5-c]quinoléin-2-one |
| 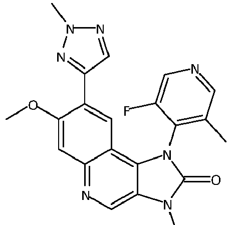 | 1-(3-Fluoro-5-méthylpyridin-4-yl)-7-méthoxy-3-méthyl-8-(2-méthyl-2H-[1,2,3]triazol-4-yl)-1,3-dihydroimidazo-[4,5-c]quinoléin-2-one |
| 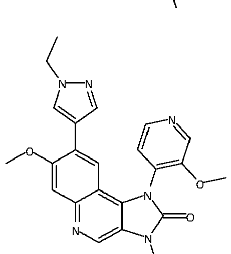 | 8-(1-Éthyl-1H-pyrazol-4-yl)-7-méthoxy-1-(3-méthoxypyridin-4-yl)-3-méthyl-1,3-dihydro-imidazo[4,5-c]quinoléin-2-one |
| 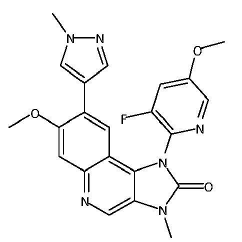 | 1-(3-Fluoro-5-méthoxypyridin-2-yl)-7-méthoxy-3-méthyl-8-(1-méthyl-1H-pyrazol-4-yl)-1,3-dihydroimidazo[4,5-c]quinoléin-2-one |
| 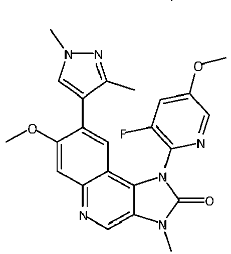 | 8-(1,3-Diméthyl-1H-pyrazol-4-yl)-1-(3-fluoro-5-méthoxypyridin-2-yl)-7-méthoxy-3-méthyl-1,3-dihydroimidazo[4,5-c]quinoléin-2-one |
| 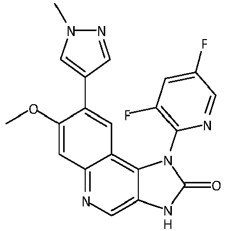 | 1-(3,5-Difluoropyridin-2-yl)-7-méthoxy-8-(1-méthyl-1H-pyrazol-4-yl)-1,3-dihydro-imidazo[4,5-c]quinoléin-2-one |
| 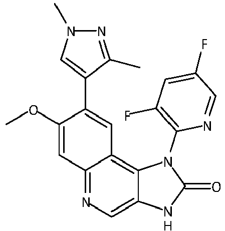 | 1-(3,5-Difluoropyridin-2-yl)-8-(1,3-diméthyl-1H-pyrazol-4-yl)-7-méthoxy-1,3-dihydro-imidazo[4,5-c]quinoléin-2-one |
| 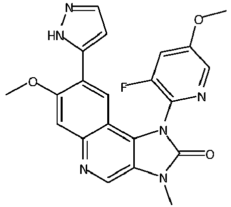 | 1-(3-Fluoro-5-méthoxypyridin-2-yl)-7-méthoxy-3-méthyl-8-(2H-pyrazol-3-yl)-1,3-dihydroimidazo[4,5-c]quinoléin-2-one |
| 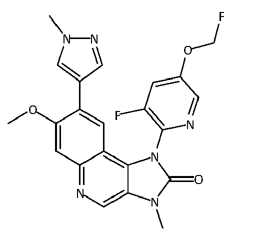 | 1-(3-Fluoro-5-fluorométhoxypyridin-2-yl)-7-méthoxy-3-méthyl-8-(1-méthyl-1H-pyrazol-4-yl)-1,3-dihydroimidazo[4,5-c]quinoléin-2-one |
| 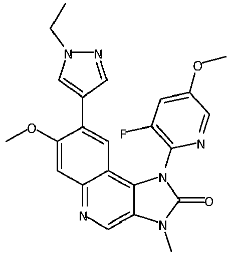 | 8-(1-Éthyl-1H-pyrazol-4-yl)-1-(3-fluoro-5-méthoxypyridin-2-yl)-7-méthoxy-3-méthyl-1,3-dihydroimidazo[4,5-c]quinoléin-2-one |
| 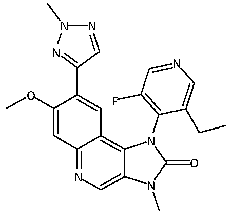 | 1-(3-Éthyl-5-fluoropyridin-4-yl)-7-méthoxy-3-méthyl-8-(2-méthyl-2H-[1,2,3]triazol-4-yl)-1,3-dihydroimidazo[4,5-c]quinoléin-2-one |
| 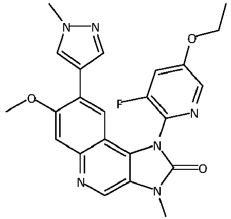 | 1-(5-Éthoxy-3-fluoropyridin-2-yl)-7-méthoxy-3-méthyl-8-(1-méthyl-1H-pyrazol-4-yl)-1,3-dihydroimidazo[4,5-c]quinoléin-2-one |
| 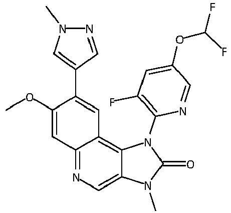 | 1-(5-Difluorométhoxy-3-fluoropyridin-2-yl)-7-méthoxy-3-méthyl-8-(1-méthyl-1H-pyrazol-4-yl)-1,3-dihydroimidazo[4,5-c]quinoléin-2-one |
| 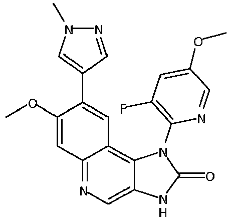 | 1-(3-Fluoro-5-méthoxypyridin-2-yl)-7-méthoxy-8-(1-méthyl-1H-pyrazol-4-yl)-1,3-dihydroimidazo[4,5-c]quinoléin-2-one |
| 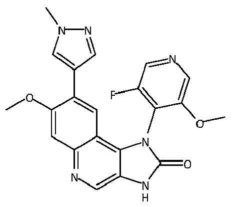 | 1-(3-Fluoro-5-méthoxypyridin-4-yl)-7-méthoxy-8-(1-méthyl-1H-pyrazol-4-yl)-1,3-dihydroimidazo[4,5-c]quinoléin-2-one |
| 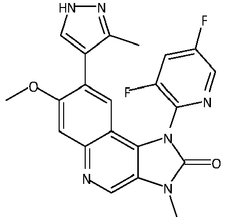 | 1-(3,5-Difluoropyridin-2-yl)-7-méthoxy-3-méthyl-8-(3-méthyl-1H-pyrazol-4-yl)-1,3-dihydroimidazo[4,5-c]quinoléin-2-one |
| 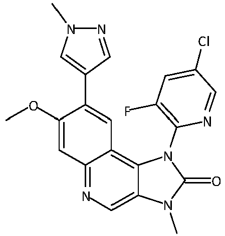 | 1-(5-Chloro-3-fluoropyridin-2-yl)-7-méthoxy-3-méthyl-8-(1-méthyl-1H-pyrazol-4-yl)-1,3-dihydroimidazo[4,5-c]quinoléin-2-one |
| 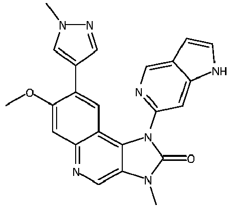 | 7-Méthoxy-3-méthyl-8-(1-méthyl-1H-pyrazol-4-yl)-1-(1H-pyrrolo[3,2-c]pyridin-6-yl)-1,3-dihydroimidazo[4,5-c]quinoléin-2-one |
| 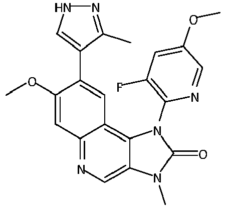 | 1-(3-Fluoro-5-méthoxypyridin-2-yl)-7-méthoxy-3-méthyl-8-(3-méthyl-1H-pyrazol-4-yl)-1,3-dihydroimidazo[4,5-c]quinoléin-2-one |
| 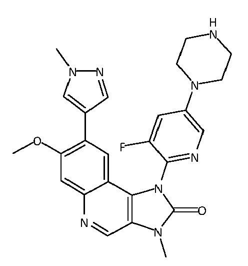 | 1-(3-Fluoro-5-pipérazin-1-yl-pyridin-2-yl)-7-méthoxy-3-méthyl-8-(1-méthyl-1H-pyrazol-4-yl)-1,3-dihydroimidazo[4,5-c]quinoléin-2-one |
| 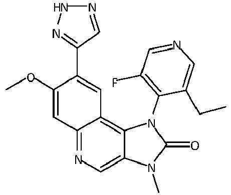 | 1-(3-Éthyl-5-fluoropyridin-4-yl)-7-méthoxy-3-méthyl-8-(2H-[1,2,3]triazol-4-yl)-1,3-dihydroimidazo[4,5-c]quinoléin-2-one |
| 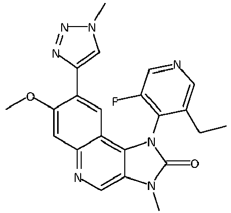 | 1-(3-Éthyl-5-fluoropyridin-4-yl)-7-méthoxy-3-méthyl-8-(1-méthyl-1H-[1,2,3]triazol-4-yl)-1,3-dihydroimidazo[4,5-c]quinoléin-2-one |
| 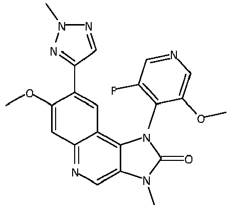 | 1-(3-Fluoro-5-méthoxypyridin-4-yl)-7-méthoxy-3-méthyl-8-(2-méthyl-2H-[1,2,3]triazol-4-yl)-1,3-dihydroimidazo-[4,5-c]quinoléin-2-one |
| 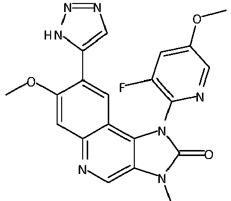 | 1-(3-Fluoro-5-méthoxypyridin-2-yl)-7-méthoxy-3-méthyl-8-(3H-[1,2,3]triazol-4-yl)-1,3-dihydroimidazo[4,5-c]quinoléin-2-one |
| 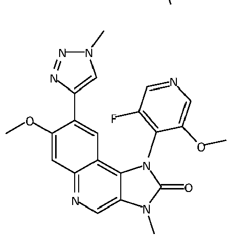 | 1-(3-Fluoro-5-méthoxypyridin-4-yl)-7-méthoxy-3-méthyl-8-(1-méthyl-1H-[1,2,3]-triazol-4-yl)-1,3-dihydroimidazo[4,5-c]-quinoléin-2-one |
| 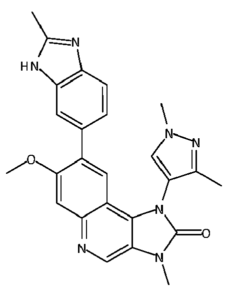 | 1-(1,3-Diméthyl-1H-pyrazol-4-yl)-7-méthoxy-3-méthyl-8-(2-méthyl-3H-benzoimidazol-5-yl)-1,3-dihydroimidazo[4,5-c]quinoléin-2-one |
| 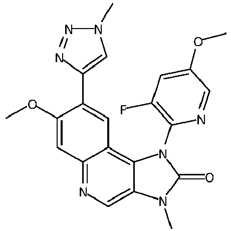 | 1-(3-Fluoro-5-méthoxypyridin-2-yl)-7-méthoxy-3-méthyl-8-(1-méthyl-1H-[1,2,3]-triazol-4-yl)-1,3-dihydroimidazo[4,5-c]-quinoléin-2-one |
| 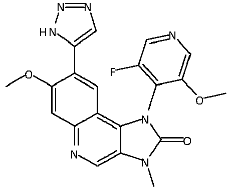 | 1-(3-Fluoro-5-méthoxypyridin-4-yl)-7-méthoxy-3-méthyl-8-(3H-[1,2,3]triazol-4-yl)-1,3-dihydroimidazo[4,5-c]quinoléin-2-one |
| 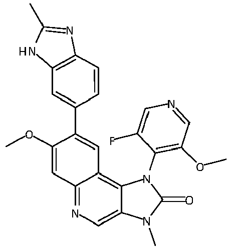 | 1-(3-Fluoro-5-méthoxypyridin-4-yl)-7-méthoxy-3-méthyl-8-(2-méthyl-3H-benzo-imidazol-5-yl)-1,3-dihydroimidazo[4,5-c]-quinoléin-2-one |
| 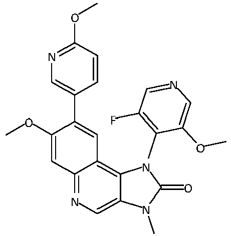 | 1-(3-Fluoro-5-méthoxypyridin-4-yl)-7-méthoxy-8-(6-méthoxypyridin-3-yl)-3-méthyl-1,3-dihydroimidazo[4,5-c]quinoléin-2-one |
| 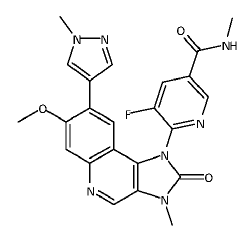 | 5-Fluoro-6-[7-méthoxy-3-méthyl-8-(1-méthyl-1H-pyrazol-4-yl)-2-oxo-2,3-dihydro-imidazo[4,5-c]quinoléin-1-yl]-N-méthyl-nicotinamide |
| 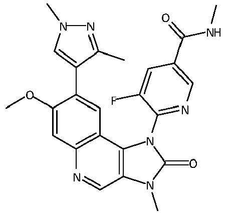 | 6-[8-(1,3-Diméthyl-1H-pyrazol-4-yl)-7-méthoxy-3-méthyl-2-oxo-2,3-dihydro-imidazo[4,5-c]quinoléin-1-yl]-5-fluoro-N-méthylnicotinamide |
| 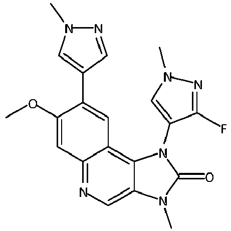 | 1-(3-Fluoro-1-méthyl-1H-pyrazol-4-yl)-7-méthoxy-3-méthyl-8-(1-méthyl-1H-pyrazol-4-yl)-1,3-dihydroimidazo[4,5-c]quinoléin-2-one |
| 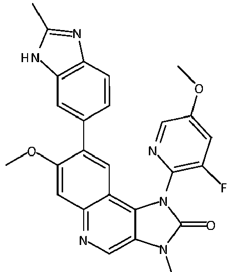 | 1-(3-Fluoro-5-méthoxypyridin-2-yl)-7-méthoxy-3-méthyl-8-(2-méthyl-3H-benzoimidazol-5-yl)-1,3-dihydroimidazo-[4,5-c]quinoléin-2-one |
| 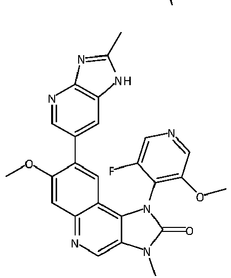 | 1-(3-Fluoro-5-méthoxypyridin-4-yl)-7-méthoxy-3-méthyl-8-(2-méthyl-1H-imidazo-[4,5-b]pyridin-6-yl)-1,3-dihydroimidazo-[4,5-c]quinoléin-2-one |
| 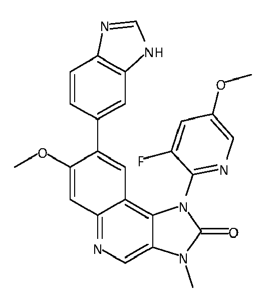 | 8-(3H-Benzimidazol-5-yl)-1-(3-fluoro-5-méthoxypyridin-2-yl)-7-méthoxy-3-méthyl-1,3-dihydroimidazo[4,5-c]quinoléin-2-one |
| 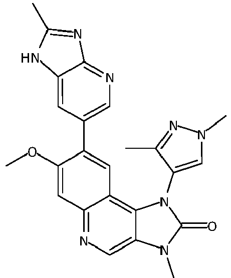 | 1-(1,3-Diméthyl-1H-pyrazol-4-yl)-7-méthoxy-3-méthyl-8-(2-méthyl-1H-imidazo[4,5-b]-pyridin-6-yl)-1,3-dihydroimidazo[4,5-c]-quinoléin-2-one |
| 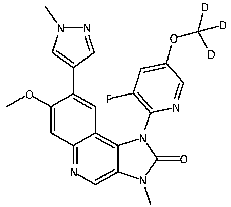 | 1-[3-Fluoro-5-(trideutériométhoxy)-2-pyridyl]-7-méthoxy-3-méthyl-8-(1-méthyl-pyrazol-4-yl)imidazo[4,5-c]quinoléin-2-one |
| 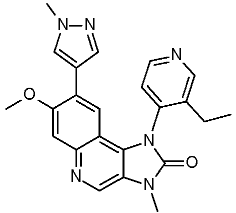 | 1-(3-Éthylpyridin-4-yl)-7-méthoxy-3-méthyl-8-(1-méthyl-1H-pyrazol-4-yl)-1,3-dihydro-imidazo[4,5-c]quinoléin-2-one |
| 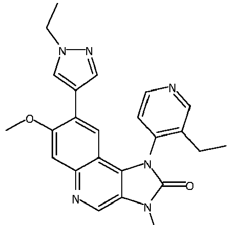 | 8-(1-Éthyl-1H-pyrazol-4-yl)-1-(3-éthyl-pyridin-4-yl)-7-méthoxy-3-méthyl-1,3-dihydroimidazo[4,5-c]quinoléin-2-one |
| 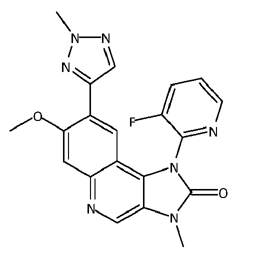 | 1-(3-Fluoropyridin-2-yl)-7-méthoxy-3-méthyl-8-(2-méthyl-2H-[1,2,3]triazol-4-yl)-1,3-dihydroimidazo[4,5-c]quinoléin-2-one |
| 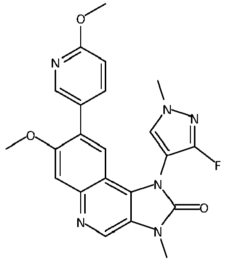 | 1-(3-Fluoro-1-méthyl-1H-pyrazol-4-yl)-7-méthoxy-8-(6-méthoxypyridin-3-yl)-3-méthyl-1,3-dihydroimidazo[4,5-c]quinoléin-2-one |
| 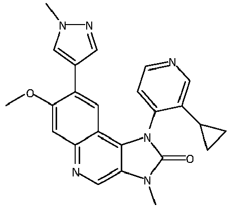 | 1-(3-Cyclopropylpyridin-4-yl)-7-méthoxy-3-méthyl-8-(1-méthyl-1H-pyrazol-4-yl)-1,3-dihydroimidazo[4,5-c]quinoléin-2-one |
| 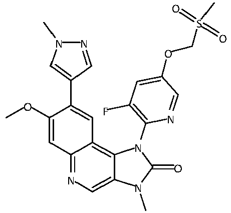 | 1-(3-Fluoro-5-méthylsulfonylméthoxypyridin-2-yl)-7-méthoxy-3-méthyl-8-(1-méthyl-1H-pyrazol-4-yl)-1,3-dihydroimidazo[4,5-c]-quinoléin-2-one |
| 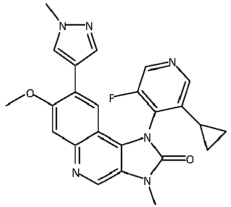 | 1-(3-Cyclopropyl-5-fluoropyridin-4-yl)-7-méthoxy-3-méthyl-8-(1-méthyl-1H-pyrazol-4-yl)-1,3-dihydroimidazo[4,5-c]quinoléin-2-one |
| 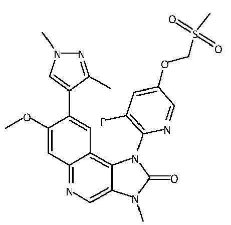 | 8-(1,3-Diméthyl-1H-pyrazol-4-yl)-1-(3-fluoro-5-méthylsulfonylméthoxypyridin-2-yl)-7-méthoxy-3-méthyl-1,3-dihydroimidazo-[4,5-c]quinoléin-2-one |
| 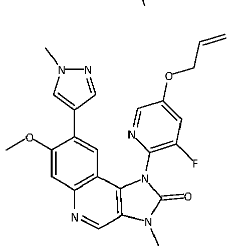 | 1-(5-Allyloxy-3-fluoropyridin-2-yl)-7-méthoxy-3-méthyl-8-(1-méthyl-1H-pyrazol-4-yl)-1,3-dihydroimidazo[4,5-c]quinoléin-2-one |
| 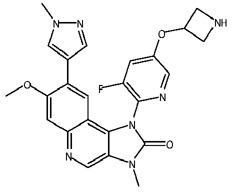 | 1-[5-(Azétidin-3-yloxy)-3-fluoropyridin-2-yl]-7-méthoxy-3-méthyl-8-(1-méthyl-1H-pyrazol-4-yl)-1,3-dihydroimidazo[4,5-c]-quinoléin-2-one |
| 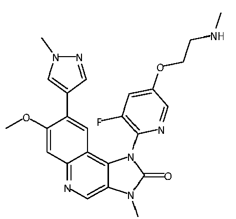 | 1-[3-Fluoro-5-(2-méthylaminoéthoxy)-pyridin-2-yl]-7-méthoxy-3-méthyl-8-(1-méthyl-1H-pyrazol-4-yl)-1,3-dihydro-imidazo[4,5-c]quinoléin-2-one |
| 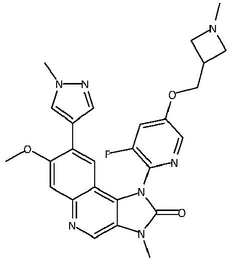 | 1-[3-Fluoro-5-(1-méthylazétidin-3-yl-méthoxy)pyridin-2-yl]-7-méthoxy-3-méthyl-8-(1-méthyl-1H-pyrazol-4-yl)-1,3-dihydro-imidazo[4,5-c]quinoléin-2-one |
| 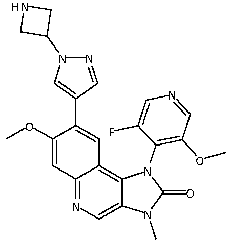 | 8-[1-(Azétidin-3-yl)-1H-pyrazol-4-yl]-1-(3-fluoro-5-méthoxypyridin-4-yl)-7-méthoxy-3-méthyl-1H,2H,3H-imidazo[4,5-c]quinoléin-2-one |
et/ou un sel, solvate ou tautomère pharmaceutiquement utilisable de celui-ci.

2. Composé selon la revendication 1 et/ou un sel, solvate ou tautomère pharmaceutiquement utilisable de celui-ci, pour une utilisation comme médicament.

3. Composé selon la revendication 1 et/ou un sel, solvate ou tautomère pharmaceutiquement utilisable de celui-ci, pour une utilisation dans le traitement d'un cancer, de tumeurs et/ou de métastases.

4. Composé et/ou un sel, solvate ou tautomère pharmaceutiquement utilisable de celui-ci, pour une utilisation dans le traitement d'un cancer, de tumeurs et/ou de métastases selon la revendication 3, en combinaison avec une radiothérapie.

5. Composé et/ou un sel, solvate ou tautomère pharmaceutiquement utilisable de celui-ci, pour une utilisation dans le traitement d'un cancer, de tumeurs et/ou de métastases selon la revendication 3, en combinaison avec au moins un agent anticancéreux.

6. Composé et/ou un sel, solvate ou tautomère pharmaceutiquement utilisable de celui-ci, pour une utilisation selon l'une des revendications 3 à 5, la tumeur étant choisie dans le groupe des maladies de l'épithélium squameux, de la vessie, de l'estomac, des reins, de la tête, du cou, de l'oesophage, du col de l'utérus, de la thyroïde, des intestins, des os, du foie, du cerveau, de la prostate, du tractus urogénital, du système lymphatique, du larynx, du poumon, de la peau, du sang et du système immunitaire, et/ou le cancer étant choisi dans le groupe constitué par la leucémie monocytique, l'adénocarcinome du poumon, le carcinome du poumon à petites cellules, le cancer du pancréas, le glioblastome, le carcinome de l'intestin, le carcinome du sein, la leucémie myéloïde aiguë, la leucémie myéloïde chronique, la leucémie lymphoïde aiguë, la leucémie lymphoïde chronique, le lymphome hodgkinien et le lymphome non hodgkinien.

7. Composé selon la revendication 1 et/ou un sel, solvate ou tautomère pharmaceutiquement utilisable de celui-ci, pour une utilisation dans la sensibilisation de cellules cancéreuses vis-à-vis d'un agent anticancéreux et/ou d'un rayonnement ionisant.

8. Composition pharmaceutique comprenant une quantité efficace d'au moins un composé selon la revendication 1 et/ou d'un sel, solvate ou tautomère pharmaceutiquement utilisable de celui-ci et au moins un excipient pharmaceutiquement toléré.

9. Composition pharmaceutique selon la revendication 8, comprenant en outre au moins un autre composé actif médicamenteux.

10. Composition pharmaceutique selon la revendication 9, dans laquelle l'autre composé actif médicamenteux est un agent anticancéreux.

11. Kit, constitué de conditionnements séparés
(a) d'une quantité efficace d'un composé selon la revendication 1 et/ou d'un sel, solvate ou tautomère pharmaceutiquement utilisable de celui-ci, et
(b) d'une quantité efficace d'un autre composé actif médicamenteux.

12. Kit selon la revendication 11, dans lequel l'autre composé actif médicamenteux est un agent anticancéreux.
